# EUROPEAN PATENT APPLICATION

(11) **EP 2 722 404 A1**
(43) Date of publication of application: **23.04.2014**
(21) Application number: 13196462.9
(22) Date of filing: 26.04.2006
(51) Int. Cl.: C12Q 1/68

(54) **Methods of predicting methotrextrate efficacy and toxicity**

(30) Priority: 28.04.2005 US 676442 P; 27.10.2005 US 731598 P; 25.04.2006 US 380171
(62) Divisional of application: 06751770.6
(71) Applicant: Nestec S.A., 1800 Vevey (CH)
(72) Inventor: Dervieux, Thierry, Encino, CA 91316 (US)
(74) Representative: Rupp, Christian

(57) **Abstract**

The present invention provides methods for analyzing genetic and/or metabolite biomarkers to individualize methotrexate (MTX) therapy. For example, the assay methods of the present invention are useful for predicting whether a patient will respond to MTX and/or has a risk of developing toxicity to MTX based upon the genotype of one or more folate pathway genes. The assay methods of the present invention are also useful for optimizing the dose of MTX in a patient already receiving the drug to achieve therapeutic efficacy and/or reduce toxic side-effects based upon the genotype of one or more folate pathway genes. In addition, the assay methods of the present invention are useful for predicting or optimizing the therapeutic response to MTX in a patient based upon the methotrexate polyglutamate and/or folate polyglutamate levels in a sample from the patient.

SEQUENCE LISTING

<110> Dervieux, Thierry

<120> Methods of Predicting Methotrexate Efficacy and
      Toxicity

<130> P43339/EP

<140> WO PCT/US06/16246
<141> 2006-04-26

<150> US 60/676,442
<151> 2005-04-28

<150> US 60/731,598
<151> 2005-10-27

<150> US 11/380,171
<151> 2006-04-25

<160> 32

<170> PatentIn Ver. 2.1

<210> 1
<211> 20
<212> DNA
<213> Artificial Sequence

<220>
<223> Description of Artificial Sequence:forward primer
      for detection of thymidylate synthase (TS) gene
      promoter region tandem repeat sequences

<400> 1
gtggctcctg cgtttccccc                                              20


<210> 2
<211> 38
<212> DNA
<213> Artificial Sequence

<220>
<223> Description of Artificial Sequence:reverse primer
      for detection of thymidylate synthase (TS) gene
      promoter region tandem repeat sequences

<400> 2
ccaagcttgg ctccgagccg gccacaggca tggcgcgg                          38


<210> 3
<211> 22
<212> DNA
<213> Artificial Sequence

<220>
<223> Description of Artificial Sequence:forward primer
      for amplifying a 292-bp fragment containing serine
      hydroxymethyltransferase (SHMT1) gene polymorphism

<400> 3
gtgtggggtg acttcatttg tg                                              22


<210> 4
<211> 21
<212> DNA
<213> Artificial Sequence

<220>
<223> Description of Artificial Sequence:reverse primer
      for amplifying a 292-bp fragment containing serine
      hydroxymethyltransferase (SHMT1) gene polymorphism

<400> 4
ggagcagctc atccatctct c                                               21


<210> 5
<211> 13
<212> DNA
<213> Artificial Sequence

<220>
<223> Description of Artificial Sequence:real-time PCR
      assay 3'-minor groove binding fluorescent probe to
      detect wild-type serine hydroxymethyltransferase
      (SHMT1) (C1420T) polymorphism

<220>
<221> modified_base
<222> (1)
<223> n = c modified by fluorescent reporter dye FAM

<220>
<221> modified_base
<222> (13)
<223> n = c modified by DNA minor groove binder (MGB)

<400> 5
ngcctctctc ttn                                                        13


<210> 6
<211> 13
<212> DNA
<213> Artificial Sequence

<220>
<223> Description of Artificial Sequence:real-time PCR
      assay 3'-minor groove binding fluorescent probe to
      detect mutant serine hydroxymethyltransferase
      (SHMT1) (C1420T) polymorphism

<220>
<221> modified_base
<222> (1)
<223> n = c modified by fluorescent reporter dye VIC

<220>
<221> modified_base
<222> (13)
<223> n = c modified by DNA minor groove binder (MGB)

<400> 6
ngcctctttc ttn                                                          13


<210> 7
<211> 22
<212> DNA
<213> Artificial Sequence

<220>
<223> Description of Artificial Sequence:real-time PCR
      assay forward primer to detect serine
      hydroxymethyltransferase (SHMT1) (C1420T)
      polymorphism

<400> 7
cagagccacc ctgaaagagt tc                                                22


<210> 8
<211> 18
<212> DNA
<213> Artificial Sequence

<220>
<223> Description of Artificial Sequence:real-time PCR
      assay reverse primer to detect serine
      hydroxymethyltransferase (SHMT1) (C1420T)
      polymorphism

<400> 8
agtgggcccg ctccttta                                                     18


<210> 9
<211> 25
<212> DNA
<213> Artificial Sequence

<220>
<223> Description of Artificial Sequence:standard real-time
      TaqMan allelic discrimination assay forward primer to
      detect aminoimidazole carboxamide ribonucleotide
      transformylase (ATIC) C347G polymorphism

<400> 9
cctgcaatct ctatcccttt gtaaa                                             25

<210> 10
<211> 26
<212> DNA
<213> Artificial Sequence

<220>
<223> Description of Artificial Sequence:standard real-time
      TaqMan allelic discrimination assay reverse primer to
      detect aminoimidazole carboxamide ribonucleotide
      transformylase (ATIC) C347G polymorphism

<400> 10
ttctgactta ccaatgtcaa tttgct                                          26


<210> 11
<211> 16
<212> DNA
<213> Artificial Sequence

<220>
<223> Description of Artificial Sequence:real-time TaqMan allelic
      discrimination assay 3'-minor groove binding fluorescent probe
      to detect wild-type aminoimidazole carboxamide ribonucleotide
      transformylase (ATIC) C347G polymorphism

<220>
<221> modified_base
<222> (1)
<223> n = c modified by fluorescent reporter dye FAM

<220>
<221> modified_base
<222> (16)
<223> n = g modified by DNA minor groove binder (MGB)

<400> 11
ncaggtgtaa gtgttn                                                     16


<210> 12
<211> 16
<212> DNA
<213> Artificial Sequence

<220>
<223> Description of Artificial Sequence:real-time TaqMan allelic
      discrimination assay 3'-minor groove binding fluorescent probe
      to detect mutant aminoimidazole carboxamide ribonucleotide
      transformylase (ATIC) C347G polymorphism

<220>
<221> modified_base
<222> (1)
<223> n = t modified by fluorescent reporter dye VIC

<220>
<221> modified_base
<222> (16)
<223> n = t modified by DNA minor groove binder (MGB)

<400> 12
nccaggtgta actgtn                                                    16


<210> 13
<211> 22
<212> DNA
<213> Artificial Sequence

<220>
<223> Description of Artificial Sequence:standard
      real-time TaqMan allelic discrimination assay
      forward primer to detect methylenetetrahydrofolate
      reductase (MTHFR) C677T polymorphism

<400> 13
cacaaagcgg aagaatgtgt ca                                             22


<210> 14
<211> 24
<212> DNA
<213> Artificial Sequence

<220>
<223> Description of Artificial Sequence:standard
      real-time TaqMan allelic discrimination assay
      reverse primer to detect methylenetetrahydrofolate
      reductase (MTHFR) C677T polymorphism

<400> 14
aagcacttga aggagaaggt gtct                                           24


<210> 15
<211> 15
<212> DNA
<213> Artificial Sequence

<220>
<223> Description of Artificial Sequence:standard real-time TaqMan
      allelic discrimination assay 3'-minor groove binding
      fluorescent probe to detect wild-type methylenetetrahydro-
      folate reductase (MTHFR) C677T polymorphism

<220>
<221> modified_base
<222> (1)
<223> n = t modified by fluorescent reporter dye FAM

<220>
<221> modified_base
<222> (15)
<223> n = g modified by DNA minor groove binder (MGB)

<400> 15
ngaaatcggc tcccn                                                     15

<210> 16
<211> 15
<212> DNA
<213> Artificial Sequence

<220>
<223> Description of Artificial Sequence:standard real-time TaqMan
      allelic discrimination assay 3'-minor groove binding
      fluorescent probe to detect mutant methylenetetrahydrofolate
      reductase (MTHFR) C677T polymorphism

<220>
<221> modified_base
<222> (1)
<223> n = t modified by fluorescent reporter dye VIC

<220>
<221> modified_base
<222> (15)
<223> n = g modified by DNA minor groove binder (MGB)

<400> 16
ngaaatcgac tcccn                                                        15


<210> 17
<211> 26
<212> DNA
<213> Artificial Sequence

<220>
<223> Description of Artificial Sequence:real-time
      TaqMan allelic discrimination assay forward primer
      to detect methionine synthase (MS) A2756G
      polymorphism

<400> 17
gaatactttg aggaaatcat ggaaga                                            26


<210> 18
<211> 29
<212> DNA
<213> Artificial Sequence

<220>
<223> Description of Artificial Sequence:real-time
      TaqMan allelic discrimination assay reverse primer
      to detect methionine synthase (MS) A2756G
      polymorphism

<400> 18
tctgtttcta ccacttacct tgagagact                                         29


<210> 19
<211> 16
<212> DNA
<213> Artificial Sequence

<220>
<223> Description of Artificial Sequence:real-time TaqMan allelic
      discrimination assay 3'-minor groove binding fluorescent
      probe to detect wild-type methionine synthase (MS) A2756G
      polymorphism

<220>
<221> modified_base
<222> (1)
<223> n = a modified by fluorescent reporter dye FAM

<220>
<221> modified_base
<222> (16)
<223> n = g modified by DNA minor groove binder (MGB)

<400> 19
ngacaggacc attatn                                                    16


<210> 20
<211> 14
<212> DNA
<213> Artificial Sequence

<220>
<223> Description of Artificial Sequence:real-time TaqMan allelic
      discrimination assay 3'-minor groove binding fluorescent
      probe to detect mutant methionine synthase (MS) A2756G
      polymorphism

<220>
<221> modified_base
<222> (1)
<223> n = a modified by fluorescent reporter dye VIC

<220>
<221> modified_base
<222> (14)
<223> n = g modified by DNA minor groove binder (MGB)

<400> 20
ncagggccat tatn                                                      14


<210> 21
<211> 18
<212> DNA
<213> Artificial Sequence

<220>
<223> Description of Artificial Sequence:real-time
      TaqMan allelic discrimination assay forward primer
      to detect methionine synthase reductase (MTRR)
      A66G polymorphism

<400> 21
gcaaaggcca tcgcagaa                                                  18

<210> 22
<211> 24
<212> DNA
<213> Artificial Sequence

<220>
<223> Description of Artificial Sequence:real-time
      TaqMan allelic discrimination assay reverse primer
      to detect methionine synthase reductase (MTRR)
      A66G polymorphism

<400> 22
gatctgcaga aaatccatgt acca                                              24


<210> 23
<211> 14
<212> DNA
<213> Artificial Sequence

<220>
<223> Description of Artificial Sequence:real-time TaqMan allelic
      discrimination assay 3'-minor groove binding fluorescent probe
      to detect wild-type methionine synthase reductase (MTRR)
      A66G polymorphism

<220>
<221> modified_base
<222> (1)
<223> n = t modified by fluorescent reporter dye FAM

<220>
<221> modified_base
<222> (14)
<223> n = c modified by DNA minor groove binder (MGB)

<400> 23
ngctcacata tttn                                                         14


<210> 24
<211> 15
<212> DNA
<213> Artificial Sequence

<220>
<223> Description of Artificial Sequence:real-time TaqMan allelic
      discrimination assay 3'-minor groove binding fluorescent probe
      to detect mutant methionine synthase reductase (MTRR) A66G
      polymorphism

<220>
<221> modified_base
<222> (1)
<223> n = c modified by fluorescent reporter dye VIC

<220>
<221> modified_base
<222> (15)
<223> n = t modified by DNA minor groove binder (MGB)

<400> 24
nttgctcaca cattn                                                        15


<210> 25
<211> 7
<212> PRT
<213> Artificial Sequence

<220>
<223> Description of Artificial Sequence:methotrexate
      polyglutamate (MTXPG), long-chain MTXPG

<220>
<221> MOD_RES
<222> (1)
<223> Xaa = Glu amide bonded to p-aminobenzoyl moiety of
      methotrexate (MTX)

<220>
<221> MOD_RES
<222> (3)..(7)
<223> Glu from positions 3-7 may be present or absent

<400> 25
Xaa Glu Glu Glu Glu Glu Glu
  1               5


<210> 26
<211> 5
<212> PRT
<213> Artificial Sequence

<220>
<223> Description of Artificial Sequence:methotrexate
      polyglutamate (MTXPG), MTXPG-5, long-chain MTXPG

<220>
<221> MOD_RES
<222> (1)
<223> Xaa = Glu amide bonded to p-aminobenzoyl moiety of
      methotrexate (MTX)

<400> 26
Xaa Glu Glu Glu Glu
  1               5


<210> 27
<211> 6
<212> PRT
<213> Artificial Sequence

<220>
<223> Description of Artificial Sequence:methotrexate
      polyglutamate (MTXPG), MTXPG-6, long-chain MTXPG

<220>
<221> MOD_RES
<222> (1)
<223> Xaa = Glu amide bonded to p-aminobenzoyl moiety of
      methotrexate (MTX)

<400> 27
Xaa Glu Glu Glu Glu Glu
  1               5


<210> 28
<211> 7
<212> PRT
<213> Artificial Sequence

<220>
<223> Description of Artificial Sequence:methotrexate
      polyglutamate (MTXPG), MTXPG-7, long-chain MTXPG

<220>
<221> MOD_RES
<222> (1)
<223> Xaa = Glu amide bonded to p-aminobenzoyl moiety of
      methotrexate (MTX)

<400> 28
Xaa Glu Glu Glu Glu Glu Glu
  1               5


<210> 29
<211> 7
<212> PRT
<213> Artificial Sequence

<220>
<223> Description of Artificial Sequence:folate
      polyglutamate, folate PG

<220>
<221> MOD_RES
<222> (1)
<223> Xaa = Glu bonded to folate

<220>
<221> MOD_RES
<222> (3)..(7)
<223> Glu at positions 3-7 may be present or absent

<400> 29
Xaa Glu Glu Glu Glu Glu Glu
  1               5


<210> 30
<211> 5
<212> PRT
<213> Artificial Sequence

<220>
<223> Description of Artificial Sequence:folate
      polyglutamate, folate PG, pteroylpentaglutamate

<220>
<221> MOD_RES
<222> (1)
<223> Xaa = Glu bonded to folate

<400> 30
Xaa Glu Glu Glu Glu
  1               5


<210> 31
<211> 6
<212> PRT
<213> Artificial Sequence

<220>
<223> Description of Artificial Sequence:folate
      polyglutamate, folate PG, pteroylhexaglutamate

<220>
<221> MOD_RES
<222> (1)
<223> Xaa = Glu bonded to folate

<400> 31
Xaa Glu Glu Glu Glu Glu
  1               5


<210> 32
<211> 7
<212> PRT
<213> Artificial Sequence

<220>
<223> Description of Artificial Sequence:folate
      polyglutamate, folate PG, pteroylheptaglutamate

<220>
<221> MOD_RES
<222> (1)
<223> Xaa = Glu bonded to folate

<400> 32
Xaa Glu Glu Glu Glu Glu Glu
  1               5

## Description

### CROSS-REFERENCES TO RELATED APPLICATIONS

This application claims priority to U.S. Provisional Application No. 60/676,442, filed April 28, 2005, and U.S. Provisional Application No. 60/731,598, filed October 27, 2005, the disclosures of which are hereby incorporated by reference in their entirety for all purposes.

### BACKGROUND OF THE INVENTION

Folate (folic acid) is a vitamin that is essential for the life-sustaining processes of DNA synthesis, replication, and repair. Folate is also important for protein biosynthesis, another process that is central to cell viability. The pteridine compound, methotrexate (MTX), is structurally similar to folate and as a result can bind to the active sites of a number of enzymes that normally use folate as a coenzyme for biosynthesis of purine and pyrimidine nucleotide precursors of DNA and for interconversion of amino acids during protein biosynthesis. Despite its structural similarity to folic acid, MTX cannot be used as a cofactor by enzymes that require folate, and instead competes with the folate cofactor for enzyme binding sites, thereby inhibiting protein and DNA biosynthesis and, hence, cell division.

The ability of the folate antagonist MTX to inhibit cell division has been exploited in the treatment of a number of diseases and conditions that are characterized by rapid or aberrant cell growth. For example, MTX is currently one of the most widely prescribed drugs for the treatment of rheumatoid arthritis, psoriasis, and cancer *(*Weinblatt et al., Eng. J. Med., 312:818-822 (1985); Kremer et al., Arthritis Rheum., 29:822-831 (1986)). Although MTX is among the best tolerated of the disease-modifying anti-rheumatic drugs, a major drawback of MTX therapy is a troublesome inter-patient variability in the clinical response and an unpredictable appearance of side-effects including gastrointestinal disturbances, alopecia, elevation of liver enzymes, and bone marrow suppression (Weinblatt et al., Arthritis Rheum., 37:1492-1498 (1994); Walker et al, Arthritis Rheum., 36:329-335 (1993)).

MTX enters cells through the reduced folate carrier (RFC-1) and is intracellularly activated by folylpolyglutamate synthase to methotrexate polyglutamates (MTXPGs) (Chabner et al., J. Clin. Invest., 76:907-912 (1985)). The γ-linked sequential addition of glutamic acid residues enhances intracellular retention of MTX (Allegra et al., Proc. Natl. Acad. Sci. USA, 82:4881-4885 (1985)). The polyglutamation process is in competition with deconjugation by gamma-glutamyl hydrolase (GGH) (Rhee et al., Mol. Pharmacol., 53:1040-1046 (1998); Yao et al., Proc. Natl. Acad. Sci. USA, 93:10134-10138 (1996); Panetta et al., Clin. Cancer Res., 8:2423-2429 (2002)), a lysosomal enzyme having high affinity towards long chain polyglutamates. (Masson et al., J. Clin. Invest., 97:73-80 (1996)).

The accumulation of MTXPGs is critical to the pharmacological effects of MTX. *In* vivo, the concentration of MTXPGs in lymphoblasts and erythrocytes appear to correlate with the therapeutic response to MTX in patients with leukemia (Dervieux et al., Blood, 100:1240-1247 (2002); Dervieux et al., Arthritis Rheum., 50:2766-2774 (2004)) or rheumatoid arthritis (Angelis-Stoforidis et al., Clin. Exp. Rheumatol., 17:313-320 (1999); Allegra *et al., supra*)*.* Polyglutamation of MTX is thought to promote the sustained inhibition of *de novo* purine synthesis by 5-aminoimidazole carboxamide-ribonucleotide transformylase (ATIC) (Dervieux et al., Blood, 100:1240-1247 (2002); Allegra *et al., supra*)*,* thereby promoting the build-up of adenosine, a potent anti-inflammatory agent (Baggott et al., Biochem. J., 236:193-200 (1986); Morabito et al., J. Clin. Invest., 101:295-300 (1998); Montesinos et al., Arthritis, 48:240-247 (2003); Cronstein et. al., J. Clin. Invest., 92:2675-2682 (1993)). Furthermore, MTXPGs are inhibitors of thymidylate synthase (TS) (Allegra et al., J. Biol. Chem., 260:9720-9726 (1985)). TS methylates deoxyuridine monophosphate to produce deoxythymidylate, providing a unique *de novo* source of thymidylate.

Part of the unpredictability of side-effects associated with MTX therapy may be related to common polymorphisms in genes implicated in MTX pharmacokinetics or pharmacodynamics. To date, the only genetic marker associated with MTX toxicity in patients with rheumatoid arthritis is a common polymorphism in 5,10-methylenetetrahydrofolate reductase, *MTHFR* C677T. However, the penetrance of this polymorphism is low, and its effect can be confounded by the concurrent administration of folic acid or by polymorphisms in other folate pathway genes (van Ede et al., Arthritis Rheum, 44:2525-2530 (2001); Uhich et al., Pharmacogenomics, 3:299-313 (2002)).

Because individual differences in the magnitude and occurrence of MTX toxicity can be difficult to predict, there exists a need in the art for methods that evaluate the risk of side-effects so that MTX therapy can be rendered safer and more effective. Likewise, there exists a need in the art for methods that evaluate the therapeutic efficacy of MTX therapy so that patients have an increased likelihood of responding to therapy. The present invention satisfies these needs and provides related advantages as well.

### BRIEF SUMMARY OF THE INVENTION

The present invention provides methods for analyzing genetic and/or metabolite biomarkers to individualize methotrexate (MTX) therapy in patients who have been diagnosed with a disease such as an inflammatory disease, autoimmune disease, or cancer. In particular, the assay methods of the present invention are useful for predicting whether a patient will respond to MTX and/or has a risk of developing toxicity to MTX based upon the genotype of one or more folate pathway genes. The assay methods of the present invention are also useful for optimizing the dose of MTX in a patient already receiving the drug to achieve therapeutic efficacy and/or reduce toxic side-effects based upon the genotype of one or more folate pathway genes. In addition, the assay methods of the present invention are useful for predicting or optimizing the therapeutic response to MTX in a patient based upon the methotrexate polyglutamate (MTXPG) and/or folate polyglutamate (folate PG) levels in a sample from the patient.

As such, in one aspect, the present invention provides an assay method for evaluating the likelihood that a subject will respond to MTX, the method comprising:
(a) determining the genotype of at least one folate pathway gene selected from the group consisting of a methylenetetrahydrofolate reductase (*MTHFR*) gene, a thymidylate synthase (*TS*) gene, a serine hydroxymethyltransferase (*SHMT1*) gene, and a combination thereof in a sample from the subject;
(b) generating an efficacy index based upon the genotype of the at least one folate pathway gene; and
(c) evaluating the likelihood that the subject will respond to MTX based upon the efficacy index.

In a related aspect, the present invention provides an assay method for optimizing dose efficacy in a subject receiving MTX, the method comprising:
(a) determining the genotype of at least one folate pathway gene selected from the group consisting of a methylenetetrahydrofolate reductase *(MTHFR)* gene, a thymidylate synthase (*TS*) gene, a serine hydroxymethyltransferase (*SHMT1*) gene, and a combination thereof in a sample from the subject;
(b) generating an efficacy index based upon the genotype of the at least one folate pathway gene; and
(c) recommending a subsequent dose of MTX based upon the efficacy index.

In another aspect, the present invention provides an assay method for evaluating the risk that a subject will develop toxicity to MTX, the method comprising:
(a) determining the genotype of at least one folate pathway gene selected from the group consisting of a methylenetetrahydrofolate reductase (*MTHFR*) gene, a thymidylate synthase (*TS*) gene, a serine hydroxymethyltransferase (*SHMT1*) gene, an aminoimidazole carboxamide ribonucleotide transformylase (*ATIC*) gene, a gamma-glutamyl hydrolase (*GGH*) gene, a methionine synthase (*MS*) gene, a methionine synthase reductase (*MTRR*) gene, and a combination thereof in a sample from the subject;
(b) generating a toxicogenetic index based upon the genotype of the at least one folate pathway gene; and
(c) evaluating the risk that the subject will develop toxicity to MTX based upon the toxicogenetic index.

In a related aspect, the present invention provides an assay method for reducing toxicity in a subject receiving MTX, the method comprising:
(a) determining the genotype of at least one folate pathway gene selected from the group consisting of a methylenetetrahydrofolate reductase (*MTHFR*) gene, a thymidylate synthase (*TS*) gene, a serine hydroxymethyltransferase (*SHMT1*) gene, an aminoimidazole carboxamide ribonucleotide transformylase (*ATIC*) gene, a gamma-glutamyl hydrolase (*GGH*) gene, a methionine synthase (*MS*) gene, a methionine synthase reductase (*MTRR*) gene, and a combination thereof in a sample from the subject;
(b) generating a toxicogenetic index based upon the genotype of the at least one folate pathway gene; and
(c) recommending a subsequent dose of MTX based upon the toxicogenetic index.

In yet another aspect, the present invention provides an assay method for evaluating the likelihood that a subject will respond to MTX, the method comprising:
(a) determining a level of MTXPGs in a sample from the subject; and
(b) evaluating the likelihood that the subject will respond to MTX based upon the level of MTXPGs.

In a related aspect, the present invention provides an assay method for evaluating the likelihood that a subject will respond to MTX, the method comprising:
(a) determining a level of folate PGs in a sample from the subject; and
(b) evaluating the likelihood that the subject will respond to MTX based upon the level of folate PGs.

In a further aspect, the present invention provides an assay method for optimizing dose efficacy in a subject receiving MTX, the method comprising:
(a) determining a level of MTXPGs in a sample from the subject; and
(b) recommending a subsequent dose of MTX based upon the level of MTXPGs.

In a related aspect, the present invention provides an assay method for optimizing dose efficacy in a subject receiving MTX, the method comprising.
(a) determining a level of folate PGs in a sample from the subject; and
(b) recommending a subsequent dose of MTX based upon the level of folate PGs.

In some aspects, the present invention further provides systems and kits for predicting or optimizing the response to MTX in a subject comprising:
(a) a genotypic profile module for genotyping the subject at a polymorphic site in at least one folate pathway gene; and
(b) a pharmacogenetic profile module for generating an efficacy index based upon the genotype of the at least one folate pathway gene.

In other aspects, the present invention further provides systems and kits for predicting or reducing toxicity to MTX in a subject comprising:
(a) a genotypic profile module for genotyping the subject at a polymorphic site in at least one folate pathway gene; and
(b) a pharmacogenetic profile module for generating a toxicogenetic index based upon the genotype of the at least one folate pathway gene.

Other objects, features, and advantages of the present invention will be apparent to one of skill in the art from the following detailed description and figures.

### BRIEF DESCRIPTION OF THE DRAWINGS

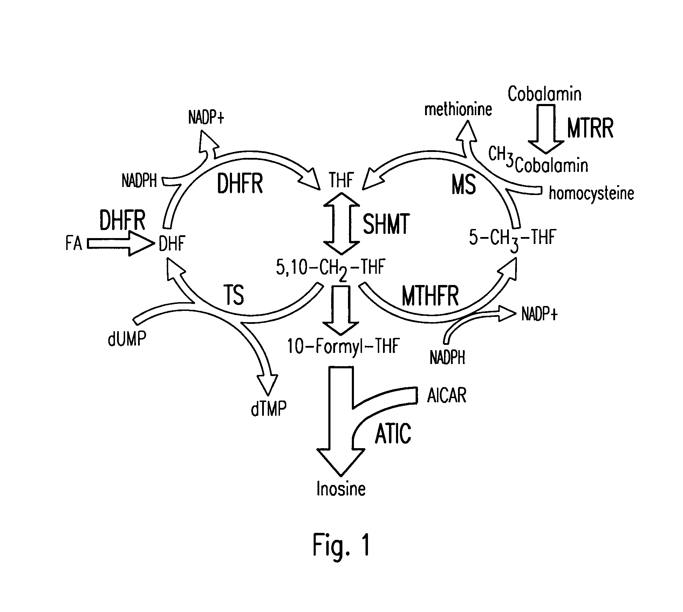

Figure 1 shows a schematic of the folate pathway. Abbreviations: FA, folic acid; DHF, dihydrofolate; THF, tetrahydrofolate; 5,10-CH₂-THF, 5,10 methylenetetrahydrofolate; 10-formyl-THF, 10 formyltetrahydrofolate; 5-CH₃-THF, 5-methyltetrahydrofolate; dUMP, deoxyuridine monophosphate; dTMP, deoxythymidine monophosphate; DHFR, dihydrofolate reductase; TS, thymidylate synthase; SHMT1, serine hydroxymethyltransferase; MTHFR, methylenetetrahydrofolate reductase; MS, methionine synthase; MTRR, methionine synthase reductase; ATIC, AICAR transformylase (inosine monophosphate synthetase); AICAR, aminidoimidazole carboxamide ribonuleotide.

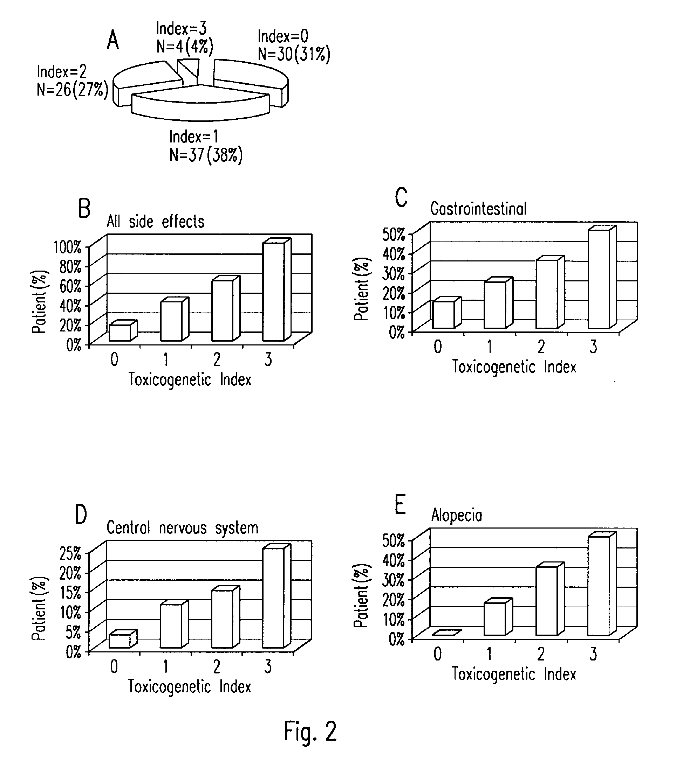

Figure 2 shows the contribution of the toxicogenetic index to the occurrence of side-effects. In Figure 2A, the toxicogenetic index was calculated as the sum of the presence of the *MTHFR* 677T/T, *ATIC* 347G/G, *TS* *2/*2, and *SHMT* 1420C/C risk genotypes. The number of patients (%) is given for each index value. Figure 2B shows the percentage of patients with side-effects to MTX therapy for a particular index value. Figures 2C-2E show the percentage of patients with gastrointestinal side-effects, central nervous system side-effects, and alopecia for a particular index value.

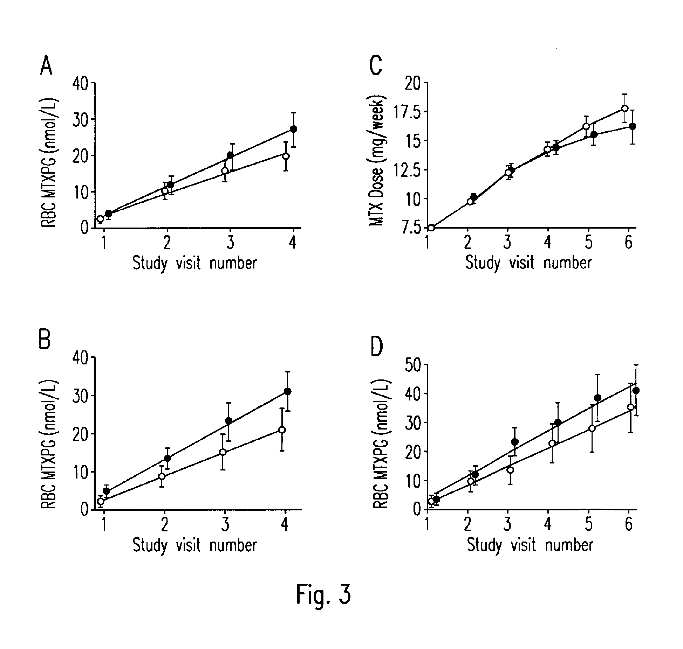

Figure 3 shows the erythrocyte MTXPG levels and response to MTX therapy. In Figure 3A, responders (filled circles) to therapy at the fourth study visit presented higher formation of RBC MTXPGs than non-responders (open circles) (generalized linear model; estimate = 0.034 ± 0.020; p=0.095). Figure 3B shows that a decrease in the physician's assessment of disease activity VAS from baseline to visit 4 was associated with greater formation of RBC MTXPGs (p=0.0002). RBC MTXPG levels in patients having a decrease in the physician's assessment of disease activity VAS above median (filled circles) versus below group median (open circles; decrease of 47%) are plotted. In Figure 3C, a lesser decrease in disease activity from baseline to visit 6 was associated with higher MTX doses administered (p=0.0234). MTX doses administered in patients having a change in the disease activity above median (filled circles) versus below group median (open circles; decrease of 36%) are presented. Figure 3D shows that a lesser decrease in disease activity from baseline to visit 6 was associated with lower RBC MTXPG levels (p=0.0046). RBC MTXPG levels in patients having a change in the disease activity above median (filled circles) versus below group median (open circles; decrease of 41%) are presented. Bars represent mean with 95% confidence interval.

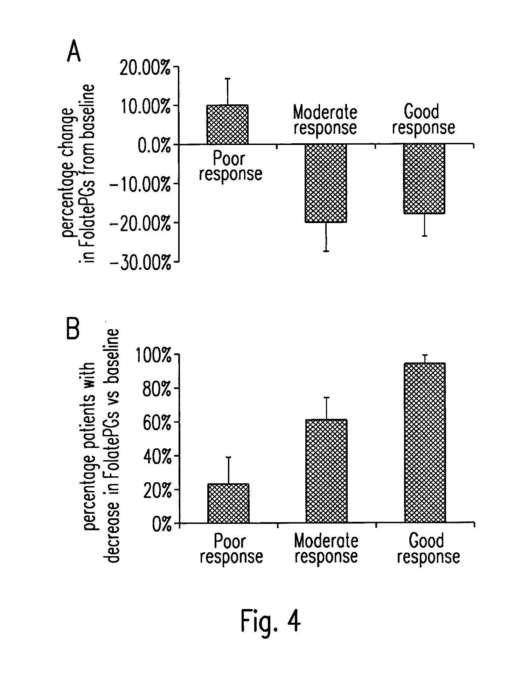

Figure 4 shows the erythrocyte folate PG levels and response to MTX therapy. In Figure 4A, the decrease in erythrocyte folate PG levels from baseline to the fourth study visit was higher in responders than in non-responders (p<0.01). Figure 4B shows that the percentage of patients with a decrease in erythrocyte folate PG levels from initiation of therapy to the fourth study visit was higher in responders than in non-responders (p<0.01). Bars represent mean ± SEM.

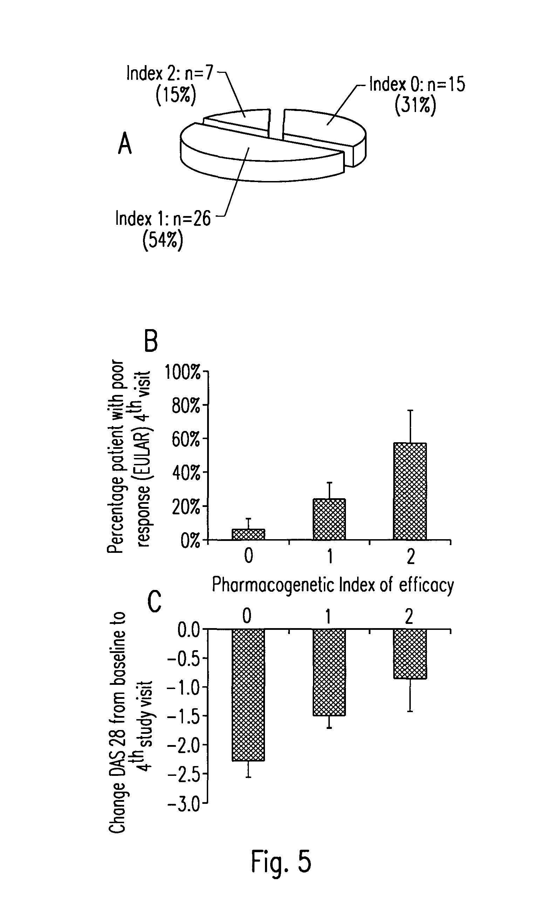

Figure 5 shows the pharmacogenetic and therapeutic response to MTX therapy. In Figure 5A, the efficacy index was calculated as the sum of the presence of the *MTHFR* 677T/T, *SHMT1* 1420C/T or T/T, and *TS* *2/*2 risk genotypes. Index values with the percentage of patients are given. Figure 5B shows that an increased index value was associated with an increased percentage of patients with a poor response (EULAR criteria) at the fourth study visit (p=0.02). In Figure 5C, an increased index value was associated with a lower decrease in the disease activity score from baseline to visit 4 (p=0.02). Bars represent mean ± SEM.

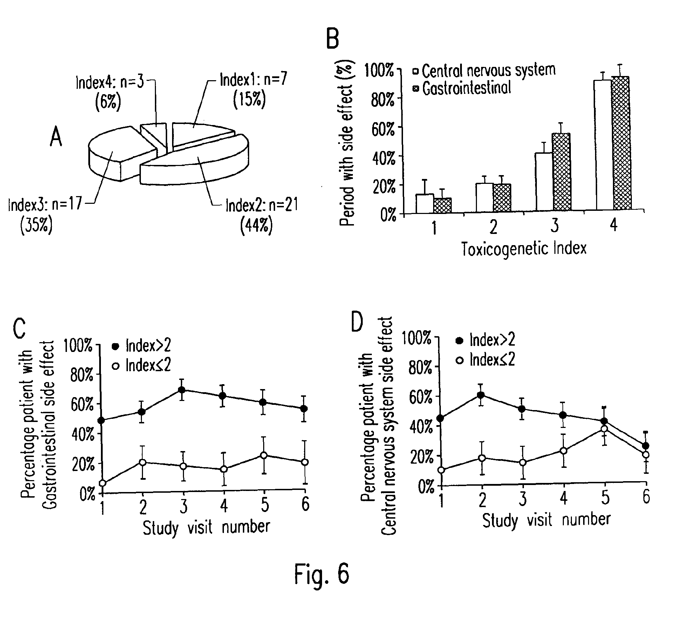

Figure 6 shows the toxicogenetic index and the percentage of 4-6 week periods with central nervous system and gastrointestinal side-effects. In Figure 6A, the toxicogenetic index was calculated as the sum of the presence of the GGH -401C/C, *ATIC* 347G/G, *MTHFR* 1298A/C or C/C, *MS* 2756A/A, and *MTRR* 66G/G risk genotypes. Index values with the percentage of patients are given. Figure 6B shows that an increased toxicogenetic index was associated with an increase in the percentage of 4-6 week periods (per patient) with central nervous system and gastrointestinal side-effects. The percentage of 4-6 week periods per patient with side-effects is given. Figures 6C and 6D show that the percentage of patients with side-effects (6C, gastrointestinal; 6D, central nervous system) at each period and for an index >2 or ≤ 2. Bars represent mean ± SEM.

### DETAILED DESCRIPTION OF THE INVENTION

### L Introduction

The present invention is based, in part, on the surprising discovery that one or more genetic and/or metabolite biomarkers can be used to individualize methotrexate (MTX) therapy in patients with a disease such as an inflammatory disease, autoimmune disease, or cancer. Given the high inter-patient variability in response and toxicity to MTX, the assay methods of the present invention are particularly advantageous because they utilize a strategy that takes into account differences in the genotype and/or metabolite level of one or more molecular determinants (*i.e.,* biomarkers) to create a dosing regimen tailored for each patient that achieves therapeutic efficacy without inducing toxic side-effects. Consequently, patients who are about to begin MTX therapy can receive the full benefits of such therapy without experiencing any severe or life-threatening side-effects such as diarrhea or leucopenia by determining an appropriate initial dose of MTX. Similarly, patients already undergoing treatment with MTX can experience a reduction in toxic side-effects without compromising therapeutic efficacy by adjusting the subsequent dose of MTX.

The present invention demonstrates for the first time that common polymorphisms in genes encoding folate pathway enzymes such as folate-dependent enzymes and homocysteine remethylation-dependent enzymes are associated with a patient's response to MTX therapy and/or the risk or occurrence of side-effects to MTX therapy. In particular, Example 1 shows that generating a toxicogenetic index by measuring and cumulating *MTHFR, ATIC, TS,* and *SHMT1* genotypes can be used to evaluate a patient's risk of side-effects to MTX therapy. Similarly, Example 2 shows that generating a toxicogenetic index by measuring and cumulating *GGH, ATIC, MTHFR, MTRR,* and MS genotypes can be used to evaluate a patient's risk of side-effects to MTX therapy. Example 2 also shows that generating an efficacy index by measuring and cumulating *MTHFR, TS,* and *SHMT1* genotypes can be used to evaluate a patient's likelihood of response to MTX therapy. Additionally, Example 2 shows that red blood cell (RBC) methotrexate polyglutamate (MTXPG) and folate polyglutamate (folate PG) concentrations are associated with a patient's response to MTX therapy. As such, these results indicate that a composite index cumulating certain genotypes in folate pathway genes can be used to predict and optimize a patient's response and/or toxicity to MTX therapy, thereby classifying patients as those likely to respond to MTX therapy and/or those likely to have side-effects or adverse events associated with MTX therapy. These results also indicate that MTXPG and/or folate PG concentrations can be used to predict and optimize a patient's response to MTX therapy.

As such, the present invention provides more accurate methods for: (1) predicting whether a patient has a higher likelihood of responding to MTX; (2) identifying patients with a greater risk of developing toxic side-effects to MTX; and (3) optimizing MTX dosages (e.g., optimizing dose amount, optimizing dose efficacy, reducing drug toxicity, etc.) in patients undergoing MTX therapy.

### II. Definitions

As used herein, the following terms have the meanings ascribed to them unless specified otherwise.

The term "inflammatory disease" refers to a disease or disorder characterized or caused by inflammation. "Inflammation" refers to a local response to cellular injury that is marked by capillary dilatation, leukocytic infiltration, redness, heat, and pain that serves as a mechanism initiating the elimination of noxious agents and of damaged tissue. The site of inflammation includes the lungs, the pleura, a tendon, a lymph node or gland, the uvula, the vagina, the brain, the spinal cord, nasal and pharyngeal mucous membranes, a muscle, the skin, bone or bony tissue, a joint, the urinary bladder, the retina, the cervix of the uterus, the canthus, the intestinal tract, the vertebrae, the rectum, the anus, a bursa, a follicle, and the like. Such inflammatory diseases include, but are not limited to, inflammatory bowel disease, rheumatoid diseases (e.g., rheumatoid arthritis), other arthritic diseases (e.g., acute arthritis, acute gouty arthritis, bacterial arthritis, chronic inflammatory arthritis, degenerative arthritis (osteoarthritis), infectious arthritis, juvenile arthritis, mycotic arthritis, neuropathic arthritis, polyarthritis, proliferative arthritis, psoriatic arthritis, venereal arthritis, viral arthritis), fibrositis, pelvic inflammatory disease, acne, psoriasis, actinomycosis, dysentery, biliary cirrhosis, Lyme disease, heat rash, Stevens-Johnson syndrome, mumps, pemphigus vulgaris, and blastomycosis. Inflammatory bowel diseases are chronic inflammatory diseases of the gastrointestinal tract which include, without limitation, Crohn's disease, ulcerative colitis, and indeterminate colitis. Rheumatoid arthritis is a chronic inflammatory disease primarily of the joints, usually polyarticular, marked by inflammatory changes in the synovial membranes and articular structures and by muscle atrophy and rarefaction of the bones.

The term "autoimmune disease" refers to a disease or disorder resulting from an immune response against a self tissue or tissue component and includes a self antibody response or cell-mediated response. The term autoimmune disease, as used herein, encompasses organ-specific autoimmune diseases, in which an autoimmune response is directed against a single tissue, such as Type I diabetes mellitus, myasthenia gravis, vitiligo, Graves' disease, Hashimoto's disease, Addison's disease, autoimmune gastritis, and autoimmune hepatitis. The term autoimmune disease also encompasses non-organ specific autoimmune diseases, in which an autoimmune response is directed against a component present in several or many organs throughout the body. Such autoimmune diseases include, for example, systemic lupus erythematosus, progressive systemic sclerosis and variants, polymyositis, and dermatomyositis. Additional autoimmune diseases include, but are not limited to, pernicious anemia, primary biliary cirrhosis, autoimmune thrombocytopenia, Sjögren's syndrome, and multiple sclerosis.

The term "cancer" refers to any of various malignant neoplasms characterized by the proliferation of anaplastic cells that tend to invade surrounding tissue and metastasize to new body sites. Examples of different types of cancer include, but are not limited to, lung cancer, breast cancer, bladder cancer, thyroid cancer, liver cancer, pleural cancer, pancreatic cancer, ovarian cancer, cervical cancer, testicular cancer, colon cancer, anal cancer, bile duct cancer, gastrointestinal carcinoid tumors, esophageal cancer, gall bladder cancer, rectal cancer, appendix cancer, small intestine cancer, stomach (gastric) cancer, renal cancer, cancer of the central nervous system, skin cancer, choriocarcinomas; head and neck cancers, blood cancers, osteogenic sarcomas, B-cell lymphoma, non-Hodgkin's lymphoma, Burkitt's lymphoma, fibrosarcoma, neuroblastoma, glioma, melanoma, monocytic leukemia, myelogenous leukemia, acute lymphocytic leukemia, and acute myelocytic leukemia.

A "folate pathway gene" refers to any gene involved in folate homeostasis and/or metabolism and includes the proteins encoded by these genes. Examples of folate pathway genes include, but are not limited to, folate-dependent enzyme genes such as 5,10-methylenetetrahydrofolate reductase (*MTHFR*), 5-aminoimidazole-4-carboxamide ribonucleotide transformylase (*ATIC*), thymidylate synthase (*TS*), serine hydroxymethyltransferase (*SHMT*), dihydrofolate reductase (*DHFR*), 10-formyltetrabydrofolate synthetase (*FTHFS*), 10-formyltetrahydrofolate dehydrogenase (*FTHFD*)*,* glycinamide ribonucleotide transformylase (*GART*), reduced folate carrier (*RFC-*1), folylpolyglutamate synthase (*FPGS*), gamma-glutamyl hydrolase (GGH), and combinations thereof; and homocysteine remethylation-dependent enzyme genes such as methionine synthase (MS), methionine synthase reductase (*MTRR*), betaine-homocysteine methyltransferase (*BHMT*)*,* and combinations thereof. A schematic of the folate pathway is provided in Figure 1.

The term "gene" refers to the segment of DNA involved in producing a polypeptide chain; it includes regions preceding and following the coding region, such as the promoter and 3'-untranslated region, respectively, as well as intervening sequences (introns) between individual coding segments (exons).

The term "nucleic acid" or "polynucleotide" refers to deoxyribonucleotides or ribonucleotides and polymers thereof in either single- or double-stranded form including, for example, genomic DNA, cDNA, and mRNA. This term encompasses nucleic acid molecules of both natural and synthetic origin as well as molecules of linear, circular, or branched configuration representing either the sense or antisense strand, or both, of a native nucleic acid molecule. It is understood that such nucleic acids can be unpurified, purified, or attached, for example, to a synthetic material such as a bead or column matrix. The term also encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), polymorphisms, alleles, orthologs, SNPs, and complementary sequences as well as the sequence explicitly indicated. The term nucleic acid is used interchangeably with gene, cDNA, and mRNA encoded by a gene.

The term "polymorphism" refers to the occurrence of two or more genetically determined alternative sequences or alleles in a population. A "polymorphic site" refers to the locus at which divergence occurs. Preferred polymorphic sites have at least two alleles, each occurring at a particular frequency in a population. A polymorphic locus may be as small as one base pair (single nucleotide polymorphism, or SNP). Polymorphic markers include restriction fragment length polymorphisms, variable number of tandem repeats (VNTR's), hypervariable regions, minisatellites, dinucleotide repeats, trinucleotide repeats, tetranucleotide repeats, simple sequence repeats, and insertion elements such as Alu. The first identified allele is arbitrarily designated as the reference allele, and other alleles are designated as alternative alleles, "variant alleles," or "variance. The alleles occurring most frequently in a selected population are sometimes referred to as the "wild-type" allele. Diploid organisms may be homozygous or heterozygous for the variant alleles. The variant allele may or may not produce an observable physical or biochemical characteristic ("phenotype") in an individual carrying the variant allele. For example, a variant allele may alter the enzymatic activity of a protein encoded by a gene of interest.

A "single nucleotide polymorphism" or "SNP" occurs at a polymorphic site occupied by a single nucleotide, which is the site of variation between allelic sequences. The site is usually preceded by and followed by highly conserved sequences of the allele (*e.g*., sequences that vary in less than 1/100 or 1/1000 members of the populations). A SNP usually arises due to substitution of one nucleotide for another at the polymorphic site. A transition is the replacement of one purine by another purine or one pyrimidine by another pyrimidine. A transversion is the replacement of a purine by a pyrimidine or vice versa. Single nucleotide polymorphisms can also arise from a deletion of a nucleotide or an insertion of a nucleotide relative to a reference allele.

The term "genotype" refers to the genetic composition of an organism, including, for example, whether a diploid organism is wild-type, heterozygous, or homozygous for one or more variant alleles of interest.

The term "risk genotype" refers to any wild-type, heterozygous, or homozygous genotype for a variant allele of interest that is associated with a decreased or low therapeutic response to MTX and/or an increased or high risk or occurrence of side-effects to MTX. Examples of risk genotypes for evaluating therapeutic response to MTX include, without limitation, *MTHFR* 677T/T, *TS* *2/*2, *SHMT1* 1420C/T or T/T, and combinations thereof. Non-limting examples of risk genotypes for evaluating the risk or occurrence of side-effects associated with MTX include *MTHFR* 677T/T, *MTHFR* 1298A/C or C/C, *ATIC* 347G/G, *TS* *2/*2, *SHMT1* 1420C/C, *GGH*-401C/C, *MTRR* 66G/G, *MS* 2756A/A, and combinations thereof.

The term "side-effect" refers to an undesirable secondary effect of a drug or therapy. Typical side-effects associated with MTX therapy include, without limitation, gastrointestinal side-effects (e.g., nausea, diarrhea, stomatatis, dyspepsia), central nervous system side-effects (e.g., headache, lethargy), hematopoietic system side-effects (*e.g*., leucopenia, anemia), pulmonary system side-effects, alopecia, and combinations thereof.

The term "subject" or "patient" typically refers to humans, but also to other animals including, e.g., other primates, rodents, canines, felines, equines, ovines, porcines, and the like.

As used herein, the term "biomarker" or "marker" refers to any biochemical marker, serological marker, genetic marker, or other clinical or echographic characteristic that can be used in predicting or determining MTX efficacy or toxicity in a subject according to the methods of the present invention. Examples of biochemical or serological markers include, without limitation, polyglutamated derivatives of MTX (MTXPGs) and polyglutamated derivatives of folate (folate PGs). Preferably, the biochemical or serological markers described herein are measured to determine their levels in a subject's sample. Examples of genetic markers include, without limitation, any of the above-described folate pathway genes. Preferably, the genetic markers described herein are genotyped to detect the presence or absence of a variant allele.

The term "sample" refers to any biological specimen obtained from a subject. Samples include, without limitation, whole blood, plasma, serum, buccal cells, red blood cells, white blood cells (e.g., peripheral blood mononuclear cells), saliva, urine, stool (*i.e*., feces), tears, any other bodily fluid, a tissue sample (e.g., tumor tissue) such as a biopsy of a tumor, and cellular extracts thereof. In certain instances, the sample is whole blood, serum, or plasma. In certain other instances, the sample is tumor tissue, e.g., from a solid tumor. Preferably, the tumor tissue sample is a formalin fixed paraffin embedded (FFPE) tumor tissue sample.

The term "course of therapy" or "therapy" refers to any therapeutic approach taken to relieve and/or prevent one or more symptoms associated with a disease or disorder such as an inflammatory disease, autoimmune disease, or cancer. The term encompasses administering any compound, drug, therapeutic agent, procedure, or regimen useful for improving the health of a subject having the disease or disorder. One skilled in the art will appreciate that either the course of therapy or the dose of the current course of therapy can be changed based upon the pharmacogenetic indexes and/or metabolite levels determined using the methods of the present invention. Examples of therapies suitable for use in the methods of the present invention include, without limitation, MTX therapy, therapy with other folate antagonists, antibiotic therapy, immunosuppressive therapy, anti-inflammatory therapy, conventional chemotherapy, radiation therapy, hormonal therapy, immunotherapy, and combinations thereof.

The term "recommending" as used herein refers to providing dosing instructions for MTX or an alternative therapy based on the pharmacogenetic index (e.g., efficacy index, toxicogenetic index, etc.) calculated for a particular subject and/or the metabolite levels (*e.g*., MTXPG levels, folate PG levels, *etc*.) determined for that subject. In some embodiments, the methods of the present invention for evaluating the likelihood that a subject will respond to MTX or the risk that a subject will develop toxicity to MTX rely on determining the genotype of at least one folate pathway gene and/or the level of at least one long-chain MTXPG (e.g., MTXPG₃) to provide a recommendation of a dose of the drug. In other embodiments, the methods of the present invention for optimizing or reducing toxicity to MTX therapy in a subject already receiving the drug rely on determining the genotype of at least one folate pathway gene and/or the level of at least one long-chain MTXPG to provide a recommendation of a subsequent dose of the drug or an alternative therapy. Dosing instructions include, without limitation, lab results with preferred drug doses, data sheets, look-up tables setting forth preferred drug doses, instructions or guidelines for using the drug, package inserts to accompany the drug, professional medical advice and the like. In certain aspects, the term "recommending" associates a particular pharmacogenetic index value and/or metabolite level with side-effects or efficacy.

The term "methotrexate polyglutamate" is synonymous with "MTXPG" and refers to a derivative of methotrexate having two or more glutamates which are amide bonded to the p-aminobenzoyl moiety of methotrexate. The number of glutamates in a methotrexate polyglutamate varies from two to seven [SEQ ID NO:25] or more; the number of glutamate moieties can be denoted by "n" using the nomenclature MTXPGₙ such that, for example, MTXPG₂ is MTXPG having two glutamates, MTXPG₃ is MTXPG having three glutamates, MTXPG₄ is MTXPG having four glutamates, MTXPG₅ is MTXPG having five glutamates [SEQ ID NO:26], MTXPG₆ is MTXPG having six glutamates [SEQ ID NO:27], MTXPG₇ is MTXPG having seven glutamates [SEQ ID NO:28], and MTXPG₃₋₅ is a mixture containing MTXPG₃, MTXPG₄, and MTXPG₅ [SEQ ID NO:26], with the ratio of the individual polyglutamated forms in the mixture not defined. As used herein, the term "long-chain MTXPG" refers to any MTX having at least three glutamates attached thereto (e.g., MTXPG₃, MTXPG₄, MTXPG₅ [SEQ ID NO:26], MTXPG₆ [SEQ ID NO:27], and/or MTXPG₇ [SEQ ID NO:28]).

The term "folate polyglutamate" is synonymous with "folate PG" and refers to a derivative of folate having two or more glutamates which are bonded thereto via the action of folylpolyglutamate synthase. The number of glutamates in a folate polyglutamate varies from two to seven [SEQ ID NO:29] or more. For example, folate polyglutamates can include, without limitation, folate metabolites such as the pteroyldiglutamate, pteroyltriglutamate, pteroyltetraglutamate, pteroylpentaglutamate [SEQ ID NO:30], pteroylhexaglutamate [SEQ ID NO:31], and/or pteroylheptaglutamate [SEQ ID NO:32] forms of folate.

The term "genotypic profile module" refers to any device or apparatus for genotyping a subject at a polymorphic site in at least one gene. Suitable genotypic profile modules for use in the systems of the present invention include, without limitation, microarrays such as oligonucleotide or polynucleotide arrays, polymerase chain reaction (PCR)-based devices, sequencing apparatuses, and electrophoretic apparatuses. Preferably, the genotypic profile module is a microarray. A description of arrays suitable for use in the systems of the present invention is provided below.

The term "pharmacogenetic profile module" refers to any device, apparatus, software code, or a combination thereof for generating a pharmacogenetic index. Suitable pharmacogenetic profile modules for use in the systems of the present invention include, without limitation, any device or apparatus capable of calculating one or pharmacogenetic indexes using, for example, one or more of the algorithms described below. As a non-limiting example, computers comprising software including computer readable medium having computer executable instructions for performing algorithmic calculations are within the scope of the systems of the present invention. Alternatively, the pharmacogenetic profile module is a computer software program capable of performing algorithmic calculations to generate pharmacogenetic indexes. Suitable computer readable medium include, without limitation, floppy disk, CD-ROM/DVD/DVD-ROM, hard-disk drive, flash memory, ROM/RAM, magnetic tapes, etc. The computer executable instructions may be written in a suitable computer language or a combination of several languages. Basic computational biology methods are described in, e.g., Setubal et al., Introduction to Computational Biology Methods, PWS Publishing Company, Boston (1997); Salzberg, Searles, Kasif, (Ed.), Computational Methods in Molecular Biology, Elsevier, Amsterdam (1998); Rashidi and Buehler, Bioinformatics Basics: Application in Biological Science and Medicine, CRC Press, London (2000); and Ouelette and Bzevanis, Bioinformatics: A Practical Guide for Analysis of Gene and Proteins, Wiley & Sons, Inc., 2nd Ed. (2001). In certain instances, the pharmacogenetic profile module of the present invention can be used in conjunction with the genotypic profile module for probe design, management of data, analysis, and/or instrument operation.

As used herein, the term "administering" means oral administration, administration as a suppository, topical contact, intravenous, intraperitoneal, intramuscular, intralesional, intrathecal, intranasal or subcutaneous administration, or the implantation of a slow-release device, e.g., a mini-osmotic pump, to a subject. Administration is by any route, including parenteral and transmucosal (*e.g*., buccal, sublingual, palatal, gingival, nasal, vaginal, rectal, or transdermal). Parenteral administration includes, *e.g*., intravenous, intramuscular, intra-arteriole, intradermal, subcutaneous, intraperitoneal, intraventricular, and intracranial. Other modes of delivery include, but are not limited to, the use of liposomal formulations, intravenous infusion, transdermal patches, *etc.* By "co-administer" it is meant that MTX or another therapeutic agent is administered at the same time, just prior to, or just after the administration of one or more additional drugs or therapeutic regimens.

The term "evaluating the likelihood that a subject will respond to MTX" refers to the use of the pharmacogenetic indexes and/or metabolite levels of the present invention to determine whether a subject would likely respond to MTX therapy. Although rheumatoid arthritis is used herein as a non-limiting example, one skilled in the art will appreciate that subjects having other diseases or disorders in which MTX provides some therapeutic benefit can also be evaluated according to the methods of the present invention. In some embodiments, the pharmacogenetic index (*i.e*., efficacy index) of a subject having rheumatoid arthritis is calculated based upon the genotype of at least one folate pathway gene and compared to an index cutoff value. In certain instances, the subject has a decreased or low likelihood of responding to MTX when the efficacy index is greater than the index cutoff value. Alternatively, the subject has an increased or high likelihood of responding to MTX when the efficacy index is less than or equal to the index cutoff value. In other embodiments, the level of MTXPGs and/or folate PGs in a subject having rheumatoid arthritis is measured and compared to a threshold level. In certain instances, the subject has an increased or high likelihood of responding to MTX when the level of MTXPGs is greater than an MTXPG threshold level and/or the level of folate PGs is less than a folate PG threshold level. Alternatively, the subject has a decreased or low likelihood of responding to MTX when the level of MTXPGs is less than the MTXPG threshold level and/or the level of folate PGs is greater than the folate PGthreshold level.

The term "evaluating the risk that a subject will develop toxicity to MTX" refers to the use of the pharmacogenetic indexes and/or metabolite levels of the present invention to determine whether a subject would be at risk of developing side-effects or adverse events to MTX therapy. In some embodiments, the pharmacogenetic index (*i.e*., toxicogenetic index) of a subject having a disease or disorder such as rheumatoid arthritis is calculated based upon the genotype of at least one folate pathway gene and compared to an index cutoff value. In certain instances, the subject is at an increased or high risk of developing toxicity to MTX when the toxicogenetic index is greater than the index cutoff value. Alternatively, the subject is at a decreased or low risk of developing toxicity to MTX when the toxicogenetic index is less than or equal to the index cutoff value. In other embodiments, the level of MTXPGs and/or folate PGs in a subject having a disease or disorder such as rheumatoid arthritis is measured and compared to a toxic level. In certain instances, the subject is at an increased or high risk of developing toxicity to MTX when the level of MTXPGs and/or folate PGs is greater than the toxic level. Alternatively, the subject is at a decreased or low risk of developing toxicity to MTX when the level of MTXPGs and/or folate PGs is less than the toxic level.

The term "optimizing dose efficacy in a subject receiving MTX" refers to the use of the pharmacogenetic indexes and/or metabolite levels of the present invention to adjust the subsequent dose of MTX or to change the course of therapy for a subject after the drug has been administered in order to optimize its therapeutic efficacy. In some embodiments, the pharmacogenetic index (*i.e*., efficacy index) of a subject having a disease or disorder such as rheumatoid arthritis is calculated based upon the genotype of at least one folate pathway gene and compared to an index cutoff value. In certain instances, the subsequent dose of MTX is increased or an alternative therapy administered when the efficacy index is greater than the index cutoff value. Alternatively, the subsequent dose of MTX is maintained when the efficacy index is less than or equal to the index cutoff value. In other embodiments, the level of MTXPGs and/or folate PGs in a subject having a disease or disorder such as rheumatoid arthritis is measured and compared to a threshold level. In certain instances, the subsequent dose of MTX is increased or an alternative therapy administered when the level of MTXPGs is less than an MTXPG threshold level and/or the level of folate PGs is greater than a folate PG threshold level. Alternatively, the subsequent dose of MTX is maintained when the level of MTXPGs is greater than the MTXPG threshold level and/or the level of folate PGs is less than the folate PG threshold level.

The term "reducing toxicity in a subject receiving MTX" refers to the use of the pharmacogenetic indexes and/or metabolite levels of the present invention to adjust the subsequent dose of MTX or to change the course of therapy for a subject after the drug has been administered in order to reduce any side-effects associated with the drug. In some embodiments, the pharmacogenetic index (*i.e*., toxicogenetic index) of a subject having a disease or disorder such as rheumatoid arthritis is calculated based upon the genotype of at least one folate pathway gene and compared to an index cutoff value. In certain instances, the subsequent dose of MTX is maintained when the toxicogenetic index is less than or equal to the index cutoff value. Alternatively, the subsequent dose of MTX is decreased or an alternative therapy administered when the toxicogenetic index is greater than the index cutoff value. In other embodiments, the level of MTXPGs and/or folate PGs in a subject having a disease or disorder such as rheumatoid arthritis is measured and compared to a toxic level. In certain instances, the subsequent dose of MTX is decreased or an alternative therapy administered when the level of MTXPGs and/or folate PGs is greater than the toxic level. Alternatively, the subsequent dose of MTX is maintained when the level of MTXPGs and/or folate PGs is less than the toxic level.

The term "index cutoff value" refers to a number chosen on the basis of population analysis that is used for comparison to a pharmacogenetic index calculated for a subject. Those of skill in the art will recognize that an index cutoff value can be determined according to the needs of the user and characteristics of the analyzed population. As a non-limiting example, the pharmacogenetic index can be compared to an index cutoff value described in Examples 1 and 2 below to predict, monitor, or optimize MTX therapy according to the methods of the present invention.

### III. Description of the Embodiments

The present invention provides methods for analyzing genetic and/or metabolite biomarkers to individualize methotrexate (MTX) therapy. For example, the assay methods of the present invention are useful for predicting whether a patient will respond to MTX therapy and/or has a risk of developing toxicity to MTX. The assay methods of the present invention are also useful for optimizing the dose of MTX in a patient receiving the drug by adjusting the subsequent dose of the drug to achieve therapeutic efficacy and/or reduce toxicity.

Accordingly, in one aspect, the present invention provides an assay method for evaluating the likelihood that a subject will respond to MTX, the method comprising:
(a) determining the genotype of at least one folate pathway gene selected from the group consisting of a methylenetetrahydrofolate reductase (*MTHFR*) gene, a thymidylate synthase (*TS*) gene, a serine hydroxymethyltransferase (*SHMT1*) gene, and a combination thereof in a sample from the subject;
(b) generating an efficacy index based upon the genotype of the at least one folate pathway gene; and
(c) evaluating the likelihood that the subject will respond to MTX based upon the efficacy index.

In a related aspect, the present invention provides an assay method for optimizing dose efficacy in a subject receiving MTX, the method comprising:
(a) determining the genotype of at least one folate pathway gene selected from the group consisting of a methylenetetrahydrofolate reductase (*MTHFR*) gene, a thymidylate synthase (*TS*) gene, a serine hydroxymethyltransferase (*SHMT1*) gene, and a combination thereof in a sample from the subject;
(b) generating an efficacy index based upon the genotype of the at least one folate pathway gene; and
(c) recommending a subsequent dose of MTX based upon the efficacy index.

The subject typically has a disease or disorder in which MTX may provide some therapeutic benefit such as an inflammatory disease, an autoimmune disease, or cancer. For example, the subject may have rheumatoid arthritis. In some embodiments, the genotype of the folate pathway gene(s) is determined at a polymorphic site. In certain instances, the polymorphic site is located in a coding region, or alternatively, in a non-coding region such as a promoter. Preferably, the polymorphic site is a single nucleotide polymorphism (SNP). In other embodiments, the genotype of at least two, three, four, five, six, seven, eight, nine, ten, or more folate pathway genes is determined. Suitable genotyping techniques are known in the art and are described below. The sample used for genotypic analysis is usually a whole blood, serum, plasma, or buccal cell sample.

In the methods of the present invention for predicting a subject's response to MTX prior to administration of the drug or for optimizing MTX dose efficacy in a subject already receiving the drug, the efficacy index is generated based upon the genotype of at least one of the *MTHFR, TS,* and *SHMT1* genes. Each of these genotypes typically comprises a wild-type, heterozygous, or homozygous genotype for a variant allele at a polymorphic site in the gene. For example, the *MTHFR* coding sequence at nucleotide 677 can comprise a wild-type genotype (*MTHFR* 677C/C), a heterozygous genotype *(MTHFR* 677C/T), or a homozygous risk genotype (*MTHFR* 677T/T). The *TS* promoter sequence can comprise a wild-type genotype *(TS* *3/*3), a heterozygous genotype (*TS* *3/*2), or a homozygous risk genotype (*TS **2/***2)*.* The *SHMT1* coding sequence at nucleotide 1420 can comprise a wild-type genotype (*SHMT1* 1420C/C), a heterozygous risk genotype (*SHMT1* 1420C/T), or a homozygous risk genotype (*SHMT1* 1420T/T). In certain instances, the efficacy index is calculated based upon the genotype of all three genes.

Other folate pathway genes can also be genotyped to predict or optimize MTX therapy according to the methods of the present invention. Examples include, but are not limited to, an aminoimidazole carboxamide ribonucleotide transformylase (*ATIC*) gene, a gamma-glutamyl hydrolase (*GGH*) gene, a reduced folate carrier (*RFC-1*) gene, a methionine synthase (*MS*) gene, a methionine synthase reductase (*MTRR*) gene, and combinations thereof.

In some embodiments, the efficacy index is generated using an algorithm such as by calculating the sum of or the difference between the number the risk genotypes in the *MTHFR, TS*, and/or *SHMT1* genes that are present in a subject. However, one skilled in the art will appreciate that other methods for generating the efficacy index such as those described below are within the scope of the present invention. In certain instances, the efficacy index is generated by calculating the sum of the *MTHFR* 677T/T, *TS* *2/*2, and *SHMT1* 1420C/F or T/T risk genotypes. Depending on the value of the efficacy index, a low, intermediate, or high initial or subsequent dose of MTX can be recommended for administration to the subject. Alternatively, a different therapeutic agent can be recommended for administration to the subject.

In other embodiments, the efficacy index is compared to an index cutoff value. In the methods of the present invention for predicting a subject's response to MTX prior to administration of the drug, an efficacy index greater than the index cutoff value may indicate that the subject does not have a high likelihood of responding to MTX, and the methods of the present invention may further comprise recommending a high initial dose of MTX or an alternative therapy to be administered. Alternatively, an efficacy index less than or equal to the index cutoff value may indicate that the subject has a moderate or high likelihood of responding to MTX, and the methods of the present invention may further comprise recommending a low or intermediate initial dose of MTX to be administered. However, one skilled in the art will appreciate that the correlation between the efficacy index and the likelihood of response to MTX depends on whether index values above, below, or equal to the index cutoff value are associated with an increased or high likelihood of response to MTX.

Similarly, in the methods of the present invention for optimizing MTX dose efficacy in a subject already receiving the drug, an efficacy index greater than the index cutoff value may indicate that the subsequent dose of MTX should be increased (e.g., to intermediate or high dose MTX therapy) or an alternative therapy should be administered. As a non-limiting example, the subsequent dose of MTX can be increased by about 2.5 mg/week or a multiple or fraction thereof. Alternatively, an efficacy index less than or equal to the index cutoff value may indicate that the subsequent dose of MTX should be maintained. However, one skilled in the art will appreciate that the correlation between the efficacy index and the optimal dose of MTX depends on whether index values above, below, or equal to the index cutoff value are associated with efficacious doses of MTX.

In another aspect, the present invention provides an assay method for evaluating the risk that a subject will develop toxicity to MTX, the method comprising:
(a) determining the genotype of at least one folate pathway gene selected from the group consisting of a methylenetetrahydrofolate reductase (*MTHFR*) gene, a thymidylate synthase (*TS*) gene, a serine hydroxymethyltransferase (*SHMT1*) gene, an aminoimidazole carboxamide ribonucleotide transformylase (*ATIC*) gene, a gamma-glutamyl hydrolase (*GGH*) gene, a methionine synthase (*MS*) gene, a methionine synthase reductase (*MTRR*) gene, and a combination thereof in a sample from the subject;
(b) generating a toxicogenetic index based upon the genotype of the at least one folate pathway gene; and
(c) evaluating the risk that the subject will develop toxicity to MTX based upon the toxicogenetic index.

In a related aspect, the present invention provides an assay method for reducing toxicity in a subject receiving MTX, the method comprising:
(a) determining the genotype of at least one folate pathway gene selected from the group consisting of a methylenetetrahydrofolate reductase (*MTHFR*) gene, a thymidylate synthase (*TS*) gene, a serine hydroxymethyltransferase (*SHMT1*) gene, an aminoimidazole carboxamide ribonucleotide transformylase (*ATIC*) gene, a gamma-glutamyl hydrolase (*GGH*) gene, a methionine synthase (*MS*) gene, a methionine synthase reductase (*MTRR*) gene, and a combination thereof in a sample from the subject;
(b) generating a toxicogenetic index based upon the genotype of the at least one folate pathway gene; and
(c) recommending a subsequent dose of MTX based upon the toxicogenetic index.

The subject typically has a disease or disorder in which MTX may provide some therapeutic benefit such as an inflammatory disease, an autoimmune disease, or cancer. For example, the subject may have rheumatoid arthritis. In some embodiments, the genotype of the folate pathway gene(s) is determined at a polymorphic site. In certain instances, the polymorphic site is located in a coding region, or alternatively, in a non-coding region such as a promoter. Preferably, the polymorphic site is a single nucleotide polymorphism (SNP). In other embodiments, the genotype of at least two, three, four, five, six, seven, eight, nine, ten, or more folate pathway genes is determined. The sample used for genotypic analysis is usually a whole blood, serum, plasma, or buccal cell sample. Typical adverse events associated with MTX therapy include, but are not limited to, gastrointestinal side-effects, central nervous system side-effects, hematopoietic system side-effects, pulmonary system side-effects, alopecia, and a combination thereof.

In the methods of the present invention for predicting a subject's risk of developing toxicity to MTX prior to administration of the drug or for reducing MTX toxicity in a subject already receiving the drug, the toxicogenetic index is generated based upon the genotype of at least one of the *MTHFR, ATIC, TS, SHMT1, GGH, MTRR,* and *MS* genes. Each of these genotypes typically comprises a wild-type, heterozygous, or homozygous genotype for a variant allele at a polymorphic site in the gene. For example, the *MTHFR* coding sequence at nucleotide 677 can comprise a wild-type genotype (*MTHFR* 677C/C), a heterozygous genotype (*MTHFR* 677C/T), or a homozygous risk genotype *(MTHFR* 677T/T). Alternatively, *the MTHFR* coding sequence at nucleotide 1298 can comprise a wild-type genotype *(MTHFR* 1298A/A), a heterozygous risk genotype (*MTHFR* 1298A/C), or a homozygous risk genotype *(MTHFR* 1298C/C). The *TS* promoter sequence can comprise a wild-type genotype *(TS* *3/*3), a heterozygous genotype *(TS* *3/*2), or a homozygous risk genotype (*TS* *2/*2). The *SHMT1* coding sequence at nucleotide 1420 can comprise a wild-type risk genotype *(SHMT1* 1420C/C), a heterozygous genotype *(SHMT1* 1420C/T), or a homozygous genotype {*SHMT1* 1420T/T). The A77C coding sequence at nucleotide 347 can comprise a wild-type genotype (*ATIC* 347C/C), a heterozygous genotype *(ATIC* 347C/G), or a homozygous risk genotype (*ATIC* 347G/G). The promoter region 5' of the *GGH* coding sequence can comprise a wild-type risk genotype (*GGH* -401C/C), a heterozygous genotype (*GGH* -401C/T), or a homozygous genotype *(GGH* -401T/T). The *MS* coding sequence at nucleotide 2756 can comprise a wild-type risk genotype (MS 2756A/A), a heterozygous genotype (MS 2756A/G), or a homozygous genotype (*MS* 2756G/G). The *MTRR* coding sequence at nucleotide 66 can comprise a wild-type genotype (*MTRR* 66A/A), a heterozygous genotype *(MTRR* 66A/G), or a homozygous risk genotype (*MTRR* 66G/G). In certain instances, the toxicogenetic index is calculated based upon the genotype of the *MTHFR, ATIC, TS,* and *SHMT1* genes. In certain other instances, the toxicogenetic index is calculated based upon the genotype of the *MTHFR, ATIC, GGH, MTRR,* and *MS* genes.

Other folate pathway genes can also be genotyped to predict whether a subject has a risk of developing toxic side-effects to MTX according to the methods of the present invention. Examples include, but are not limited to, a reduced folate carrier (*RFC-1*) gene.

In some embodiments, the toxicogenetic index is generated using an algorithm such as by calculating the sum of or the difference between the number the risk genotypes in the *MTHFR, ATIC, TS, SHMT1, GGH, MTRR,* and/or *MS* genes that are present in a subject. However, one skilled in the art will appreciate that other methods for generating the toxicogenetic index such as those described below are within the scope of the present invention. In certain instances, the toxicogenetic index is generated by calculating the sum of the *MTHFR* 677T/T, *ATIC* 347G/G, *TS* *2/*2, and *SHMT1* 1420C/C risk genotypes. In certain other instances, the toxicogenetic index is generated by calculating the sum of the *GGH* -401C/C, *ATIC* 347G/G, *MTHFR* 1298A/C or C/C, *MTRR* 66G/G, and *MS* 2756A/A risk genotypes. Depending on the value of the toxicogenetic index, a low, intermediate, or high initial or subsequent dose of MTX can be recommended for administration to the subject. Alternatively, a different therapeutic agent can be recommended for administration to the subject.

In other embodiments, the toxicogenetic index is compared to an index cutoff value. In the methods of the present invention for predicting a subject's risk of developing toxicity to MTX prior to administration of the drug, a toxicogenetic index greater than the index cutoff value may indicate that the subject is at moderate or high risk of developing toxicity to MTX, and the methods of the present invention may further comprise recommending a low or intermediate initial dose of MTX or an alternative therapy to be administered. Alternatively, a toxicogenetic index less than or equal to the index cutoff value may indicate that the subject is not at high risk of developing toxicity to MTX, and the methods of the present invention may further comprise recommending a high initial dose of MTX to be administered. However, one skilled in the art will appreciate that the correlation between the toxicogenetic index and the likelihood of developing toxicity to MTX depends on whether index values above, below, or equal to the index cutoff value are associated with an increased or high risk of developing toxicity to MTX.

Similarly, in the methods of the present invention for reducing MTX toxicity in a subject already receiving the drug, a toxicogenetic index greater than the index cutoff value may indicate that the subsequent dose of MTX should be decreased (e.g., to intermediate or low dose MTX therapy) or an alternative therapy should be administered. As a non-limiting example, the subsequent dose of MTX can be decreased by about 2.5 mg/week or a multiple or fraction thereof. Alternatively, a toxicogenetic index less than or equal to the index cutoff value may indicate that the subsequent dose of MTX should be maintained. However, one skilled in the art will appreciate that the correlation between the toxicogenetic index and the optimal dose of MTX depends on whether index values above, below, or equal to the index cutoff value are associated with toxic doses of MTX.

In yet another aspect, the present invention provides an assay method for evaluating the likelihood that a subject will respond to MTX, the method comprising:
(a) determining a level of MTXPGs in a sample from the subject; and
(b) evaluating the likelihood that the subject will respond to MTX based upon the level of MTXPGs.

The subject typically has a disease or disorder in which MTX may provide some therapeutic benefit such as an inflammatory disease, an autoimmune disease, or cancer. For example, the subject may have rheumatoid arthritis. In some embodiments, the level of at least one long-chain MTXPG is determined. Non-limiting examples of long-chain MTXPGs include MTXPG₃, MTXPG₄, MTXPG₅ [SEQ ID NO:26], MTXPG₆ [SEQ ID NO:27], MTXPG₇ [SEQ ID NO:28], and a combination thereof. In certain instances, the level of MTXPG₃ alone is determined. In certain other instances, the level of MTXPG₃, together with MTXPG₄, MTXPG [SEQ ID NO:26], MTXPG₆ [SEQ ID NO:27], and/or MTXPG₇ [SEQ ID NO:28], is determined. Alternatively, the level of MTXPG₄ and/or MTXPG₅ [SEQ ID NO:26] is determined. The level of MTXPGs such as long-chain MTXPGs is usually determined in red blood cells (RBCs) or a cellular extract (e.g., red blood cellular extract) obtained from the subject.

MTXPG₃ is the predominant polyglutamate species in RBCs and is strongly predictive of the long-chain MTXPG concentration expressed as the sum of MTXPG₃ + MTXPG₄ + MTXPG₅ [SEQ ID NO:26] (MTXPG₃₋₅). The level of MTXPG₃ in RBCs is also predictive of the long-chain MTXPG concentration expressed as the sum of MTXPG₄ + MTXPG₅ [SEQ ID NO:26] (MTXPG₄₋₅) and of MTXPG₅ [SEQ ID NO:26]. As such, RBC MTXPG₃ levels can be used as a marker of MTXPG₃₋₅, MTXPG₄₋₅, and/or MTXPG₅ [SEQ ID NO: 26] levels. Preferably, the level of MTXPGs is determined as described below using a high performance liquid chromatography (HPLC)-fluorometry procedure with a post-column photo-oxidation technique. Alternatively, the level of MTXPGs can be determined using mass spectrometry.

In some embodiments, the level of one or more long-chain MTXPGs is determined within the first 6 months of starting MTX therapy. As a non-limiting example, the level of MTXPG₃ can be measured in RBCs within about 1, 2, 3, 4, 5, or 6 months of starting MTX therapy. In certain instances, a level of MTXPG₃ greater than a threshold level of about 20 nmol/L RBCs indicates that a subject has a high likelihood of responding to MTX about 3 months later. For example, a level of MTXPG₃ greater than about 20 nmol/L RBCs, when measured at about 3 months after starting MTX therapy, is highly predictive of a subject having a therapeutic response to MTX at about 6 months into therapy. Alternatively, a level of MTXPG₃ greater than about 20 nmol/L RBCs, when measured at about 1, 2, 4, 5, or 6 months after starting MTX therapy, can be predictive of a subject having a therapeutic response to MTX at about 4, 5, 7, 8, or 9 months, respectively, into therapy. In certain other instances, a level of MTXPG₃ greater than a threshold level of about 10, 15, 16, 17, 18, 19, 21, 22, 23, 24, 25, 30, 35, 40, 45, 50, 55, 60, 65, 70, 75, 80, 85, 90, 95, or 100 nmol/L RBCs indicates that a subject has a high likelihood of responding to MTX about 3 months later.

In other embodiments, the level of one or more long-chain MTXPGs is determined at any time during MTX therapy. As a non-limiting example, the level of MTXPG₃ can be measured in RBCs at about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, or more months after starting MTX therapy. In certain instances, a detectable level of MTXPG₃ indicates that a subject has a high likelihood of responding to MTX about 1 month later. For example, the presence of MTXPG₃ in a RBC sample, when determined at about 1, 2, or 3 months after starting MTX therapy, is predictive of a subject having a therapeutic response to MTX at about 2, 3, or 4 months, respectively, into therapy. One skilled in the art will appreciate that the detection limit of MTXPGs in RBCs typically depends on the analytical method used. For example, RBC MTXPG levels that are measured as described below using an HPLC-fluorometry procedure with a post-column photo-oxidation technique provide a quantification limit of about 5 nmol/L RBCs and a detection limit of about 2 nmol/L RBCs.

In a related aspect, the present invention provides an assay method for evaluating the likelihood that a subject will respond to MTX, the method comprising:
(a) determining a level of folate PGs in a sample from the subject; and
(b) evaluating the likelihood that the subject will respond to MTX based upon the level of folate PGs.

The subject typically has a disease or disorder in which MTX may provide some therapeutic benefit such as an inflammatory disease, an autoimmune disease, or cancer. For example, the subject may have rheumatoid arthritis. The level of folate PGs is usually determined in red blood cells (RBCs) or a cellular extract (e.g., red blood cellular extract) obtained from the subject. In some embodiments, the level of folate PGs is compared to a threshold level. As a non-limiting example, the threshold can be a level of about 100, 200, 300, 400, 500, 600, 700, 800, 900, 1000, 1100, 1200, 1300, 1400, 1500, or 1600 nmol/L RBCs. One skilled in the art will appreciate that the optimal threshold level may be slightly higher or lower depending on the indication. In certain instances, a level of folate PGs less than the threshold level indicates that the subject has a high likelihood of responding to MTX. In certain other instances, a level of folate PGs greater than the threshold level indicates that the subject does not have a high likelihood of responding to MTX. The level of folate PGs present in a sample may be determined using a radioassay such as a competitive protein binding radioassay available from BioRad (Hercules, CA). However, one skilled in the art will know of additional techniques for determining the concentration of folate PGs in a sample.

In a further aspect, the present invention provides an assay method for optimizing dose efficacy in a subject receiving MTX, the method comprising:
(a) determining a level of MTXPGs in a sample from the subject; and
(b) recommending a subsequent dose of MTX based upon the level of MTXPGs.

The subject typically has a disease or disorder in which MTX may provide some therapeutic benefit such as an inflammatory disease, an autoimmune disease, or cancer. For example, the subject may have rheumatoid arthritis. In some embodiments, the level of at least one long-chain MTXPG is determined. Non-limiting examples of long-chain MTXPGs include MTXPG₃, MTXPG₄, MTXPG₅ [SEQ ID NO:26], MTXPG₆ [SEQ ID NO:27], MTXPG₇ [SEQ ID NO:28], and a combination thereof. The level of MTXPGs such as long-chain MTXPGs is usually determined in red blood cells (RBCs) or a cellular extract (e.g., red blood cellular extract) obtained from the subject. Preferably, the level of MTXPGs is determined using an HPLC-fluorometry procedure with a post-column photo-oxidation technique. Alternatively, the level of MTXPGs can be determined using mass spectrometry.

In some embodiments, the level of MTXPGs is compared to a level of MTXPGs from the subject at an earlier time. The earlier measurement of MTXPG levels can occur, for example, at a time of about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, or more months before the subsequent measurement of MTXPG levels. In certain instances, an increase or the absence of any change in the level of MTXPGs over the time period analyzed indicates that the subsequent dose of MTX should be maintained. In certain other instances, a decrease in the level of MTXPGs over the time period analyzed indicates that the subsequent dose of MTX should be increased (*e.g*., to high dose MTX therapy) or an alternative therapy should be administered. As a non-limiting example, the subsequent dose of MTX can be increased by about 2.5 mg/week or a multiple or fraction thereof.

In a related aspect, the present invention provides an assay method for optimizing dose efficacy in a subject receiving MTX, the method comprising:
(a) determining a level of folate PGs in a sample from the subject; and
(b) recommending a subsequent dose of MTX based upon the level of folate PGs.

The subject typically has a disease or disorder in which MTX may provide some therapeutic benefit such as an inflammatory disease, an autoimmune disease, or cancer. For example, the subject may have rheumatoid arthritis. The level of folate PGs is usually determined in red blood cells (RBCs) or a cellular extract (e.g., red blood cellular extract) obtained from the subject. The level of folate PGs present in a sample may be determined using a radioassay such as a competitive protein binding radioassay.

In some embodiments, the level of folate PGs is compared to a level of folate PGs from the subject at an earlier time. The earlier measurement of folate PG levels can occur, for example, at a time of about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, or more months before the subsequent measurement of folate PG levels. In certain instances, a decrease in the level of folate PGs over the time period analyzed indicates that the subsequent dose of MTX should be maintained. In certain other instances, an increase or the absence of any change in the level of folate PGs over the time period analyzed indicates that the subsequent dose of MTX should be increased (e.g., to high dose MTX therapy) or an alternative therapy should be administered. As a non-limiting example, the subsequent dose of MTX can be increased by about 2.5 mg/week or a multiple or fraction thereof.

In some aspects, the present invention further provides systems and kits for predicting or optimizing the response to MTX in a subject comprising:
(a) a genotypic profile module for genotyping the subject at a polymorphic site in at least one folate pathway gene; and
(b) a pharmacogenetic profile module for generating an efficacy index based upon the genotype of the at least one folate pathway gene.

In other aspects, the present invention further provides systems and kits for predicting or reducing toxicity to MTX in a subject comprising:
(a) a genotypic profile module for genotyping the subject at a polymorphic site in at least one folate pathway gene; and
(b) a pharmacogenetic profile module for generating a toxicogenetic index based upon the genotype of the at least one folate pathway gene.

The subject typically has a disease or disorder in which MTX may provide some therapeutic benefit such as an inflammatory disease, an autoimmune disease, or cancer. For example, the subject may have rheumatoid arthritis.

The genotypic profile module may comprise any device or apparatus suitable for genotyping the subject at a polymorphic site in at least one folate pathway gene. Examples of genotypic profile modules include, without limitation, microarrays such as oligonucleotide or polynucleotide arrays, polymerase chain reaction (PCR)-based devices, sequencing apparatuses, electrophoretic apparatuses and bioinformatic software. Preferably, the genotypic profile module is a microarray. In certain instances, the array comprises a plurality of nucleic acid probes which hybridize to at least one of the folate pathway genes. The pharmacogenetic profile module may comprise any device or apparatus suitable for generating an efficacy index to predict or optimize MTX response and/or a toxicogenetic index to predict or reduce MTX toxicity. Examples of pharmacogenetic profile modules include, without limitation, computer software programs capable of performing algorithmic calculations to generate pharmacogenetic indexes and computers containing such software programs. In certain instances, the algorithm is based upon the presence or absence of a specific genotype (*e.g*., risk genotype) in one or more folate pathway genes. One skilled in the art will know of additional genotypic profile modules and pharmacogenetic profile modules suitable for use in the systems of the present invention. In some embodiments, the genotypic profile module is suitable for genotyping at least two, three, four, five, six, seven, eight, nine, ten, or more folate pathway genes.

The kits of the present invention may further comprise directions for use of the genotypic profile module and the pharmacogenetic profile module. Suitable genotypic profile modules and pharmacogenetic profile modules for use in the kits are described above. Preferably, the genotypic profile module is a microarray such as an oligonucleotide or polynucleotide array.

### IV. Methotrexate Therapy

Methotrexate (MTX) is well known in the art as an inhibitor of dihydrofolate reductase (DHFR), which acts to decrease production of tetrahydrofolate (THF) from dihydrofolate (DHF). As a consequence, MTX indirectly inhibits purine and thymidine synthesis and amino acid interconversion. MTX also exhibits anti-proliferative activity through inhibition of thymidylate synthesis, which is required to synthesize DNA (Calvert, Semin. Oncol., 26:3-10 (1999)). MTX, its synthesis, and its properties are described in further detail in U.S. Patent Nos. 2,512,572; 3,892,801; 3,989,703; 4,057,548; 4,067,867; 4,079,056; 4,080,325; 4,136,101; 4,224,446; 4,306,064; 4,374,987; 4,421,913; and 4,767,859. Methods for using MTX to treat cancer are described, for example, in U.S. Patent Nos. 4,106,488; 4,558,690; and 4,662,359.

MTX, which is useful in the treatment of a variety of inflammatory diseases, autoimmune diseases, and cancers, can be administered by oral or parenteral routes. The drug is readily distributed to body tissues, where it is transported into cells by a specific carrier system that includes components such as the reduced folate carrier (RFC-1) and the folate receptor. Due to its high polarity at physiological pH, MTX does not readily pass through the cell membrane, and the majority of the drug enters cells via specific carriers. Once inside the cell, MTX is converted to methotrexate polyglutamates (MTXPGs) by specific enzymes such as folylpolyglutamate synthase (FPGS), which adds one or more glutamic acid moieties, linked by iso-peptidic bonds to the γ-carboxyl of MTX as described, *e.g.,* in Kamen, Semin. Oncol., S 18:30-39 (1997).

The methods of the present invention can also be used to predict or optimize the response and/or risk associated with MTX analogs or other polyglutamylatable anti-folate compounds. As used herein, the term "methotrexate analog" or "MTX analog"refers to a compound having structural and functional similarity to MTX. MTX analogs are functionally characterized, in part, by their inhibitory activity against dihydrofolate reductase. A MTX analog useful in the present invention acts as a substrate for polyglutamation in a cell by an enzyme such as FPGS. MTX analogs include, without limitation, 4-amino derivatives with halogen substitution on the para-aminobenzoic moiety, such as dichloromethotrexate *(see, e.g.,* Frei et al., Clin. Pharmacol. Therap., 6:160-71 (1965)); 7-methyl substituted MTX *(see, e.g.,* Rosowsky et al., J. Med. Chem., 17:1308-11 (1974)); 3',5'-difluoro MTX, (*see, e.g.,* Tomcuf, J. Organic Chem., 26:3351 (1961)); 2' and 3' monofluorinated derivatives of aminopterin (*see, e.g.,* Henkin et al., J. Med. Chem., 26:1193-1196 (1983)); and 7,8-dihydro-8-methyl-MTX *(see, e.g.,* Chaykovsky, J. Org. Chem., 40:145-146 (1975)).

As used herein, the term "anti-folate" refers to a compound having structural similarity to folate and activity as a folate antagonist against one or more folate-dependent enzymes. Polyglutamylatable anti-folate compounds are anti-folate compounds that can be polyglutamated in a cell by an enzyme such as FPGS. Examples of polyglutamylatable anti-folate compounds include, without limitation, aminopterin, raltitrexed, lometrexol, multitargeted anti-folate (MTA), AQA, MTX, and analogs thereof. Aminopterin, for example, possesses a hydrogen instead of a methyl group at position N-10 compared to the structure of methotrexate. Raltitrexed is a selective inhibitor of thymidylate synthase as described, *e.g.,* in Kamen, *supra.* Lometrexol selectively inhibits glycinamide ribonucleotide formyltransferase, the first enzyme involved in the pathway of *de novo* purine synthesis as described, for example, in Calvert, *supra.* Multitargeted anti-folate (MTA) is an inhibitor of multiple folate-dependent enzymes, such as dihydrofolate reductase, thymidylate synthase, and glycinamide ribonucleotide formyltransferase *(see, e.g.,* Calvert, *supra).* Other anti-folate compounds suitable for use in the presence invention include, for example, edetrexate, lomotrexol, BW1843U89, and ZD1694. In certain instances, MTX is used in a combination therapy with one or more MTX analogs and/or other polyglutamylatable anti-folate compounds. The skilled person in the art understands that the methods of the present invention can be used to predict or optimize the response and/or risk associated with MTX analogs or other polyglutamylatable anti-folate compounds in the same manner as disclosed herein for MTX.

Rheumatoid arthritis and a variety of other inflammatory diseases or autoimmune disorders such as psoriasis, systemic lupus erythematosus, and graft-versus-host disease are typically treated with low dose MTX therapy. Any of a variety of cancers can be treated with either low dose or high dose MTX therapy. In one embodiment, the present invention provides methods for predicting the response to MTX therapy and/or risk associated with MTX therapy prior to administration of the drug. As a non-limiting example, a low starting dose of MTX can be recommended for those subjects who have a high likelihood of responding to MTX and/or are at high risk of developing toxicity to MTX. Alternatively, a high starting dose of MTX can be recommended for those subjects who do not have a high likelihood of responding to MTX and/or are not at high risk of developing toxicity to MTX. In another embodiment, the present invention provides methods for optimizing the response to MTX therapy and/or reducing the risk associated with MTX therapy in a subject undergoing MTX therapy. For example, a low subsequent dose of MTX can be recommended for those subjects who are experiencing any of the side-effects associated with MTX therapy. Alternatively, a high subsequent dose of MTX can be recommended for those subjects who are not responding to MTX therapy.

As used herein, the term "low dose MTX therapy" refers to administration of MTX to a subject at a dose that is less than about 40 mg/m² of body surface per week. Typically, low dose MTX therapy is administered orally at a dose in the range of from about 2.5 mg/m² to about 40 mg/m² of body surface per week, for example, from about 2.5 mg/m² to about 25 mg/m² of body surface per week, depending upon the condition being treated. The term "high dose MTX therapy" as used herein refers to administration of MTX to a subject at a dose that is at least about 40 mg/m² of body surface per day, for example, at least about 50, 100, 250, 500, 750, 1000, 1500, 3000, or 5000 mg/m² of body surface per day. One skilled in the art understands that a high dose MTX therapy is frequently used as an anti-cancer therapeutic and can be administered at doses up to about 5 g/m² of body surface per day or higher depending upon the condition or disease being treated. One skilled in the art recognizes that the doses of MTX typically used in high dose MTX therapy can be administered, for example, intravenously or orally and that such high dose MTX therapy generally requires a period of recovery, which can include leucovorin rescue or another form of folate replacement. The term "intermediate dose MTX therapy" refers to administration of MTX to a subject at a dose that is typically between a low dose and a high dose of MTX, for example, between about 40 mg/m² of body surface per week to about 40 mg/m² of body surface per day.

It will be understood that the dosage ranges of MTX set forth above in the definitions of low, intermediate, and high dose MTX therapy are generalized with respect to treatment of a variety of diseases and that the range of MTX dose that is administered for one disease can differ from the range administered for another. Accordingly, a dose of at least about 40 mg/m² of body surface per day, although generally considered high dose MTX therapy, may be considered by those skilled in the art of cancer therapy as a relatively low dose for treating cancer. Similarly, a dose of about 30 mg/m² of body surface per week, although generally considered as low dose MTX therapy, may be considered by those skilled in the art of rheumatology as a relatively high dose for treating rheumatoid arthritis.

### V. Folate Pathway Genes

In certain aspects, the methods of the present invention rely on genotyping a subject to determine the genotype of at least one folate pathway gene. Non-limiting examples of folate pathway genes include folate-dependent enzyme genes *(e.g., MTHFR, ATTC, TS, SHMT1, GGH*) and homocysteine remethylation-dependent enzyme genes *(e.g., MS, MTRR).* In some embodiments, the presence or absence of risk genotypes in at least one folate pathway gene is used in generating a pharmacogenetic index such as an efficacy index or a toxicogenetic index. Alternatively, any wild-type, heterozygous, or homozygous genotype for a variant allele of interest can be used in generating a pharmacogenetic index according to the methods of the present invention, irrespective of whether it corresponds to a risk genotype.

5,10-methylenetetrahydrofolate reductase (MTHFR) catalyzes the conversion of 5,10-methylenetetrahydrofolate to 5-methyltetrahydrofolate and is described in, *e.g*., Goyette et al., Nat. Genet., 7:195-200 (1994) and Goyette et al., Mamm. Genome, 9:652-656 (1998). The human *MTHFR* coding sequence is available as Genbank accession NM_005957 and the genomic *MTHFR* sequence is available as Genbank accession AY338232.

A variant allele at a polymorphic site in the *MTHFR* gene is located within the *MTHFR* locus, which includes coding regions of the *MTHFR* gene as well as non-coding regions such as introns and 5' and 3' untranslated regions. One skilled in the art understands that such a variant allele can be at a polymorphic site within, for example, the *MTHFR* coding sequence, a promoter region 5' of the *MTHFR* coding sequence, an enhancer region 5' or 3' of the *MTHFR* coding sequence, an intronic sequence, or an mRNA stability region 3' of the *MTHFR* coding sequence. The *MTHFR* genotype typically comprises a wild-type, heterozygous, or homozygous genotype for the variant allele. For example, the *MTHFR* coding sequence at nucleotide 677 can comprise a wild-type genotype (*MTHFR* 677C/C), a heterozygous C to T mutation *(MTHFR* 677C/T), or a homozygous C to T mutation (*MTHFR* 677T/T). Alternatively, the *MTHFR* coding sequence at nucleotide 1298 can comprise a wild-type genotype (*MTHFR* 1298A/A), a heterozygous A to C mutation *(MTHFR* 1298A/C), or a homozygous A to C mutation (*MTHFR* 1298C/C). One skilled in the art will appreciate that genotypes for other *MTHFR* variant alleles (*e.g.,* 167G/A, 482G/A, 559C/T, 692C/T, 764C/T, 792+1G/A, 985C/T, 1015C/T, 1081C/T, 1317T/C) are also within the scope of the present invention.

In certain instances, the presence of a particular *MTHFR* risk genotype is indicative of increased MTHFR levels or activity. Alternatively, the presence of a particular *MTHFR* risk genotype is indicative of decreased MTHFR levels or activity. As such, the presence or absence of a particular *MTHFR* risk genotype can be correlated to the likelihood of response and/or the risk associated with MTX therapy, which in turn can be used to determine a course of therapy with therapeutic efficacy and/or minimal side-effects (*e.g.,* low dose MTX therapy, high dose MTX therapy, or an alternative therapy). For example, the presence or absence of the *MTHFR* 677T/T risk genotype can be used to calculate an efficacy index, which can be useful for providing an indication as to whether a subject is likely to respond to MTX. Alternatively, the presence or absence of the *MTHFR* 677T/T and/or *MTHFR* 1298A/C or C/C risk genotype can be used to calculate a toxicogenetic index, which can be useful for providing an indication as to whether a subject is at risk of developing toxic side-effects to MTX.

5-aminotmidazole-4-carboxamide ribonucleotide transformylase (ATIC) is a bifunctional enzyme catalyzing the last two steps in the *de novo* purine biosynthetic nucleotide pathway and is described in, *e.g.,* Rayl et al., J. Biol. Chem., 271:2225-2233 (1996) and Vergis et al., J. Biol. Chem., 276:7727-7733 (2001). The human *ATIC* coding sequence is available as GenBank accession NM_004044 and the human A TIC genomic sequence is available as GenBank accession NT_005403.

A variant allele at a polymorphic site in the *ATIC* gene is located within the *ATIC* locus, which includes coding regions of the *ATIC* gene as well as non-coding regions such as introns and 5' and 3' untranslated regions. One skilled in the art understands that such a variant allele can be at a polymorphic site within, for example, the *ATIC* coding sequence, a promoter region 5' of the *ATIC* coding sequence, an enhancer region 5' or 3' of the *ATIC* coding sequence, an intronic sequence, or an mRNA stability region 3' of the *ATIC* coding sequence. The *ATIC* genotype typically comprises a wild-type, heterozygous, or homozygous genotype for the variant allele. For example, the *ATIC* coding sequence at nucleotide 347 can comprise a wild-type genotype (*ATIC* 347C/C), a heterozygous C to G mutation *(ATIC* 347C/G), or a homozygous C to G mutation *(ATIC* 347G/G). One skilled in the art will appreciate that genotypes for other *ATIC* variant alleles are also within the scope of the present invention.

In certain instances, the presence of a particular *ATIC* risk genotype is indicative of increased *ATIC* levels or activity. Alternatively, the presence of a particular *ATIC* risk genotype is indicative of decreased ATIC levels or activity. As such, the presence or absence of a particular *ATIC* risk genotype can be correlated to the likelihood of response and/or the risk associated with MTX therapy, which in turn can be used to determine a course of therapy with therapeutic efficacy and/or minimal side-effects. For example, the presence or absence of the *ATIC* 347G/G risk genotype can be used to calculate a toxicogenetic index, which can be useful for providing an indication as to whether a subject is at risk of developing toxic side-effects to MTX.

Thymidylate synthase (TS) methylates deoxyuridine monophosphate to produce deoxythymidylate and is described in, *e.g.,* Takeishi et al., Nucleic Acids Res., 13:2035-2043 (1985) and Kaneda et al., J. Biol. Chem., 265:20277-20284 (1990). The human *TS* coding sequence is available as GenBank accession NM_001071 and the human *TS* genomic sequence is available as GenBank accession D00596.

A variant allele at a polymorphic site in the *TS* gene is located within the *TS* locus, which includes coding regions of the *TS* gene as well as non-coding regions such as introns and 5' and 3' untranslated regions. One skilled in the art understands that such a variant allele can be at a polymorphic site within, for example, the *TS* coding sequence, a promoter region 5' of the *TS* coding sequence, an enhancer region 5' or 3' of the *TS* coding sequence, an intronic sequence, or an mRNA stability region 3' of the *TS* coding sequence. The *TS* genotype typically comprises a wild-type, heterozygous, or homozygous genotype for the variant allele. For example, the *TS* promoter sequence can comprise a wild-type genotype having two copies of three 28 base pair tandem repeats *(TS* *3/*3), a heterozygous genotype having one copy of three 28 base pair tandem repeats and one copy of two 28 base pair tandem repeats *(TS* *3/*2), or a homozygous genotype having two copies of two 28 base pair tandem repeats *(TS* *2/*2) *(see, e.g.,* Kaneda et al., Nucleic Acids Res., 15:1259-1270 (1987)). One skilled in the art will appreciate that genotypes for other *TS* variant alleles (e.g., 1494del6, IVS6 -68C/T, 1122A/G, 1053C/T) are also within the scope of the present invention.

In certain instances, the presence of a particular *TS* risk genotype is indicative of increased TS levels or activity. Alternatively, the presence of a particular *TS* risk genotype is indicative of decreased TS levels or activity. As such, the presence or absence of a particular *TS* risk genotype can be correlated to the likelihood of response and/or the risk associated with MTX therapy, which in turn can be used to determine a course of therapy with therapeutic efficacy and/or minimal side-effects. For example, the presence or absence of the *TS* *2/*2 risk genotype can be used to calculate an efficacy index, which can be useful for providing an indication as to whether a subject is likely to respond to MTX. Alternatively, the presence or absence of the *TS* *2/*2 risk genotype can be used to calculate a toxicogenetic index, which can be useful for providing an indication as to whether a subject is at risk of developing toxic side-effects to MTX.

Serine hydroxymethyltransferase (SHMT) catalyzes the reversible conversion of serine and tetrahydrofolate to glycine and methylenetetrahydrofolate and is described in, *e.g*., Garrow et al., J. Biol. Chem., 268:11910-11916 (1993) and Girgis et al., Gene, 210:315-324 (1998). Two distinct SHMT isoenzymes have been identified, one in the cytosol localized to the *SHMT1* gene on chromosome 17p11.2 and another in the mitochondrion localized to the *SHMT2* gene on chromosome 12q13.2. SHMT1 plays a pivotal role in providing one-carbon units for purine, thymidylate, and methionine synthesis, in addition to other metabolic functions. The human *SHMT1* coding sequence is available as Genbank accession NM_004169 and NM_148918.

A variant allele at a polymorphic site in the *SHMT1* gene is located within the *SHMT1* locus, which includes coding regions of the *SHMT1* gene as well as non-coding regions such as introns and 5' and 3' untranslated regions. One skilled in the art understands that such a variant allele can be at a polymorphic site within, for example, the *SHMT1* coding sequence, a promoter region 5' of the *SHMT1* coding sequence, an enhancer region 5' or 3' of the *SHMT1* coding sequence, an intronic sequence, or an mRNA stability region 3' of the *SHMT1* coding sequence. The *SHMT1* genotype typically comprises a wild-type, heterozygous, or homozygous genotype for the variant allele. For example, the *SHMT1* coding sequence at nucleotide 1420 can comprise a wild-type genotype *(SHMT1* 1420C/C), a heterozygous C to T mutation (*SHMT1* 1420C/T), or a homozygous C to T mutation *(SHMT1* 1420T/T). One skilled in the art will appreciate that genotypes for other *SHMT1* variant alleles are also within the scope of the present invention.

In certain instances, the presence of a particular *SHMT1* risk genotype is indicative of increased SHMT1 levels or activity. Alternatively, the presence of a particular *SHMT1* risk genotype is indicative of decreased SHMT1 levels or activity. As such, the presence or absence of a particular *SHMT1* risk genotype can be correlated to the likelihood of response and/or the risk associated with MTX therapy, which in turn can be used to determine a course of therapy with therapeutic efficacy and/or minimal side-effects. For example, the presence or absence of the *SHMT1* 1420C/T or T/T risk genotype can be used to calculate an efficacy index, which can be useful for providing an indication as to whether a subject is likely to respond to MTX. Alternatively, the presence or absence of the *SHMT1* 1420C/C risk genotype can be used to calculate a toxicogenetic index, which can be useful for providing an indication as to whether a subject is at risk of developing toxic side-effects to MTX.

Gamma-glutamyl hydrolase (GGH) is known in the art and is described in, *e.g.,* Yao et al., Proc. Natl. Acad. Sci., 93:10134-10138 (1996)). The human *GGH* coding sequence is available as Genbank accession U55206.

A variant allele at a polymorphic site in a *GGH* gene is located within the *GGH* locus, which includes coding regions of the *GGH* gene as well as non-coding regions such as introns and 5' and 3' untranslated regions. One skilled in the art understands that such a variant allele can be at a polymorphic site within, for example, the *GGH* coding sequence, a promoter region 5' of the *GGH* coding sequence, an enhancer region 5' or 3' of the *GGH* coding sequence, a *GGH* intronic sequence, or an mRNA stability region 3' of the *GGH* coding sequence. The *GGH* genotype typically comprises a wild-type, heterozygous, or homozygous genotype for the variant allele. For example, the promoter region 5' of the *GGH* coding sequence can comprise a wild-type genotype *(GGH* -401C/C), a heterozygous C to T mutation (*GGH* -401C/T), or a homozygous C to T mutation (*GGH* -401T/T). One skilled in the art will appreciate that genotypes for other *GGH* variant alleles are also within the scope of the present invention.

In certain instances, the presence of a particular *GGH* risk genotype is indicative of increased GGH levels or activity. Alternatively, the presence of a particular *GGH risk* genotype is indicative of decreased GGH levels or activity. As such, the presence or absence of a particular *GGH risk* genotype can be correlated to the likelihood of response and/or the risk associated with MTX therapy, which in turn can be used to determine a course of therapy with therapeutic efficacy and/or minimal side-effects. For example, the presence or absence of the *GGH* -401C/C risk genotype can be used to calculate a toxicogenetic index, which can be useful for providing an indication as to whether a subject is at risk of developing toxic side-effects to MTX.

Methionine synthase (MS), also known as 5-methyltetrahydrofolate-homocysteine methyltransferase or cobalamin-dependent methionine synthase, catalyzes the final step in methionine biosynthesis and is described in, *e.g.,* Li et al., Hum. Mol. Genet., 5:1851-1858 (1996) and Leclerc et al., Hum. Mol. Genet., 5:1867-1874 (1996). The human *MS* coding sequence is available as GenBank accession NM_000254.

A variant allele at a polymorphic site in the *MS* gene is located within the *MS* locus, which includes coding regions of the *MS* gene as well as non-coding regions such as introns and 5' and 3' untranslated regions. One skilled in the art understands that such a variant allele can be at a polymorphic site within, for example, the *MS* coding sequence, a promoter region 5' of the *MS* coding sequence, an enhancer region 5' or 3' of the *MS* coding sequence, an intronic sequence, or an mRNA stability region 3' of the *MS* coding sequence. The *MS* genotype typically comprises a wild-type, heterozygous, or homozygous genotype for the variant allele. For example, the *MS* coding sequence at nucleotide 2756 can comprise a wild-type genotype (*MS* 2756A/A), a heterozygous A to G mutation (*MS* 2756A/G), or a homozygous A to G mutation (MS 2756G/G). One skilled in the art will appreciate that genotypes for other *MS* variant alleles are also within the scope of the present invention.

In certain instances, the presence of a particular *MS* risk genotype is indicative of increased *MS* levels or activity. Alternatively, the presence of a particular *MS* risk genotype is indicative of decreased MS levels or activity. As such, the presence or absence of a particular *MS* risk genotype can be correlated to the likelihood of response and/or the risk associated with MTX therapy, which in turn can be used to determine a course of therapy with therapeutic efficacy and/or minimal side-effects. For example, the presence or absence of the *MS* 2756A/A risk genotype can be used to calculate a toxicogenetic index, which can be useful for providing an indication as to whether a subject is at risk of developing toxic side-effects to MTX.

Methionine synthase reductase (MTRR), a member of the ferredoxin-NADP(+) reductase family of electron transferases, catalyzes the regeneration of methylcobalamin, a cofactor of methionine synthase and is described in, *e.g.,* Leclerc et al., Proc. Natl. Acad. Sci. U.S.A., 95:3059-3064 (1998) and Leclerc et al., Gene, 240:75-88 (1999). The human *MTRR* coding sequence is available as GenBank accession NM_024010 and NM_002454.

A variant allele at a polymorphic site in the *MTRR* gene is located within the *MTRR* locus, which includes coding regions of the *MTRR* gene as well as non-coding regions such as introns and 5' and 3' untranslated regions. One skilled in the art understands that such a variant allele can be at a polymorphic site within, for example, the *MTRR* coding sequence, a promoter region 5' of the *MTRR* coding sequence, an enhancer region 5' or 3' of the *MTRR* coding sequence, an intronic sequence, or an mRNA stability region 3' of the *MTRR* coding sequence. The *MTRR* genotype typically comprises a wild-type, heterozygous, or homozygous genotype for the variant allele. For example, the *MTRR* coding sequence at nucleotide 66 can comprise a wild-type genotype (*MTRR* 66A/A), a heterozygous A to G mutation (*MTRR* 66A/G), or a homozygous A to G mutation *(MTRR* 66G/G). One skilled in the art will appreciate that risk genotypes for other *MTRR* variant alleles are also within the scope of the present invention.

In certain instances, the presence of a particular *MTRR* risk genotype is indicative of increased MTRR levels or activity. Alternatively, the presence of a particular *MTRR* risk genotype is indicative of decreased MTRR levels or activity. As such, the presence or absence of a particular *MTRR* risk genotype can be correlated to the likelihood of response and/or the risk associated with MTX therapy, which in turn can be used to determine a course of therapy with therapeutic efficacy and/or minimal side-effects. For example, the presence or absence of the *MTRR* 66G/G risk genotype can be used to calculate a toxicogenetic index, which can be useful for providing an indication as to whether a subject is at risk of developing toxic side-effects to MTX.

### VI. Pharmacogenetic Indexes

In some aspects, the present invention provides assay methods for predicting, monitoring, or optimizing MTX therapy in a subject by generating a pharmacogenetic index based upon a genotypic analysis of at least one folate pathway gene in a sample from the subject. Any of a variety of methods or algorithms for generating various pharmacogenetic indexes can be used in the methods of the present invention. In some embodiments, the pharmacogenetic index is calculated as either the sum of or the difference between the number of variant alleles at one or more polymorphic sites. For example, if a subject is heterozygous for a variant allele at a polymorphic site (*i.e*., having 1 copy of the variant allele), the variant allele can contribute a value of 1 to the pharmacogenetic index. Likewise, if a subject is homozygous for a variant allele at a polymorphic site *(i.e.,* having 2 copies of the variant allele), the variant allele can contribute a value of 2 to the pharmacogenetic index. If a subject is wild-type at a polymorphic site, there is no contribution from the variant allele to the pharmacogenetic index. In other embodiments, the pharmacogenetic index is calculated as either the sum of or the difference between the number of homozygous variant alleles at one or more polymorphic sites. For example, if a subject is homozygous for a variant allele at a polymorphic site, the variant allele can contribute a value of 1 to the pharmacogenetic index. In this algorithm, if a subject is wild-type or heterozygous at a polymorphic site, there is no contribution from the variant allele to the pharmacogenetic index.

In certain instances, the pharmacogenetic index is a toxicogenetic index, which can be generated to evaluate the risk of MTX toxicity prior to therapy or to reduce the toxic side-effects associated with MTX during therapy. In one embodiment, the toxicogenetic index can be calculated as the sum of or the difference between the number of wild-type, heterozygous, or homozygous genotypes for at least one folate pathway gene. As a non-limiting example, the toxicogenetic index can be calculated as the sum of the number of wild-type, heterozygous, or homozygous genotypes for the *MTHFR, ATIC, TS,* and/or *SHMT1* genes. Preferably, at least two, three, or all four of these genes are genotyped in a panel and used to calculate the toxicogenetic index. As such, in certain embodiments, a toxicogenetic index for predicting, monitoring, or optimizing MTX therapy can be calculated by adding together the values assigned to one or more of these genes as follows: (1) the *MTHFR* gene is assigned a value of 1 when an *MTHFR* 677T/T risk genotype is present or a value of 0 when an *MTHFR* 677C/C or C/T genotype is present; (2) the *ATIC* gene is assigned a value of 1 when an *ATIC* 347G/G risk genotype is present or a value of 0 when an *ATIC* 347C/C or C/G genotype is present; (3) the *TS* gene is assigned a value of 1 when a *TS* *2/*2 risk genotype is present or a value of 0 when a *TS* *3/*3 or *3/*2 genotype is present; and/or (4) the *SHMT1* gene is assigned a value of 1 when a *SHMT1* 1420C/C risk genotype is present or a value of 0 when a *SHMT1* 1420T/T or C/T genotype is present. As a non-limiting example, the toxicogenetic index can be calculated according to the formula: *MTHFR* value + *ATIC* value + *TS* value *+ SHMT1* value *(see, e.g.,* Example 1). However, one skilled in the art will understand that any combination of these genes, including other genes having risk genotypes, can be genotyped and used to calculate the toxicogenetic index in an alternative algorithm.

As another non-limiting example, the toxicogenetic index can be calculated as the sum of the number of wild-type, heterozygous, or homozygous genotypes for the *GGH, ATIC, MTHFR, MTRR,* and/or *MS* genes. Preferably, at least two, three, four, or all five of these genes are genotyped and used in a panel to calculate the toxicogenetic index. As such, in certain instances, a toxicogenetic index for predicting, monitoring, or optimizing MTX therapy can be calculated by adding together the values assigned to one or more of these genes as follows: (1) the *GGH* gene is assigned a value of 1 when a *GGH* -401C/C risk genotype is present or a value of 0 when a *GGH* -401T/T or C/T genotype is present; (2) the *ATIC* gene is assigned a value of 1 when an *ATIC* 347G/G risk genotype is present or a value of 0 when an *ATIC* 347C/C or C/G genotype is present; (3) the *MTHFR* gene is assigned a value of 1 when an *MTHFR* 1298A/C or C/C risk genotype is present or a value of 0 when an *MIHFR* 1298A/A genotype is present; (4) the *MTRR* gene is assigned a value of 1 when an *MTRR* 66G/G risk genotype is present or a value of 0 when an *MTRR* 66A/A or A/G genotype is present; and/or (5) the MS gene is assigned a value of 1 when an *MS* 2756A/A risk genotype is present or a value of 0 when an *MS* 2756A/G or G/G genotype is present. As a non-limiting example, the toxicogenetic index can be calculated according to the formula: *GGH* value *+ ATIC* value + *MTHFR* value + *MTRR* value + *MS* value *(see, e.g.,* Example 2). However, one skilled in the art will understand that any combination of these genes, including other genes having risk genotypes, can be genotyped and used to calculate the toxicogenetic index in an alternative algorithm.

In certain other instances, the pharmacogenetic index is an efficacy index, which is calculated to evaluate the likelihood of response to MTX prior to therapy or to optimize the dose efficacy of MTX during therapy. In one embodiment, the efficacy index is calculated as the sum of or the difference between the number of wild-type, heterozygous, or homozygous genotypes for at least one folate pathway gene. As a non-limiting example, the efficacy index can be calculated as the sum of the number of wild-type, heterozygous, or homozygous genotypes for the *MTHFR, TS,* and/or *SHMT1* genes. Preferably, at least two or all three of these genes are genotyped and used in a panel to calculate the efficacy index. As such, in certain instances, an efficacy index for predicting, monitoring, or optimizing MTX therapy can be calculated by adding together the values assigned to one or more of these genes as follows: (1) the *MTHFR* gene is assigned a value of 1 when an *MTHFR* 677T/T risk genotype is present or a value of 0 when an *MTHFR* 677C/C or C/T genotype is present; (2) the *TS* gene is assigned a value of 1 when a *TS* *2/*2 risk genotype is present or a value of 0 when a *TS* *3/*3 or *3/*2 genotype is present; and/or (3) the *SHMT1* gene is assigned a value of 1 when a *SHMT1* 1420C/T or T/T risk genotype is present or a value of 0 when a *SHMT1* 1420C/C genotype is present. As a non-limiting example, the efficacy index can be calculated according to the formula: *MTHFR* value + *TS* value + *SHMT1* value (*see, e.g.,* Example 2). However, one skilled in the art will understand that any combination of these genes, including other genes having risk genotypes, can be genotyped and used to calculate the efficacy index in an alternative algorithm.

The present invention is not limited to the foregoing methods or algorithms for generating a pharmacogenetic index such as a toxicogenetic index or an efficacy index. Using other statistical analyses, a pharmacogenetic index can be calculated. These methods include, for example, identifying the presence or absence of wild-type, heterozygous, or homozygous genotypes in other genes including, without limitation, influx/efflux transporters such as multidrug resistance proteins (*e.g., MRP2*); aldehyde oxidase; purine synthesis genes such as glutamine PRPP amidotransferase, glycinamide ribonucleotide (GAR) synthetase, formylglycinamide ribonucleotide (FGAR) amidotransferase, formylglycinamidine ribonucleotide (FGAM) cyclase, 5-aminoimidazole ribonucleotide (AIR) carboxylase, N-succinylo-5-aminoimidazole-4-carboxamide ribonucleotide (SAICAR) synthetase, SAICAR lyase, IMP synthase, adenylosuccinate synthetase, adenylosuccinate lyase, IMP dehydrogenase, and XMP-glutamine amidotransferase; pyrimidine synthesis genes such as ribonucleotide reductase, nucleoside diphosphate kinase, deaminase, deoxyuridine triphosphatase, aspartate transcarbamoylase, dihydroorotase, dihydroorotate dehydrogenase, orotate phosphoribosyl transferase, orotidylate decarboxylase, and cytidylate synthetase; and combinations thereof. Furthermore, certain genotypes or polymorphic sites can have a weighted contribution such that the importance of wild-type, homozygosity, or heterozygosity at that specific site contributes more weight to the pharmacogenetic index. Other parameters such as phenotypic parameters can also be used in the algorithms.

### VII. Methods of Genotyping

A variety of techniques can be used to genotype a subject at a polymorphic site in at least one folate pathway gene according to the methods of the present invention. For example, enzymatic amplification of nucleic acid from a subject can be conveniently used to obtain nucleic acid for subsequent analysis. However, the presence or absence of a variant allele can also be determined directly from the subject's nucleic acid without enzymatic amplification (*e.g*., using hybridization techniques). Genotyping of nucleic acid from a subject, whether amplified or not, can be performed using any of various techniques known to one of skill in the art. Useful techniques include, without limitation, polymerase chain reaction (PCR)-based analysis, sequence analysis, array-based analysis, and electrophoretic analysis, which can be used alone or in combination.

A nucleic acid sample can be obtained from a subject using routine methods. Such samples comprise any biological matter from which nucleic acid can be prepared. As non-limiting examples, suitable samples include whole blood, serum, plasma, saliva, cheek swab, urine, or other bodily fluid or tissue that contains nucleic acid. In one embodiment, the methods of the present invention are performed using whole blood or fractions thereof such as serum or plasma, which can be obtained readily by non-invasive means and used to prepare genomic DNA. In another embodiment, genotyping involves the amplification of a subject's nucleic acid using PCR. Use of PCR for the amplification of nucleic acids is well known in the art *(see, e.g.,* Mullis et al., The Polymerase Chain Reaction, Birkhäuser, Boston, (1994)). In yet another embodiment, PCR amplification is performed using one or more fluorescently labeled primers. In a further embodiment, PCR amplification is performed using one or more labeled or unlabeled primers containing a DNA minor groove binder. Generally, protocols for the use of PCR in identifying mutations and polymorphisms in a gene of interest are described in Theophilus et al., "PCR Mutation Detection Protocols," Humana Press (2002). Further protocols are provided in Innis et al., "PCR Applications: Protocols for Functional Genomics," 1st Edition, Academic Press (1999).

Any of a variety of different primers can be used to PCR amplify a subject's nucleic acid. For example, the PCR primers decribed in Examples 1 and 2 can be used to amplify the sequence surrounding a particular polymorphic site. As understood by one skilled in the art, additional primers for PCR analysis can be designed based on the sequence flanking the polymorphic site(s) of interest. As a non-limiting example, a sequence primer can contain between about 15 to about 30 nucleotides of a sequence upstream or downstream of the polymorphic site of interest. Such primers generally are designed to have sufficient guanine and cytosine content to attain a high melting temperature which allows for a stable annealing step in the amplification reaction. Several computer programs, such as Primer Select, are available to aid in the design of PCR primers.

A Taqman^{®} allelic discrimination assay available from Applied Biosystems (Foster City, CA) can be useful for genotyping a subject at a polymorphic site and thereby determining the presence or absence of a variant allele. In a Taqman^{®} allelic discrimination assay, a specific, fluorescent dye-labeled probe for each allele is constructed. The probes contain different fluorescent reporter dyes such as FAM and VIC to differentiate the amplification of each allele. In addition, each probe has a quencher dye at one end which quenches fluorescence by fluorescence resonance energy transfer. During PCR, each probe anneals specifically to complementary sequences in the nucleic acid from the subject. The 5' nuclease activity of Taq polymerase is used to cleave only probe that hybridizes to the allele. Cleavage separates the reporter dye from the quencher dye, resulting in increased fluorescence by the reporter dye. Thus, the fluorescence signal generated by PCR amplification indicates which alleles are present in the sample. Mismatches between a probe and allele reduce the efficiency of both probe hybridization and cleavage by Taq polymerase, resulting in little to no fluorescent signal. Those skilled in the art understand that improved specificity in allelic discrimination assays can be achieved by conjugating a DNA minor groove binder (MGB) group to a DNA probe as described, *e.g.,* in Kutyavin et al., Nuc. Acids Res., 28:655-661 (2000). Suitable minor groove binders for use in the present invention include, but are not limited to, compounds such as dihydrocyclopyrroloindole tripeptide (DPI3).

Sequence analysis can also be useful for genotyping a subject at a polymorphic site in at least one folate pathway gene. In one embodiment, a variant allele can be detected by sequence analysis using the appropriate primers, which are designed based on the sequence flanking the polymorphic site of interest, as is known by those skilled in the art. As a non-limiting example, a sequence primer can contain between about 15 to about 30 nucleotides of a sequence between about 40 to about 400 base pairs upstream or downstream of the polymorphic site of interest. Such primers are generally designed to have sufficient guanine and cytosine content to attain a high melting temperature which allows for a stable annealing step in the sequencing reaction.

As used herein, the term "sequence analysis" refers to any manual or automated process by which the order of nucleotides in a nucleic acid is determined. As an example, sequence analysis can be used to determine the nucleotide sequence of a sample of DNA. The term encompasses, without limitation, chemical and enzymatic methods such as dideoxy enzymatic methods including, for example, Maxam-Gilbert and Sanger sequencing as well as variations thereof. The term also encompasses, without limitation, capillary array DNA sequencing, which relies on capillary electrophoresis and laser-induced fluorescence detection and can be performed using instruments such as the MegaBACE 1000 or ABI 3700. As additional non-limiting examples, the term encompasses thermal cycle sequencing (Sears et al., Biotechniques, 13:626-633 (1992)); solid-phase sequencing (Zimmerman et al., Methods Mol. Cell Biol., 3:39-42 (1992); and sequencing with mass spectrometry, such as matrix-assisted laser desorption/ionization time-of-flight mass spectrometry (MALDI-TOF MS; Fu et al., Nature Biotech., 16:381-384 (1998)). The term further includes, without limitation, sequencing by hybridization (SBH), which relies on an array of all possible short oligonucleotides to identify a segment of sequence (Chee et al., Science, 274:610-614 (1996); Drmanac et al., Science, 260:1649-1652 (1993); Drmanac et al., Nature Biotech., 16:54-58 (1998)). One skilled in the art understands that these and additional variations are encompassed by the term as defined herein. *See,* in general, Ausubel et al., Current Protocols in Molecular Biology, Chapter 7 and Supplement 47, John Wiley & Sons, Inc., New York (1999).

In addition, electrophoretic analysis can be useful for genotyping a subject at a polymorphic site in at least one folate pathway gene. The term "electrophoretic analysis," as used herein in reference to one or more nucleic acids such as amplified fragments, refers to a process whereby charged molecules are moved through a stationary medium under the influence of an electric field. Electrophoretic migration separates nucleic acids primarily on the basis of their charge, which is in proportion to their size, with smaller molecules migrating more quickly. The term includes, without limitation, analysis using slab gel electrophoresis such as agarose or polyacrylamide gel electrophoresis, or capillary electrophoresis. Capillary electrophoretic analysis generally occurs inside a small-diameter quartz capillary in the presence of high (kilovolt-level) separating voltages with separation times of a few minutes. Using capillary electrophoretic analysis, nucleic acids are conveniently detected by UV absorption or fluorescent labeling, and single-base resolution can be obtained on fragments up to several hundred base pairs in length. Such methods of electrophoretic analysis, and variations thereof, are well known in the art, as described, for example, in Ausubel et al., Current Protocols in Molecular Biology, Chapter 2 and Supplement 45, John Wiley & Sons, Inc., New York (1999).

Restriction fragment length polymorphism (RFLP) analysis can also be useful for genotyping a subject at a polymorphic site in at least one folate pathway gene (*see, e.g.,* Jarcho et al., Current Protocols in Human Genetics, pages 2.7.1-2.7.5, John Wiley & Sons, Inc., New York; Innis et al., PCR Protocols, San Diego, Academic Press, Inc. (1990)). As used herein, "restriction fragment length polymorphism analysis" refers to any method for distinguishing polymorphic alleles using a restriction enzyme, which is an endonuclease that catalyzes degradation of nucleic acid following recognition of a specific base sequence, generally a palindrome or inverted repeat. One skilled in the art understands that the use of RFLP analysis depends upon an enzyme that can differentiate a variant allele from a wild-type or other allele at a polymorphic site.

Furthermore, allele-specific oligonucleotide hybridization can be useful for genotyping a subject at a polymorphic site in at least one folate pathway gene. Allele-specific oligonucleotide hybridization is based on the use of a labeled oligonucleotide probe having a sequence perfectly complementary, for example, to the sequence encompassing the variant allele. Under appropriate conditions, the variant allele-specific probe hybridizes to a nucleic acid containing the variant allele but does not hybridize to the one or more other alleles, which have one or more nucleotide mismatches as compared to the probe. If desired, a second allele-specific oligonucleotide probe that matches an alternate (*e.g.,* wild-type) allele can also be used. Similarly, the technique of allele-specific oligonucleotide amplification can be used to selectively amplify, for example, a variant allele by using an allele-specific oligonucleotide primer that is perfectly complementary to the nucleotide sequence of the variant allele but which has one or more mismatches as compared to other alleles (Mullis et al., The Polymerase Chain Reaction, Birkhäuser, Boston, (1994)). One skilled in the art understands that the one or more nucleotide mismatches that distinguish between the variant allele and other alleles are often located in the center of an allele-specific oligonucleotide primer to be used in the allele-specific oligonucleotide hybridization. In contrast, an allele-specific oligonucleotide primer to be used in PCR amplification generally contains the one or more nucleotide mismatches that distinguish between the variant allele and other alleles at the 3' end of the primer.

A heteroduplex mobility assay (HMA) is another well-known assay that can be used for genotyping a subject at a polymorphic site in at least one folate pathway gene. HMA is useful for detecting the presence of a variant allele since a DNA duplex carrying a mismatch has reduced mobility in a polyacrylamide gel compared to the mobility of a perfectly base-paired duplex *(see, e.g.,* Delwart et al., Science, 262:1257-1261 (1993); White et al., Genomics, 12:301-306 (1992)).

The technique of single strand conformational polymorphism (SSCP) can also be useful for genotyping a subject at a polymorphic site in at least one folate pathway gene according to the methods of the present invention *(see, e.g.,* Hayashi, Methods Applic., 1:34-38 (1991)). This technique is used to detect variant alleles based on differences in the secondary structure of single-stranded DNA that produce an altered electrophoretic mobility upon non-denaturing gel electrophoresis. Variant alleles are detected by comparison of the electrophoretic pattern of the test fragment to corresponding standard fragments containing known alleles.

Denaturing gradient gel electrophoresis (DGGE) is another useful technique for genotyping a subject at a polymorphic site in at least one folate pathway gene. In DGGE, double-stranded DNA is electrophoresed in a gel containing an increasing concentration of denaturant. Because double-stranded fragments comprising mismatched alleles have segments that melt more rapidly, such fragments migrate differently as compared to perfectly complementary sequences (Sheffield et al., "Identifying DNA Polymorphisms by Denaturing Gradient Gel Electrophoresis," in Innis et al., PCR Protocols, San Diego, Academic Press, Inc. (1990)).

Array-based methods for genotyping a subject at a polymorphic site in at least one folate pathway gene are particularly useful in the methods of the present invention. As used herein, the term "microarray" refers to an array of distinct nucleic acids (*e.g.,* polynucleotides, oligonucleotides, *etc.*) synthesized on a substrate such as paper, membrane (*e.g*., nylon), filter, chip, glass slide, or any other suitable solid support. Microarrays typically comprise a plurality of different nucleic acid probes that are coupled to a surface of a substrate in different known locations. In certain instances, microarrays may be produced using mechanical synthesis methods as described in, *e.g.,* U.S. Patent No. 5,384,261. In certain other instances, microarrays may be produced using light directed synthesis methods which incorporate a combination of photolithographic methods and solid phase oligonucleotide synthesis methods as described in, *e.g.,* Fodor et al., Science, 251:767-777 (1991); and U.S. Patent Nos. 5,143,854 and 5,424,186.

Any of a variety of genotyping techniques using microarrays is within the scope of the present invention. In one embodiment, a subject is genotyped at one or more polymorphic sites using an oligonucleotide probe array. For example, U.S. Patent No. 5,858,659 describes a method for analyzing polymorphic or biallelic markers using arrays of oligonucleotide probes that are capable of discriminating between the wild-type, heterozygous, and homozygous genotypes of genes of interest. In addition, U.S. Patent Publication No. 20050042654 describes a method for analyzing single nucleotide polymorphic sites using arrays of allele-specific oligonucleotide probes. Other genotyping methods using oligonucleotide probe arrays are described in, *e.g.,* U.S. Patent Nos. 5,856,092, 6,300,063, 6,284,460, 6,361,947, and 6,368,799; and U.S. Patent Publication Nos. 20030186279, 20040146890, and 20050074787. In another embodiment, a subject is genotyped at one or more polymorphic sites using a polynucleotide probe array. For example, Flavell et al., Nucl. Acids Res., 31:e115 (2003), describes an array-based method for detecting single nucleotide polymorphisms using biotin-terminated allele-specific PCR products spotted onto streptavidin-coated glass slides and visualized by hybridization of fluorescent detector oligonucleotides to tags attached to the allele-specific PCR primers. In addition, Ji et al., Mut. Res., 548:97-105 (2004), describes an array-based method for detecting single nucleotide polymorphisms using amplified PCR products spotted onto glass slides which are then interrogated by hybridization with dual-color probes. One skilled in the art will know of additional methods for genotyping a subject at one or more polymorphic sites using oligonucleotide or polynucleotide probe arrays.

Other molecular techniques useful for genotyping a subject at a polymorphic site in at least one folate pathway gene are also known in the art and useful in the methods of the present invention. Other well-known genotyping techniques include, without limitation, automated sequencing and RNAase mismatch techniques (Winter et al., Proc. Natl. Acad. Sci., 82:7575-7579 (1985)). Furthermore, one skilled in the art understands that, where the presence or absence of multiple variant alleles is to be determined, individual variant alleles can be detected by any combination of molecular techniques. *See,* in general, Birren et al., Genome Analysis: A Laboratory Manual, Volume 1 (Analyzing DNA), New York, Cold Spring Harbor Laboratory Press (1997). In addition, one skilled in the art understands that multiple variant alleles can be detected in individual reactions or in a single reaction, *e.g.,* using a multiplex real-time PCR assay. Kits for performing multiplex real-time PCR of cDNA or genomic DNA targets using sequence-specific probes are available from QIAGEN Inc. (Valencia, CA), *e.g.,* the QuantiTect Multiplex PCR Kit. Systems for performing multiplex real-time PCR are available from Applied Biosystems (Foster City, CA), *e.g.,* the 7300 or 7500 Real-Time PCR Systems.

In view of the above, one skilled in the art realizes that the methods of the present invention for determining the genotype of at least one folate pathway gene can be practiced using one or any combination of the well-known techniques described above or other techniques known in the art.

### I. Methods of Determining MTXPG Levels

A variety of means can be used to determine a level of methotrexate polyglutamates (MTXPGs) in the methods of the present invention. For example, MTXPG concentrations can be measured by any of the techniques described in Dervieux et al., Clin. Chem., 49:1632-41 (2003) and U.S. Patent Publication Nos. 20040043441 and 20040175834. One skilled in the art will know of other methods for measuring MTXPG concentrations.

Where a level of MTXPGs is determined in a sample such as red blood cells (RBCs) or a cellular extract, the term "level" refers to the amount or concentration of at least one, two, three, four, five, or all of the MTXPG species in the sample. It is understood that a level can be an absolute level such as a molar concentration or weight or a relative level such as a percent or fraction compared to one or more other molecules in the sample. As a non-limiting example, a level of MTXPGs in RBCs can be expressed in terms of any unit known to one skilled in the art, including nmol/L RBCs, pmol/10⁹ RBCs, pmol/8 x 10⁸ RBCs, nmol/nmol hemoglobin, nmol/mg hemoglobin, pmol/25 mg hemoglobin, pmol/100 erythrocytes, and pmol/100 µl RBCs.

In certain embodiments, a level of one or more long-chain MTXPG species is determined by resolving them from short-chain MTXPGs and other molecules. As used herein, the term "long-chain MTXPG" refers to any MTX having at least three glutamates attached thereto (e.g., MTXPG₃, MTXPG₄, MTXPG₅ [SEQ ID NO:26], MTXPG₆ [SEQ ID NO:27], and/or MTXPG₇ [SEQ ID NO:28]). Thus, resolving long-chain MTXPG species that have an observable property involves sufficiently separating them from other molecules having the same property. As a non-limiting example, MTXPG₃, which is detectable by fluorescence at a particular excitation and emission wavelength, can be resolved by separating it from other molecules that have substantial excitation and emission at the same wavelengths. The MTXPG₃ species may or may not be separated from a variety of other molecules having different excitation and emission wavelengths. In view of the foregoing, it is understood that whether or not the long-chain MTXPG is resolved is determined, in part, by the detection means utilized in the method. In some embodiments, MTXPG₃ alone is resolved. In other embodiments, MTXPG₃, together with MTXPG₄, MTXPG₅ [SEQ ID NO:26], MTXPG₆ [SEQ ID NO:27], and/or MTXPG₇ [SEQ ID NO:28], is resolved. In further embodiments, MTXPG₄ and/or MTXPG₅ is resolved.

Cellular extracts useful in the present invention can be prepared from a cell or tissue sample using methods well known in the art. Those skilled in the art will know or be able to determine an appropriate method for obtaining source cells based on their location and characteristics. As an example, red blood cells and other blood cells can be obtained by harvesting through intravenous routes. Cells can also be removed from tissues using known biopsy methods including, for example, those utilizing an open surgical incision, biopsy needle, or endoscope. Cells can be lysed by any of a variety of means depending, in part, on the properties of the cell. As non-limiting examples, cells can be lysed by mechanical disruption with glass beads, a Dounce homogenizer, french press, or sonication; enzymatic disruption with lysozyme or other enzyme that degrades the cell wall; osmotic disruption; or a combination of these methods.

A cellular extract useful in the methods of the present invention can be any cellular extract that contains at least one long-chain MTXPG. It is understood that additional exogenous MTXPGs can be added, if desired, to a cellular extract. The addition of one or more exogenous MTXPGs into a cellular extract can be useful for determining a standard curve for quantification or for optimizing detection conditions.

Long-chain MTXPGs can be resolved from the components of a cellular extract by any of a variety of methods such as chromatographic methods, spectrometric methods, or other methods that serve to separate molecules based on size or charge. Examples of useful chromatographic methods include, without limitation, liquid and gas phase chromatographic methods such as normal phase chromatography (e.g., HPLC), reverse phase chromatography (e.g., RP-HPLC), ion exchange chromatography, size exclusion chromatography, iso-electric focusing, gel electrophoresis, capillary electrophoresis, and affinity chromatography. Exemplary, but not limiting, spectrometric methods include mass spectrometry, tandem mass spectrometry, and preparative mass spectrometry with electrospray ionization. It is understood that, if desired, two or more of the above techniques can be combined to resolve MTXPGs.

As a non-limiting example, long-chain MTXPGs can be chromatographically resolved from other cellular components using reverse phase chromatography and subsequently quantitated by comparison to one or more known reference standards. In certain instances, chromatographic resolution of MTXPGs can be performed by passing a mixture of MTXPGs in a cellular extract through a C18 reverse phase column in a mobile phase consisting of a 20 minute linear gradient from 2% acetonitrile/98% mobile phase A to 12.5% acetonitrile/87.5% mobile phase A, wherein mobile phase A is 10 mM ammonium acetate, pH 6.5, with hydrogen peroxide at a final concentration of 0.2%.

A reverse phase column useful for resolving long-chain MTXPGs in a cellular extract can have, for example, dimensions of 25 cm x 4.6 mm. It is understood that columns having larger or smaller diameters, lengths or both can also be used, for example, to accommodate larger or smaller sample sizes. Flow rates can vary, without limitation, from 0.2 to 2.5 ml/minute. For example, the flow rate for the mobile phase can be 1 ml/minute. However, the flow rate of the mobile phase can be altered as desired. A slower flow rate, such as 0.8 ml/minute, 0.5 ml/minute or 0.2 ml/minute, can be used, for example, with a smaller column or to increase MTXPG retention times. Alternatively, a faster flow rate, such as 1.5 ml/minute or 2.0 ml/minute, can be used, for example, with a larger column or to decrease MTXPG retention times.

In some embodiments, soluble molecules such as MTXPGs can be separated from proteins and other materials by acid precipitation prior to using one of the above chromatographic methods. The term "acid" as used herein refers to a reagent that is capable of effecting preferential precipitation of proteinaceous material from solution, without precipitating MTXPGs. One skilled in the art understands that an acid useful in the present invention does not substantially destroy, degrade, or otherwise affect detection of MTXPGs. Exemplary acids include, without limitation, perchloric acid; sulfuric acid, phosphoric acid, and glacial acetic acid. Additional acids useful in the present invention can be identified by the ability to yield substantially similar MTXPG levels for a particular sample, as compared to a sample contacted with 70% perchloric acid.

In other embodiments, cellular extracts can be partially purified by centrifugation, liquid-liquid extraction, or solid-phase extraction to enrich for MTXPGs prior to using one of the above chromatographic methods. Techniques for obtaining and partially purifying cellular extracts are well known in the art and are described, for example, in Scopes, Protein Purification: Principles and Practice, 3rd Ed., Springer-Verlag, New York (1994); Coligan et al., Current Protocols in Protein Science, John Wiley and Sons, Baltimore, MD (2000); and Rubino, J. Chromatog., 764:217-254 (2001).

In the methods of the present invention, a level of one or more long-chain MTXPGs is typically determined using a post-column photo-oxidation chromatographic-flourometric technique by resolving the long-chain MTXPGs in the cellular extract, irradiating the resolved long-chain MTXPGs, thereby producing fluorescent long-chain MTXPG photolytic products, and detecting the fluorescent long-chain MTXPG photolytic products. In certain instances, long-chain MTXPGs can be resolved using HPLC. In certain other instances, long-chain MTXPGs can be irradiated using UV irradiation. For example, long-chain MTXPGs can be UV irradiated in a solvent having about 0.05% to about 1% H₂O₂ or about 0.1% to about 0.3% H₂O₂. Long-chain MTXPGs can be UV irradiated using radiation having a wavelength in the range of about 225 nm to about 275 nm, for example, a wavelength of about 254 nm. The irradiation can have a duration, for example, of about 0.5 to about 60 seconds or about 0.5 to about 15 seconds.

The resolved fluorescent long-chain MTXPG photolytic products can be detected, for example, by detecting fluorescence upon excitation in the range of about 240 nm to about 420 mn. In particular embodiments, fluorescence is detected upon excitation with UV radiation in the range of about 240 nm to about 300 nm, for example, upon excitation with UV radiation at 274 nm. In another embodiment, fluorescence is detected upon excitation with UV radiation in the range of about 360 nm to about 410 nm. It is understood that fluorescence is detected at an appropriate emission wavelength, such as an emission wavelength in the range of about 320 nm to about 550 nm or an emission wavelength in the range of about 440 nm to about 500 nm. In one embodiment, fluorescence is detected at an emission wavelength of 464 nm. In a further embodiment, fluorescence is detected upon excitation with UV radiation at 274 nm and at an emission wavelength of 464 nm.

The term "photolytic product" as used herein refers to a molecule that is produced by cleavage of bonds in a methotrexate polyglutamate that is electronically excited by radiation. The process of producing a photolytic product is referred to as photolysis. Photolysis of long-chain MTXPGs to produce "long-chain MTXPG photolytic products" can be performed, for example, with UV light, which is a term understood in the art to include light of any wavelength in the range of about 200 to about 400 nm. It further is understood that any light source which produces UV light can be useful for irradiating long-chain MTXPG including, for example, a lamp such as an arc lamp, a quartz halogen lamp, or a laser. It is understood that long-chain MTXPGs can be selectively irradiated with a particular wavelength in the UV range by using an appropriate light source, optical filter, or combination of these components in accordance with their known optical characteristics.

In the methods of the present invention which involve detecting fluorescent long-chain MTXPG photolytic products, long-chain MTXPGs are irradiated for an appropriate period of time to yield fluorescent long-chain MTXPG photolytic products. In particular embodiments, long-chain MTXPGs are irradiated for about 0.5 to about 60 seconds or for about 0.5 to about 15 seconds. As non-limiting examples, long-chain MTXPGs can also be irradiated for about 0.1 to about 100 seconds, about 0.2 to about 60 seconds, about 0.5 to about 45 seconds, about 0.5 to about 30 seconds, about 0.5 to about 20 seconds, about 0.5 to about 10 seconds, about 1 to about 20 seconds, about 1 to about 10 seconds, about 2 to about 20 seconds, about 2 to about 10 seconds, about 0.5 to about 6 seconds, about 0.5 to about 5 seconds, about 0.5 to about 4 seconds, about 1 to about 6 seconds, about 1 to about 5 seconds, about 1 to about 4 seconds, or about 2 to about 4 seconds. For example, irradiation of long-chain MTXPGs for 3 seconds with a 254 nm low pressure mercury ultraviolet lamp produced fluorescent long-chain MTXPG photolytic products with overlapping excitation spectra that were readily detectable upon excitation with UV radiation with a wavelength of 274 nm and at an emission wavelength of 464 nm. One skilled in the art will appreciate that the irradiation time can be varied to produce fluorescent long-chain MTXPG photolytic products having characteristic properties desired for a particular application.

Irradiation of long-chain MTXPG for three seconds with a 254 nm low pressure mercury ultraviolet lamp typically produces fluorescent long-chain MTXPG photolytic products with overlapping excitation spectra that are readily detectable, for example, upon excitation with UV radiation at a wavelength of 274 nm and at an emission wavelength of 464 nm. It is understood that the time of irradiation can be varied to produce the desired fluorescent long-chain MTXPG photolytic product having characteristic properties as desired for a particular application. A particular fluorescent photolytic product can have, for example, one or more characteristic properties such as characteristic fluorescence excitation and emission peak maxima, and characteristic fluorescence intensity levels depending, for example, upon the pH and amount of acetonitrile present during detection.

Photolysis of long-chain MTXPGs can be carried out in the presence of hydrogen peroxide (H₂O₂) or another peroxide. As a non-limiting example, when hydrogen peroxide is added during irradiation of long-chain MTXPGs, the final concentration can be about 0.03% or higher. In particular embodiments, the final concentration of hydrogen peroxide during photolysis of long-chain MTXPGs is in the range of about 0.05% to about 1%, about 0.1% to about 1%, about 0.1% to about 0.5%, or about 0.1% to about 0.3%.

A level of long-chain MTXPGs in a sample can be determined based on the level of the corresponding fluorescent long-chain MTXPG photolytic product. As a non-limiting example, the amount or concentration of the fluorescent long-chain MTX photolytic product can be determined based on the intensity of fluorescence from the photolytic product. As used herein, the term "fluorescence" refers to an emission of photons in the ultraviolet (UV), visible (VIS), or infrared (IR) region of the spectrum in response to electronic excitation by radiation. The term "fluorescent," when used in reference to a long-chain MTXPG photolytic product, refers to a photolytic product that emits photons in the UV, VIS, or IR region of the spectrum in response to electronic excitation by radiation. Thus, a fluorescent long-chain MTXPG photolytic product is a photolytic product derived from a methotrexate polyglutamate that emits photons in the UV, VIS or IR region of the spectrum in response to electronic excitation by radiation. A fluorescent long-chain MTXPG photolytic product can be characterized, for example, as emitting photons at a quantum yield of at least about 0.01 when excited by radiation in solution. In particular embodiments, a fluorescent long-chain MTXPG photolytic product is characterized by a quantum yield of fluorescence that is at least about 0.1, 0.2, 0.3, 0.4, 0.5, 0.6, 0.7, 0.8, 0.9, or higher when excited by radiation in solution.

A fluorescent molecule, such as a fluorescent long-chain MTXPG photolytic product, can also be characterized with respect to its maximum emission wavelength or maximum excitation wavelength. In particular embodiments, the methods of the present invention involve detecting a resolved fluorescent long-chain MTXPG photolytic product having a maximum excitation wavelength in the infrared, red, orange, yellow, green, blue, violet, or ultraviolet region of the spectrum. In additional embodiments, the methods of the present invention involve detecting a resolved fluorescent long-chain MTXPG photolytic product having a maximum emission wavelength in the infrared, red, orange, yellow, green, blue, violet, or ultraviolet region of the spectrum.

Fluorescence can be detected using any of a variety of excitation sources and emission detectors. Excitation of a fluorescent long-chain MTXPG photolytic product can be achieved, for example, with an excitation source such as a lamp or laser including, without limitation, any of those described above in regard to photolysis. Excitation at a particular wavelength or in a particular wavelength range can be achieved using, for example, a laser that is tuned to the desired wavelength or a lamp having an output that includes the desired wavelength range. An appropriate optical filter can be placed between the excitation source and the fluorescent long-chain MTXPG photolytic product to further limit the range of wavelengths contacting the fluorescent long-chain MTXPG photolytic product.

Each of the seven fluorescent MTXPG₁ to MTXPG₇ [SEQ ID NO:28] photolytic products typically has two excitation peaks in the range of about 240 nm to about 420 nm, including a peak from about 240 nm to about 300 nm and a peak from about 360 nm to about 410 nm. In particular embodiments, a fluorescent long-chain MTXPG photolytic product can be detected by excitation at a wavelength in the range of about 240 nm to about 420 nm, about 240 nm to about 300 nm, or about 360 nm to about 410 nm. If desired, the methods of the present invention can include excitation at or near the peak of 274 nm or in a range near this peak including, for example, excitation at a wavelength in the range of about 250 nm to about 300 nm or about 260 nm to about 285 nm. Excitation at or near the peak of 385 nm or in a range near this peak can also be useful including, for example, excitation at a wavelength in the range of about 360 nm to about 400 nm or about 375 nm to about 395 nm.

Emission can be detected from a fluorescent long-chain MTXPG photolytic product using any of a variety of detectors such as, without limitation, a photomultiplier tube, diode, diode array, or charge coupled device camera. A detector that detects light at a particular wavelength or in a particular wavelength range can also be useful. If desired, an optical filter can be placed between the fluorescent long-chain MTXPG photolytic product and the detector to limit the range of wavelengths detected. Fluorescent MTXPG₁ to MTXPG₇ [SEQ ID NO:28] photolytic products typically emit at a wavelength in the range of about 320 nm to about 550 nm, and have a primary emission peak at about 440 nm to about 520 nm. In particular embodiments, emission from a fluorescent long-chain MTXPG photolytic product can be detected at a wavelength in the range of about 320 nm to about 550 nm or about 440 nm to about 520 nm. If desired, the methods of the present invention can include detection of emission at or near the peak of 464 nm or in a range near this peak including, for example, emission at a wavelength in the range of about 430 nm to about 510 nm or about 450 nm to about 480 nm.

The content of a solution that is used to detect a resolved long-chain MTXPG or a photolytic product thereof can be varied, for example, with respect to pH or acetonitrile content. The pH at which long-chain MTXPGs or photolytic products thereof are detected can be in the range of, for example, a pH of about 2 to about 8 or a pH of about 4 to about 7. In particular embodiments, long-chain MTXPGs or photolytic products thereof can be detected, for example, at pH 4, 4.5, 5, 5.5, 6, 6.5, or 7. The amount of acetonitrile present during detection can be in the range of, for example, about 0% to about 20% or about 10% to about 20%. In particular embodiments, the amount of acetonitrile present can be, for example, about 5%, 10%, 11%, 11.5%, 12%, 12.5%, 13%, 13.5%, 15%, or 20%.

Resolved long-chain MTXPGs can also be detected based on one or more other observable, characteristic properties including, for example, ultraviolet or visible light absorption properties, fluorescence, electrochemical properties, or mass. As non-limiting examples, resolved long-chain MTXPGs can be detected with UV/Vis absorption spectroscopy, fluorimetry, electrochemical detection, or mass spectrometry. Those skilled in the art will know or be able to determine an appropriate means for detecting long-chain MTXPGs based on the accuracy and sensitivity desired and the presence of potentially interfering substances in the particular sample being analyzed.

As shown in Example 2 below, RBC long-chain MTXPG concentrations predicted response to methotrexate therapy during a dose escalation in subjects with rheumatoid arthritis. As a result, the present invention provides methods for evaluating the likelihood of response to MTX and for optimizing dose efficacy of MTX therapy in a subject by determining a level of long-chain MTXPGs in a sample from the subject.

The level of long-chain MTXPGs can be, for example, the level of long-chain MTXPGs in RBCs from the subject. MTXPG₃ is the predominant polyglutamate species in RBCs and is strongly predictive of the long-chain MTXPG concentration expressed as the sum of MTXPG₃ + MTXPG₄ + MTXPG₅ [SEQ ID NO:26] (MTXPG₃₋₅). The level of MTXPG₃ in RBCs is also predictive of the long-chain MTXPG concentration expressed as the sum of MTXPG₄ + MTXPG₅ [SEQ ID NO:26] (MTXPG₄₋₅) and of MTXPG₅ [SEQ ID NO:26]. As such, RBC MTXPG₃ levels can be used as a marker of MTXPG₃₋₅, MTXPG₄₋₅, and/or MTXPG₅ [SEQ ID NO:26] levels. Preferably, RBC MTXPG₃ levels are measured as described above using an HPLC-fluorometry procedure with a post-column photo-oxidation technique.

In some embodiments, the level of one or more long-chain MTXPGs (*e.g.,* MTXPG₃, MTXPG₄ and/or MTXPG₅ [SEQ ID NO:26]) is determined within the first 6 months of starting MTX therapy. As a non-limiting example, the level of MTXPG₃ can be measured in RBCs within about 1, 2, 3, 4, 5, or 6 months of starting MTX therapy. In certain instances, a level of MTXPG₃ greater than about 20 nmol/L RBCs indicates that a subject has a high likelihood of responding to MTX about 3 months later. For example, a level of MTXPG₃ greater than about 20 nmol/L RBCs, when measured at about 3 months after starting MTX therapy, is highly predictive of a subject having a therapeutic response to MTX at about 6 months into therapy (OR = 8.0). Alternatively, a level of MTXPG₃ greater than about 20 nmol/L RBCs, when measured at about 1, 2, 4, 5, or 6 months after starting MTX therapy, can be predictive of a subject having a therapeutic response to MTX at about 4, 5, 7, 8, or 9 months, respectively, into therapy. In certain other instances, a level of MTXPG₃ greater than about 10, 15, 16, 17, 18, 19, 21, 22, 23, 24, 25, 30, 35, 40, 45, 50, 55, 60, 65, 70, 75, 80, 85, 90, 95, or 100 nmol/L RBCs indicates that a subject has a high likelihood of responding to MTX about 3 months later.

In other embodiments, the level of one or more long-chain MTXPGs (*e.g.,* MTXPG₃, MTXPG₄ and/or MTXPG₅ [SEQ ID NO:26]) is determined at any time during MTX therapy. As a non-limiting example, the level of MTXPG₃ can be measured in RBCs at about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, or more months after starting MTX therapy. In certain instances, a detectable level of MTXPG₃ indicates that a subject has a high likelihood of responding to MTX about 1 month later. For example, the presence of MTXPG₃ in a RBC sample, when determined at about 1, 2, or 3 months after starting MTX therapy, is predictive of a subject having a therapeutic response to MTX at about 2, 3, or 4 months, respectively, into therapy. One skilled in the art will appreciate that the detection limit of MTXPGs in RBCs typically depends on the analytical method used. For example, RBC MTXPG levels that are measured as described above using an HPLC-fluorometry procedure with a post-column photo-oxidation technique provide a quantification limit of about 5 nmol/L RBCs and a detection limit of about 2 nmol/L RBCs.

### IX. Methods of Administration

According to the methods of the present invention, methotrexate (MTX) can be administered to a subject by any convenient means known in the art. The assay methods of the present invention can be used to predict MTX efficacy in subjects who have not received MTX or to optimize dosage of MTX in subjects who are currently undergoing MTX therapy. The assay methods of the present invention can also be used to predict MTX toxicity in subjects who have not received MTX or to reduce toxicity to MTX in subjects who are currently undergoing MTX therapy. One skilled in the art will appreciate that MTX can be administered alone or as part of a combined therapeutic approach with one or more additional therapeutic agents. One skilled in the art will also appreciate that one or more alternative therapeutic agents can be administered instead of MTX when MTX therapy is predicted or determined to be non-efficacious and/or toxic.

The therapeutic agents described herein can be administered with a suitable pharmaceutical excipient as necessary and can be carried out via any of the accepted modes of administration. Thus, administration can be, for example, oral, buccal, sublingual, gingival, palatal, intravenous, topical, subcutaneous, transcutaneous, transdermal, intramuscular, intra-joint, parenteral, intra-arteriole, intradermal, intraventricular, intracranial, intraperitoneal, intravesical, intrathecal, intralesional, intranasal, rectal, vaginal, or by inhalation.

As a non-limiting example, MTX can be co-administered with any compound useful for reducing or alleviating the side-effects associated with MTX therapy. Examples include, but are not limited to, folic acid and folic acid analogs such as folinic acid, dihydrofolic acid, tetrahydrofolic acid, 5-formyl-tetrahydrofolic acid, and 10-methyl-tetrahydrofolic acid. One skilled in the art will know of additional compounds that can reduce or alleviate the side-effects resulting from MTX administration.

MTX can also be co-administered with immunosuppressive agents including sirolimus (rapamycin); temsirolimus; everolimus; tacrolimus (FK-506); FK-778; thiopurine drugs such as azathioprine and metabolites thereof; anti-metabolites such as methotrexate; immunosuppressive antibodies such as anti-tumor necrosis factor (TNF) antibodies (*e.g.,* adalimumab, infliximab, *etc.*)*,* anti-lymphocyte globulin antibodies, anti-thymocyte globulin antibodies, anti-CD3 antibodies, anti-CD4 antibodies, and antibody-toxin conjugates; cyclosporine; mycophenolate; mizoribine monophosphate; scoparone; glatiramer acetate; metabolites thereof; pharmaceutically acceptable salts thereof; derivatives thereof; analogs thereof; stereoisomers thereof; prodrugs thereof; and combinations thereof. Alternatively, any of the above immunosuppressive agents can be administered instead of MTX as an alternative therapy.

Additionally, MTX can be co-administered with anti-inflammatory agents including corticosteroids such as prednisolone, methylprednisolone aceponate, mometasone furoate, hydrocortisone, clobetasol propionate, betamethasone, betamethasone valerate, betamethasone dipropionate, dexamethasone, dexamethasone acetate, fluticasone propionate, clobetasone butyrate, beclomethasone dipropionate, and loteprednol etabonate; non-steroidal anti-inflammatory agents such as diclofenac, diflunisal, etodolac, fenbufen, fenoprofen, flurbiprofen, ibuprofen, indomethacin, ketoprofen, ketorolac, meclofenamate, mefenamic acid, meloxicam, nabumetone, naproxen, nimesulide, oxaprozin, piroxicam, salsalate, sulindac, tolmetin, celecoxib, rofecoxib, and 4-biphenylylacetic acid; pharmaceutically acceptable salts thereof; derivatives thereof; analogs thereof; stereoisomers thereof; prodrugs thereof; and combinations thereof. Alternatively, any of the above anti-inflammatory agents can be administered instead of MTX as an alternative therapy.

In a further embodiment, MTX can be co-administered with antibiotics including norfloxacin, ciprofloxacin, ofloxacin, grepafloxacin, levofloxacin, sparfloxacin, clindamycin, erythromycin, tetracycline, minocycline, doxycycline, penicillin, ampicillin, carbenicillin, methicillin, cephalosporins, vancomycin, bacitracin, streptomycin, gentamycin, fusidic acid, ciprofloxin and other quinolones, sulfonamides, trimethoprim, dapsone, isoniazid, teicoplanin, avoparcin, synercid, virginiamycin, piperacillin, ticarcillin, cefepime, cefpirome, rifampicin, pyrazinamide, enrofloxacin, amikacin, netilmycin, imipenem, meropenem, inezolidcefuroxime, ceftriaxone, chloramphenicol, cefadroxil, cefazoline, ceftazidime, cefotaxime, roxithromycin, cefaclor, cefalexin, cefotiam, cefoxitin, amoxicillin, co-amoxiclav, mupirocin, cloxacillin, triclosan, co-trimoxazole, pharmaceutically acceptable salts thereof, derivatives thereof, analogs thereof, stereoisomers thereof, prodrugs thereof, and combinations thereof. Alternatively, any of the above antibiotics can be administered instead of MTX as an alternative therapy.

MTX can also be co-administered with chemotherapeutic agents including platinum-based drugs (*e.g.,* oxaliplatin, cisplatin, carboplatin, spiroplatin, iproplatin, satraplatin, *etc*.), alkylating agents (*e.g*., cyclophosphamide, ifosfamide, chlorambucil, busulfan, melphalan, mechlorethamine, uramustine, thiotepa, nitrosoureas, *etc*.), antimetabolites (e.g., 5-fluorouracil, leucovorin, capecitabine, cytarabine, floxuridine, fludarabine, gemcitabine, pemetrexed, raltitrexed, *etc*.), plant alkaloids (e.g., vincristine, vinblastine, vinorelbine, vindesine, podophyllotoxin, paclitaxel, docetaxel, etc.), topoisomerase inhibitors (*e.g*., irinotecan, topotecan, amsacrine, etoposide (VP16), etoposide phosphate, teniposide, etc.), antitumor antibiotics (e.g., doxorubicin, adriamycin, daunorubicin, epirubicin, actinomycin, bleomycin, mitomycin, mitoxantrone, plicamycin, etc.), pharmaceutically acceptable salts thereof, derivatives thereof, analogs thereof, stereoisomers thereof, prodrugs thereof, and combinations thereof. Alternatively, any of the above chemotherapeutic agents can be administered instead of MTX as an alternative therapy.

A therapeutically effective amount of any of the therapeutic agents described herein may be administered repeatedly, e.g., at least 2, 3, 4, 5, 6, 7, 8, or more times, or the dose may be administered by continuous infusion. The dose may take the form of solid, semisolid, lyophilized powder, or liquid dosage forms, such as, for example, tablets, pills, pellets, capsules, powders, solutions, suspensions, emulsions, suppositories, retention enemas, creams, ointments, lotions, gels, aerosols, foams, or the like, preferably in unit dosage forms suitable for simple administration of precise dosages.

As used herein, the term "unit dosage form" refers to physically discrete units suitable as unitary dosages for human subjects and other mammals, each unit containing a predetermined quantity of a therapeutic agent calculated to produce the desired onset, tolerability, and/or therapeutic effects, in association with a suitable pharmaceutical excipient (e.g., an ampoule). In addition, more concentrated dosage forms may be prepared, from which the more dilute unit dosage forms may then be produced. The more concentrated dosage forms thus will contain substantially more than, *e.g.,* at least 1, 2, 3, 4, 5, 6, 7, 8, 9, 10, or more times the amount of the therapeutic agent.

Methods for preparing such dosage forms are known to those skilled in the art *(see, e. g.,* REMINGTON'S PHARMACEUTICAL SCIENCES, 18TH ED., Mack Publishing Co., Easton, PA (1990)). The dosage forms typically include a conventional pharmaceutical carrier or excipient and may additionally include other medicinal agents, carriers, adjuvants, diluents, tissue permeation enhancers, solubilizers, and the like. Appropriate excipients can be tailored to the particular dosage form and route of administration by methods well known in the art (*see, e. g., REMINGTON's PHARAMCEUTICAL SCIENCES, supra*).

Examples of suitable excipients include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline, syrup, methylcellulose, ethylcellulose, hydroxypropylmethylcellulose, and polyacrylic acids such as Carbopols, e.g., Carbopol 941, Carbopol 980, Carbopol 981, *etc.* The dosage forms can additionally include lubricating agents such as talc, magnesium stearate, and mineral oil; wetting agents; emulsifying agents; suspending agents; preserving agents such as methyl-, ethyl-, and propyl-hydroxy-benzoates (*i.e*., the parabens); pH adjusting agents such as inorganic and organic acids and bases; sweetening agents; and flavoring agents. The dosage forms may also comprise biodegradable polymer beads, dextran, and cyclodextrin inclusion complexes.

For oral administration, the therapeutically effective dose can be in the form of tablets, capsules, emulsions, suspensions, solutions, syrups, sprays, lozenges, powders, and sustained-release formulations. Suitable excipients for oral administration include pharmaceutical grades of mannitol, lactose, starch, magnesium stearate, sodium saccharine, talcum, cellulose, glucose, gelatin, sucrose, magnesium carbonate, and the like.

In some embodiments, the therapeutically effective dose takes the form of a pill, tablet, or capsule, and thus, the dosage form can contain, along with a therapeutic agent, any of the following: a diluent such as lactose, sucrose, dicalcium phosphate, and the like; a disintegrant such as starch or derivatives thereof; a lubricant such as magnesium stearate and the like; and a binder such a starch, gum acacia, polyvinylpyrrolidone, gelatin, cellulose and derivatives thereof. A therapeutic agent can also be formulated into a suppository disposed, for example, in a polyethylene glycol (PEG) carrier.

Liquid dosage forms can be prepared by dissolving or dispersing a therapeutic agent and optionally one or more pharmaceutically acceptable adjuvants in a carrier such as, for example, aqueous saline (*e.g.,* 0.9% w/v sodium chloride), aqueous dextrose, glycerol, ethanol, and the like, to form a solution or suspension, e.g., for oral, topical, or intravenous administration. A therapeutic agent can also be formulated into a retention enema.

For topical administration, the therapeutically effective dose can be in the form of emulsions, lotions, gels, foams, creams, jellies, solutions, suspensions, ointments, and transdermal patches. For administration by inhalation, a therapeutic agent can be delivered as a dry powder or in liquid form via a nebulizer. For parenteral administration, the therapeutically effective dose can be in the form of sterile injectable solutions and sterile packaged powders. Preferably, injectable solutions are formulated at a pH of from about 4.5 to about 7.5.

The therapeutically effective dose can also be provided in a lyophilized form. Such dosage forms may include a buffer, e.g., bicarbonate, for reconstitution prior to administration, or the buffer may be included in the lyophilized dosage form for reconstitution with, e.g., water. The lyophilized dosage form may further comprise a suitable vasoconstrictor, e.g., epinephrine. The lyophilized dosage form can be provided in a syringe, optionally packaged in combination with the buffer for reconstitution, such that the reconstituted dosage form can be immediately administered to a subject.

### X. Examples

The following examples are offered to illustrate, but not to limit, the claimed invention.

### Example 1. A Toxicogenetic Index for Evaluating Risk Associated with MTX Therapy.

This example presents a study showing the contribution of wild-type, heterozygous, or homozygous genotypes in folate pathway genes to MTX toxicity in patients with rheumatoid arthritis. In particular, low penetrance risk genotypes in *MTHFR, ATIC, TS,* and *SHMT1* were identified and used to generate a toxicogenetic index indicative of the risk or occurrence of side-effects associated with MTX therapy.

### SUMMARY

Methotrexate (MTX) is an anti-folate compound with significant toxicity. In this example, an association between certain genotypes in folate pathway genes and the occurrence of side-effects to MTX in patients with rheumatoid arthritis was identified. The study was cross-sectional with all patients on MTX therapy for at least one month prior to enrollment. Blood was collected and side-effects occurring at the time of a single study visit were recorded. Low penetrance risk genotypes in 5,10-methylenetetrahydrofolate reductase (*MTHFR* 677T/T), 5-aminoimidazole-4-carboxamide ribonucleotide transformylase (*ATIC* 347G/G), thymidylate synthase (*TS* *2/*2), and serine hydroxymethyltransferase (*SHMT1* 1420C/C) were measured and cumulated to generate a toxicogenetic index characteristic of each patient Statistical analyses consisted of logistic regression analyses.

For the 97 patients enrolled, a total of 40 patients presented with a side-effect related to MTX therapy (*e.g*., gastrointestinal side-effect, headache, lethargy, alopecia). All risk genotypes were associated with at least one type of side-effect (p < 0.05). The toxicogenetic index ranged from 0 to 3 (median =1). An increased toxicogenetic index was associated with increased occurrence of side-effects (OR CI 95%: 29.83, 5.20-171.05; p < 0.001). In fact, 100% of patients with a toxicogenetic index of 3 presented with a side-effect, as compared to only 17% of those patents with an index of 0. In 46 patients with high disease activity, those with a toxicogenetic index above an index cutoff value of 0 were 10.5-fold (CI 95%: 1.2-90.9) more likely to experience a side-effect compared to those with a toxicogenetic index of 0 (p = 0.03). These results indicate that a composite index cumulating risk genotypes in folate-dependant enzymes can profile patients with side-effects to MTX therapy.

### METHODS

Study design: The study was cross-sectional at two investigational California sites (Cedars Sinai, Department of Rheumatology; and UCLA, Department of Rheumatology). To be eligible, patients (≥18 yr) had to meet the revised criteria of the American Rheumatism Association for Rheumatoid Arthritis and to have received low dose MTX therapy for at least one month. Concomitant disease-modifying arthritis agents (e.g., sulphasalazine, hydroxychloroquine, leflunomide), low-dose corticosteroids (<10 mg day), and folic acid supplementation were allowed. Institutional review boards approved the study and patient consent was obtained for each patient. Patient demographics were collected at the time of enrollment in the study. At a single study visit, EDTA blood was drawn and shipped overnight to Prometheus Laboratories in San Diego, CA.

Clinical status assessment: Patient clinical and demographic characteristics were collected at the single study visit. Clinical efficacy was assessed using the disease activity score (DAS28), which includes the number of tender and swollen joint counts (n = 28), the erythrocyte sedimentation rate, and the patient's global assessment of disease activity. MTX side-effects were defined as those affecting the gastrointestinal tract (*e.g.,* nausea, diarrhea, stomatatis, dyspepsia, elevation of aspartate aminotransferase above the upper limit of 40 U/L), central nervous system (*e.g*., headache, lethargy), hematopoietic system (*e.g*., leucopenia <3500/mm³, anemia with hemoglobin <80g/L), and lung (e.g., pulmonary infiltrate).

Laboratory measurements: Each attending physician and each patient was blinded to genotypes throughout the entire study, and laboratory personnel were blinded to the occurrences of patients' side-effects. Common polymorphisms in *MTHFR* (C677T), *ATIC* (C347G), *TS* (*2/*3), and *SHMT1* (C1420T) were measured as described in, *e.g.,* Dervieux et al., Arthritis Rheum., 50:2766-2774 (2004); and Skibola et al., Blood, 99:3786-3791 (2002).

For example, tandem repeat sequences in the promoter region of the *TS* gene can be detected using 0.5-1.0 µg DNA and 0.2 µM each of the following primers: forward primer, 5'-GTG GCT CCT GCG TTT CCC CC-3' (SEQ ID NO:1); and reverse primer, 5'-CCA AGC TTG GCT CCG AGC CGG CCA CAG GCA TGG CGC GG-3' (SEQ ID NO:2). Polymerase chain reaction (PCR) cycling parameters can be a 5-minute denaturation cycle at 94°C and 30 cycles of the following: 94°C for 1 minute, 60°C for 1 minute, and 72°C for 2 minutes. Amplified PCR products can be visualized on a 3% agarose gel with ethidium bromide. Homozygotes for the double tandem repeat (*TS* *2/*2) produce a singlet 220-bp band, heterozygotes (*TS* 2/*3) produce 220-bp and 250-bp fragments, and homozygotes for the triple tandem repeat (*TS* *3/*3) produce a 250-bp fragment.

*SHMT1* genotyping can be performed using a standard restriction fragment length polymorphism (RFLP) method and restriction enzyme analysis. The following primers can be used to amplify a 292-bp fragment containing the loci of interest: forward primer, 5'-GTG TGG GGT GAC TTC ATT TGT G-3' (SEQ ID NO:3); and reverse primer, 5'-GGA GCA GCT CAT CCA TCT CTC-3' (SEQ ID NO:4). Restriction enzyme digestion can be carried out using Earl, which cuts the wild-type sequence into 113-bp and 179-bp fragments. Alternatively, allelic discrimination can be performed to detect the *SHMT1* (C1420T) polymorphism using fluorogenic 3'-minor groove binding probes in a real-time PCR assay (Kutyavin et al., Nucleic Acids Res. 28:655-661 (2000)). The PCR can be conducted using the following fluorescently-labeled probes: wild-type fluorescent probe, 5'-FAM-CGC CTC TCT CTT C-MGB-3' (SEQ ID NO:5); and mutant fluorescent probe, 5'-VIC-CGC CTC TTT CTT C-MGB-3' (SEQ ID NO:6). Each 15 µl reaction can contain 200 nM of each probe, 900 nM each of forward primer, 5'-CAG AGC CAC CCT GAA AGA GTT C-3' (SEQ ID NO:7) and reverse primer 5'-AGT GGG CCC GCT CCT TTA-3' (SEQ ID NO:8), 1X Taqman Universal PCR Master Mix (Applied Biosystems; Foster City, CA), and 12 ng DNA. PCR cycling conditions can consist of one 2-minute cycle at 50°C, one 10-minute cycle at 95°C, followed by 38 cycles of 92°C for 15 seconds and 62°C for 1 minute.

The *ATIC* C347G polymorphism, which results in a threonine to serine substitution at codon 116 of *ATIC,* can be determined with a real-time TaqMan allelic discrimination assay performed using fluorogenic 3'-minor groove binding probes. The following primers can be used: forward primer, 5'-CCT GCA ATC TCT ATC CCT TTG TAA A-3' (SEQ ID NO:9); and reverse primer, 5'-TTC TGA CTT ACC AAT GTC AAT TTG CT-3' (SEQ ID NO:10). Allelic discrimination can be performed using the wild-type fluorescent probe, 5'-FAM-CCA GGT GTA AGT GTT G-MGB-3' (SEQ ID NO:11) and the mutant fluorescent probe, 5'-VIC-TCC AGG TGT AAC TGT T-MGB-3' (SEQ ID NO:12). Final reaction conditions can be as follows: 900 nM of each primer, 200 nM of each probe, 5 ng genomic DNA, and a 1X TaqMan master mix. PCR reactions can be incubated for one 2-minute cycle at 50°C, a 10-minute cycle at 95°C, and 40 cycles of 95°C for 15 seconds, 58°C for 15 seconds, and finally 60°C for 45 seconds.

The *MTHFR* C677T polymorphism, which results in an alanine to valine substitution at codon 222 of *MTHFR,* can be measured with a standard real-time TaqMan allelic discrimination assay using those probes and primers described in, *e.g*., Ulvik et al., Clin. Chem., 47:2050-2053 (2001). For example, the following primers and fluorogenic 3'-minor groove binding probes can be used: forward primer: 5'-CAC AAA GCG GAA GAA TGT GTC A-3' (SEQ ID NO:13); reverse primer: 5'-AAG CAC TTG AAG GAG AAG GTG TCT-3' (SEQ ID NO:14); wild-type fluorescent probe: 5'-FAM-TGA AAT CGG CTC CCG-MGB-3' (SEQ ID NO:15); and mutant fluorescent probe: 5'-VIC-TGA AAT CGA CTC CCG-MGB-3' (SEQ ID NO:16). Final reaction conditions can be as follows: 900 nM of each primer, 200 nM of each probe, with 5 ng genomic DNA and a 1X TaqMan master mix. PCR conditions can consist of one 2-minute cycle at 50°C followed by a 10-minute cycle at 95°C followed by 40 cycles of 95°C for 15 seconds, 58°C for 15 seconds, and finally 60°C for 45 seconds. One skilled in the art will appreciate that other methods for genotyping *MTHFR* such as, e.g., high-resolution melting of PCR amplicons (Liew et al., Clin Chem. 50:1156-1164 (2004)), Invader biplex assays (Patnaik et al., J. Mol. Diagn. 6:137-144 (2004)), microarray-based methods (Ji et al., Mutat. Res. 548:97-105 (2004)), and melting curve analyses (Deligezer et al., Mol. Diagn. 7:181-185 (2003)), are also within the scope of the present invention.

Statistical analysis: The analysis consisted of multivariate logistic regression with the occurrence of side-effects as the dependent variable. Odds ratios (OR) were calculated and adjusted for concurrent administration of folic acid, non-steroidal anti-inflammatory drugs (NSAIDs), disease-modifying arthritis agents (DMARDs), corticosteroids, number of months on MTX, and MTX dose. The following genotypes were cumulated in a composite index to generate a toxicogenetic index for each patient: *MTHFR* 677T/T, *ATIC* 347G/G, *TS* *2/*2, and *SHMT1* 1420C/C.

### RESULTS

A total of 97 patients (Cedars Sinai, 53 patients; UCLA, 44 patients) undergoing MTX therapy for at least one month were enrolled from October 2003 to November 2004. Patient demographic data are summarized in Table 1. A total of 40 patients (41.2%) presented a side-effect at the time of the visit. All side-effects were mild and did not require MTX withdrawal. Signs of hepatotoxiciy were observed in 6 patients (AST >40U/L) (Kremer et al., Arthritis Rheum., 37:316-328 (1994)). None of the patients presented an elevation of AST twice the upper normal value, pulmonary toxicity, or hematopoietic toxicity.

**Table 1: Clinical characteristics of the 97 patients enrolled in the study. Results are expressed as median (inter-quartile range) or number (%) as appropriate.**

| **Parameter** | **Value** |
|---|---|
| Age (yr) | 57 (47-65) |
| Number of months under methotrexate | 30 (8-73) |
| Rheumatoid factors (IgM) | 33 (34.0%) |
| Low dose corticosteroids | 25 (25.7%) |
| Concurrent DMARD | 20 (29.9%) |
| Concurrent NSAID | 48 (49.5%) |
| Weekly methotrexate dose (mg) | 12.5 (10-17.5) |
| Folic acid supplementation | 86 (88.6%) |
| Disease activity score (DAS28) | 5.04 (3.93-6.12) |
| Overall side-effects | 40 (41.2%) |
| Gastrointestinal tract | 24 (24.7%) |
| Nausea | 9 (9.2%) |
| Diarrhea | 6 (6.2%) |
| Dyspepsia | 7 (7.2%) |
| Stomatatis | 3 (3.1%) |
| AST >40U/L | 6 (6.1%) |
| Central nervous system | 11 (11.3%) |
| Headache | 8 (5.1%) |
| Lethargy | 8 (8.2%) |
| Alopecia | 17 (17.5%) |

The allelic frequency for *MTHFR* 677T was 32% (CI 95%: 24-36; 677T/T, n=8). The allelic frequency for *ATIC* 347G was 33% (CI 95%: 28-38; 347G/G, n=15). The allelic frequency for *TS* *2 was 47% (CI 95%: 42-52; *2/*2, n=30). The allelic frequency for *SHMT1* 1420T was 27% (CI 95%: 23-21; 1420C/C, n=48).

The multivariate logistic regression revealed that the genotypes associated with increased occurrence of gastrointestinal side-effects were the *ATIC* 347G/G genotype (OR = 5.24; CI 95%: 1.37-19.93; p = 0.015), and the *SHMT1* 1420C/C genotype (OR = 3.36; CI 95%: 1.10-10.21; p = 0.032). The *MTHFR* 677T/T and *TS**2/*2 genotypes were not associated with increased risk of gastrointestinal side-effects (p > 0.12). Increased occurrence of alopecia was associated with the *TS**2/*2 genotype (OR = 6.63; CI 95%: 1.74-25.26; p = 0.006) and the *SHMT1* 1420C/C genotype (OR = 5.55; CI 95%; 1.1.35-22.75; p = 0.016). The *ATIC* 347G/G and *MTHFR* 677T/T genotypes were not associated with alopecia (p > 0.13). *MTHFR* 677T/T genotype was the only genotype associated with increased risk for side-effects affecting the central nervous system (OR = 6.07; CI 95%: 1.2-30.87; p = 0.030), with an occurrence of 62.5% in patients with the *MTHFR* 677T/T genotype compared to 9.0% in those with the *MTHFR* 677C/T or 677C/C genotype.

A toxicogenetic index was calculated as the sum of the risk genotypes carried by each patient according to the following formula: *MTHFR* 677T/T + *ATIC* 347G/G + *TS* *2/*2 + *SHMT1* 1420C/C. The toxicogenetic index ranged from 0 to 3 (median = 1). As shown in Table 2 and Figure 2, an increased index value resulted in an increased occurrence of side-effects (p < 0.0001). This association was significant at both sites (UCLA, p = 0.001; Cedar Sinai, p = 0.02). Subsequently, the contribution of the toxicogenetic index to the occurrence of side-effects in a subset of patients having high disease activity (DAS > 5.1; n = 46 patients) was evaluated. A total of 18 patients presented with high disease activity and side-effects. In the subset of patients with high disease activity, an increased toxicogenetic index was associated with an increased frequency of side-effects (OR = 36.5; range CI 95%: 2.76-490.87). Further, patients with a toxicogenetic index above an index cutoff value of 0 were 10.5-fold (CI 95%: 1.2-90.9) more likely to experience a side-effect than those patients with an index of 0 (p = 0.03).

**Table 2: Toxicogenetic index and occurrence of side-effects.**

| **Side-Effect** | **Regression Estimate** | **Odds Ratio CI 95% (unit change)** | **Odds Ratio CI 95% (range)** | **p value** |
|---|---|---|---|---|
| Gastrointestinal | 0.604 ± 0.283 | 1.82 (1.04-3.20) | 6.12 (1.13-33.01) | 0.035 |
| Central nervous system | 0.765 ± 0.385 | 2.14 (1.00-4.61) | 9.93 (1.00-98.54) | 0.049 |
| Alopecia | 1.31 ± 0.385 | 3.72 (1.73-7.99) | 51.60 (5.21-511.06) | 0.001 |
| All side-effects | 1.13 ± 0.293 | 3.10 (1.73-5.55) | 29.83 (5.20-171.05) | <0.001 |

### DISCUSSION

This is the first study to report that polymorphisms other than *MTHFR* C677T contribute to MTX-related side-effects in patients with rheumatoid arthritis.

Because the integrity of folate and nucleobase enzymes is critical for cellular homeostasis, mutations that severely decrease the expression of these enzymes confer a high penetrance devastating phenotype during childhood in the form of dysmorphic features, neurological defects, and congenital blindness in the case of ATIC deficiency (Marie et al., Am. J. Hum. Genet., 74:1276-1281 (2004)) or Smith-Magenis syndrome in the case of *SHMT* deficiency (Elsea et al., Am. J. Hum. Genet., 57:1342-1350 (1995)). Therefore, transmissible common polymorphisms altering the expression of folate-dependent enzymes are likely to present low phenotypic penetrance, even in the context of a homozygous genotype.

This was the rationale for generating a composite index comprising a summation of homozygous risk genotypes. The risk genotypes considered were the *MTHFR* 677T/T genotype, the *TS* *2/*2 genotype, the *ATIC* 347G/G genotype, and the *SHMT1* 1420C/C genotype (*i.e*., leucine to phenylalanine substitution at codon 474). *SHMT1* encodes a vitamin B6-dependent enzyme that plays a pivotal role in providing one-carbon units for purine and thymidylate synthesis (Ulrich et al., Pharmacogenomics, 3:299-313 (2002)), and individuals who are carriers of the 1420C/C genotype have reduced plasma and red blood cell folate levels compared to those with the 1420C/T or T/T genotype (Heil et al., Mol. Genet. Metab., 73:164-172 (2001)). The effect of the *ATIC* 347C/G polymorphism (*i.e*., threonine to serine substitution at codon 116) is still unknown; however, the 347G/G genotype may be associated with decreased *de novo* purine synthesis activity.

This study revealed that the genotypes associated with the occurrence of side-effects to MTX were the *MTHFR* 677T/T, *ATIC* 347G/G, *TS* *2/*2, and *SHMT1* 1420C/C genotypes. Although each of these risk genotypes contributed to an increased occurrence of side-effects, the summation of two or more of them into a toxicogenetic index maximized the penetrance and indicated that an increasing index value was associated with increased risk or occurrence of side-effects. Patients in this study were on long-term therapy and the side-effects observed were mild and did not require MTX withdrawal.

There is great inter-patient variability in the response to MTX and the drug is often inefficient at controlling disease activity. In this population of patients, 47% presented with a high disease activity and 39% of them were also experiencing a side-effect at the time of the visit. An increasing toxicogenetic index was associated with an increased risk or occurrence of side-effects in this subset of patients with high disease activity. Altogether, this study indicates that the toxicogenetic index can be used as a tool to rule out MTX therapy in patients predicted to have an increased risk of side-effects or as a tool to adjust the dose of MTX in patients not responding to therapy and/or experiencing toxicity. As such, the methods of the present invention for generating a composite index of genotypes in folate pathway genes have utility in evaluating a patient's risk of side-effects associated MTX therapy.

### Example 2. Pharmacogenomic and Metabolic Biomarkers in the Folate Pathway are Associated with MTX Effects During a Dose Escalation in Rheumatoid Arthritis.

This example presents a study showing the contribution of pharmacogenomic and metabolic biomarkers to methotrexate (MTX) efficacy and toxicity in patients with early rheumatoid arthritis who have not been previously treated with this anti-folate. In particular, common polymorphisms in folate pathway genes were identified as contributing to MTX efficacy and/or toxicity. As described herein, wild-type, heterozygous, or homozygous genotypes in these genes can be determined and cumulated in a pharmacogenomic index to predict MTX therapy, e.g., to evaluate the risk of developing MTX toxicity and/or to evaluate the likelihood of response to MTX. Erythrocyte methotrexate and/or folate polyglutamate levels can also be used to predict a patient's response to MTX.

### SUMMARY

In this example, 48 adult patients naïve to MTX were enrolled in a prospective longitudinal study. MTX therapy was initiated at 7.5 mg/week and increased every 4-6 weeks until a therapeutic response was achieved. Response was assessed using the change in disease activity score. Erythrocyte methotrexate and folate polyglutamate levels were measured with 9 common polymorphisms in the folate pathway. Statistical analysis consisted of generalized linear models and multivariate regressions.

A poor response was associated with low formation of erythrocyte methotrexate polyglutamates (MTXPGs) despite high MTX doses administered. A decrease in red blood cell (RBC) folate polyglutamates (folate PGs) resulted in an 8-fold higher likelihood of response (p < 0.01). The *MTHFR* 677T/T, TS *2/*2, and *SHMT1* 1420C/T or T/T genotypes contributed to a poor response to MTX therapy (p < 0.001). An efficacy index cumulating these risk genotypes revealed that an efficacy index above an index cutoff value of 1 was associated with higher RBC folate PG levels and a 6.3-fold greater likelihood of having a poor response.

The *GGH* -401C/C, *ATIC* 347G/G, *MTHFR* 1298A/C or C/C, *MS* 2756A/A, and *MTRR* 66G/G genotypes were associated with toxicity (p < 0.01). Side-effects consisted mainly of gastrointestinal and neurological toxicities (*e.g*., headache, lethargy). A toxicogenetic index cumulating these risk genotypes revealed that a toxicogenetic index above an index cutoff value of 1 was associated with a 14-fold higher likelihood of having side-effects.

### METHODS

Patients and Study Protocol: The study was a prospective longitudinal study in adult rheumatoid arthritis patients (≥18 yr) who were naïve to MTX treatment. All patients had to meet the revised diagnostic criteria of the American Rheumatism Association for Rheumatoid Arthritis. All patients were studied at a single center (The Center for Rheumatology; Albany, NY). An Institutional Review Board approved the study, and patient consent was obtained for each patient. Oral MTX therapy was initiated at 7.5 mg/week and increased by 2.5 mg/week every 4-6 weeks until a therapeutic response was achieved. The clinical assessment at each study visit consisted of a tender and swollen joint count, a patient's assessment of disease activity (10 cm-VAS), a physician's assessment of disease activity (10 cm-VAS), and a C-Reactive protein (CRP) determination. The decision to modify the MTX dose was at the discretion of the physician and was based on both efficacy and toxicity considerations at each visit. Treating physicians were blinded to RBC MTXPG, RBC folate PG, and pharmacogenetic biomarker measurements throughout the entire study. Concurrent medications allowed included corticosteroids, sulphasalazine, and hydroxychloroquine. Patients were started on 1 mg folic acid daily at the time of MTX initiation.

The occurrence of side-effects was recorded at the time of each study visit. MTX side-effects were defined as those affecting the gastrointestinal tract (*e.g*., nausea, diarrhea, stomatitis, dyspepsia, elevation of aspartate aminotransferase (AST) above the upper limit of normal (40 U/L)), the central nervous system (e.g., headache, lethargy), the hematopoietic system (*e.g.,* white blood cell count < 3500/mm³, hemoglobin < 80g/L, MCV > 120 fmol), and the lung (*e.g*., cough, dyspnea, pulmonary infiltrate). Leukocyte count, hemoglobin, and liver AST were measured on the day of the study visit using standard laboratory methods. Toxicity was assessed by asking each patient if they were experiencing any side-effects at the time of each study visit, and each category of MTX related side-effects was explored. All data were captured on Case Report Forms using a standardized questionnaire. This consisted of the presence of nausea, dyspepsia, diarrhea, headache, lethargy, stomatitis, alopecia, and pulmonary toxicity (*e.g.,* cough, dyspnea, pulmonary infiltrates). Patients were evaluated by the same physician at the time of each study visit. Toxicities were graded as "mild," "moderate," and "severe." EDTA whole blood was drawn from each patient and shipped overnight from Albany, NY to Prometheus Laboratories in San Diego, CA.

Laboratory measurements: Red blood cell (RBC) long-chain MTXPG concentrations (MTXPG₃; nmol/L RBC) were measured using a post-column photo-oxidation HPLC-fluorometry technique as described in, *e.g.,* Dervieux et al., Clin. Chem., 49:1632-1641 (2003). RBC folate polyglutamates (Folate PGs; expressed as nmol/L RBCs) were measured using a radio-assay (Biorad; Hercules, CA). The *MTHFR* C677T polymorphism can be measured with a real-time TaqMan allelic discrimination assay as described above. The *MS* A2756G polymorphism, which results in an aspartic acid to glycine substitution at codon 919 of *MS,* can be measured with a real-time TaqMan allelic discrimination assay using the following primers and fluorogenic 3'-minor groove binding probes: forward primer: 5'-GAA TAC TTT GAG GAA ATC ATG GAA GA-3' (SEQ ID NO:17); reverse primer: 5'-TCT GTT TCT ACC ACT TAC CTT GAG AGA CT-3' (SEQ ID NO:18); wild-type fluorescent probe: 5'-FAM-AGA CAG GAC CAT TAT G-MGB-3' (SEQ ID NO:19); and mutant fluorescent probe: 5'-VIC-ACA GGG CCA TTA TG-MGB-3' (SEQ ID NO:20). The *MTRR* A66G polymorphism, which results in a methionine to isoleucine substitution at codon 22, can be measured with a real-time TaqMan allelic discrimination assay using the following primers and fluorogenic 3'-minor groove binding probes: forward primer: 5'-GCA AAG GCC ATC GCA GAA-3' (SEQ ID NO:21); reverse primer: 5'-GAT CTG CAG AAA ATC CAT GTA CCA-3' (SEQ ID N0:22); wild-type fluorescent probe: 5'-FAM-TGC TCA CAT ATT TC-MGB-3' (SEQ ID NO:23); and mutant fluorescent probe: 5'-VIC-CTT GCT CAC ACA TTT-MGB-3' (SEQ ID NO:24). The final conditions for the assays can be 900 nM of each primer, 200 nM of each probe, with 5 ng genomic DNA and a 1X TaqMan master mix (Applied Biosystem; Foster City, CA). PCR conditions can consist of one 2-minute cycle at 50°C followed by a 10-minute cycle at 95°C followed by 40 cycles of 95°C for 15 seconds, 58°C for 15 seconds, and finally 60°C for 45 seconds. Polymorphisms in gamma-glutamyl hydrolase (*GGH*-401C/T), serine hydroxymethyltransferase *(SHMT1* C1420T), AICAR transformylase *(ATIC* C347G), and thymidylate synthase *(TS* *2/*2) were measured as described in, *e.g.,* Dervieux et al., Arthritis Rheum., 50:2766-2774 (2004); Dervieux et al., Pharmacogenetics, 14:733-739 (2004); and Skibola et al., Blood, 99:3786-3791 (2002).

Statistical analysis: The disease activity score (DAS28) for each patient was calculated as follows using the number of tender joint count (maximum 28), swollen joint count (maximum 28), CRP level (in mg/L), and the patient's assessment of disease activity (10cm-VAS): DAS28 = 0.56 x (tender joint count)^{1/2} + 0.28 x (swollen joint coint)^{1/2} + 0.36*In(CRP + 1) + 0.14 x (patient's assessment of disease activity) + 0.96. In two patients, the DAS28 was calculated using the erythrocyte sedimentation rate and the appropriate formula (*see, e.g.,* http://www.das-score.nl). Response to therapy was assessed using the EULAR (European League Against Rheumatism) response criteria (change in DAS28 from baseline and level of attained) (van Gestel et al., J. Rheumatol., 26:705-711 (1999)). Therapeutic response was defined as "good response," "moderate response," or "poor response." Patients presenting a moderate to good response were categorized as "responders" and compared to non-responders (*i.e*., those with a poor response) as appropriate. The percentage change in the physician's assessment of patient's disease activity visual analogue scale or alternatively in the percentage change in DAS score from baseline were also calculated.

Group comparisons were performed using the Wilcoxon exact test or the Kruskall-Wallis test as appropriate. Longitudinal data were analyzed using a generalized linear model. Multivariate linear or logistic regression was also used. The *MTHFR* 677T/T, *MTHFR* 1298A/C or C/C, *MS* 2756A/A, *MTRR* 66G/G, *SHMT1* 1420C/T or T/T, *RFC-1* 80A/A, *GGH* -401C/C, *ATIC* 347G/G, and *TS* *2/*2 genotypes were considered. Toxicities were analyzed using the percentage of 4-6 week periods with side-effects per patient. Adjustments for concurrent medication (*e.g*., DMARDs, NSAIDs, and prednisone) were made as appropriate. Statistical analyses were performed using the software package SAS (Release 8.2; SAS Institute Inc.; Cary, NC).

### RESULTS

### Patients

A total of 48 patients enrolled from November 2002 to March 2004 received MTX for an average of 6.9 ± 1.4 months. All patients were followed for 4 consecutive study visits (4 months on MTX) and 35 (73%) were followed for 2 additional study visits (6 months on MTX). Patient demographics and characteristics are shown in Table 3. Allelic frequencies for common polymorphisms measured are shown in Table 4.

**Table 3: Clinical characteristics of the patients enrolled in the study. Results are expressed as median (interquartile range) or number (%) as appropriate.**

| **Parameter** | **Value** |
|---|---|
| Age (yr) | 55 (45-64) |
| Number of years with rheumatoid arthritis | 1.0 (0.3-5.0) |
| Low dose corticosteroids | 28 (58.3%) |
| Folic acid supplementation | 46 (95.8%) |
| Concurrent DMARD | 14 (29.1 %) |
| Concurrent NSAIDs | 30 (62.5%) |
| DAS28: | |
| baseline | 5.1 (4.5-6.0) |
| visit 4 | 3.5 (2.7-4.2) |
| visit 6 | 3.1 (2.3-3.7) |
| Physician's assessment of disease activity 10 cm VAS | |
| baseline | 5.6 (4.8-6.6) |
| visit 4 | 2.6 (1.4-3.7) |
| visit 6 | 2.1 (0.7-4.6) |

**Table 4: Allelic frequency of common polymorphisms in folate pathway genes.**

| | Allelic Frequency CI 95% | Risk genotype frequency (number) |
|---|---|---|
| *GGH-*401 C/T | 21% (12%-30%) | -401C/C: 65% (n = 31) |
| *MTHFR* C677T | 39% (29%-48%) | 677T/T: 12% (n=6) |
| *MTHFR* A1298C | 33% (23%-44%) | 1298A/C or C/C: 52% (n = 25) |
| *ATIC* C347G | 30% (20%-41%) | 347G/G: 15% (n = 7) |
| *MS* A2756G | 20% (11%-28%) | 2756A/A: 65% (n = 31) |
| *TS* *2/*3 | 46% (34%-57%) | *2/*2: 27% (n = 13) |
| *MTRR* A66G | 60% (50%-71 %) | 66G/G: 37% (n = 18) |
| *SHMT1* C1420T | 25% (16%-34%) | 1420C/T or T/T: 44% (n = 21) |
| *RFC-1* G80A | 44% (34%-53%) | 80A/A: 17% (n = 8) |

### Efficacy

The change in DAS28 and EULAR response criteria was evaluated in 47 patients at visit 4 and in 34 patients at visit 6. In one patient, a missing joint count precluded the determination of the DAS28 change. Median decrease (from baseline) in DAS28 was 36% (interquartile range: 18-45%; n = 47) at visit 4 and 41% (interquartile range: 28-55%; n = 34) at visit 6 (DAS28 values are provided in Table 3 above). Median decrease (from baseline) in the physician's assessment of disease activity VAS was 47% (interquartile range: 32-77%; n = 48) and 52% (interquartile range: 32-87; n = 35) at visit 4 and 6, respectively.

At the fourth study visit (average 4.6 months under MTX), the median MTX dose administered was 15 mg/week (interquartile range: 12.5-15 mg/week). A total of 11 patients (23%) presented a poor response, while 36 patients (77%) were responding to therapy (moderate response in 11 patients; good response in 15 patients). A generalized linear model indicated that lower RBC MTXPG levels tended to result in a lower likelihood of response (Figure 3A). Weekly MTX dose escalation was not associated with response at visit 4 (p = 0.63). However, higher weekly MTX doses were administered to patients with a lesser decrease (less improvement) in the physician's assessment of disease activity VAS (percentage change from baseline, estimate = 0.043 ± 0.017; p = 0.0097), and higher levels of RBC MTXPG resulted in a greater decrease (greater improvement) in the physician's assessment of disease activity VAS (percentage change from baseline, estimate = -0.013 ± 0.004; p = 0.0002) (Figure 3B). Thus, an escalation of weekly MTX dose from 7.5 to 15 mg/week with an increase in MTXPG levels of 40 mnol/L RBCs at visit 4 (40 unit change) resulted in a 19.8% decrease in the physician's assessment of disease activity compared to baseline (equation = 100 x (0.043 x 7.5 - 0.013 x 40)). There were no significant associations between the change in the DAS28 and weekly MTX dose or RBC MTXPGs. These observations remained unchanged after adjustment with concurrent medication (DMARDs, NSAIDs; and prednisone).

At the time of the sixth study visit (average of 7.5 months under MTX), the median weekly MTX dose was 17.5 mg (interquartile range: 15-20 mg/week; n = 35). Only three patients (9%) were non-responders and 10 (32%) of the 31 responders presented a moderate response. Non-responders tended to present lower RBC MTXPG levels (27 ± 6 nmol/L RBCs) than responders (40 ± 18 nmol/L RBCs) (p = 0.17) at visit 6. A generalized linear model indicated that a lesser decrease (less improvement) in the physician's assessment of disease activity (percentage change from baseline) was associated with a higher weekly MTX dose administered (estimate = 0.029 ± 0.012; p = 0.023) and low RBC MTXPG levels (estimate = -0.0077 ± 0.025; p = 0.0018) (Figures 3C and 3D). Similarly, a lesser decrease in the DAS28 (percentage change from baseline) was associated with a higher MTX dose administered (estimate = 0.011 ± 0.051; p = 0.023) and lower formation of RBC MTXPGs (estimate = -0.003 ± 0.001; p = 0.0046). These observations remained unchanged after adjustment with concurrent medications (DMARDs, NSAIDs, and prednisone).

Median RBC folate PG levels measured at the initial visit (38 patients) or in the first month following enrollment (5 patients) were 1222 nmol/L RBCs (interquartile range: 1006-1513 nmol/L; n = 43). In 5 patients the change in RBC folate PG levels could not be evaluated because of insufficient blood volume. RBC folate PG levels decreased to a median of 1065 nmol/L RBCs (interquartile range: 547-1334 nmol/L RBCs) at the fourth study visit (compared to baseline; p = 0.002; n = 43). As shown in Figure 4, response to therapy at visit 4 was associated with a decrease in RBC folate PG levels from baseline. In fact, patients experiencing a decrease in RBC folate PGs were 7.7-fold more likely to achieve a response to MTX (OR CI 95%: 1.4-40.8; p = 0.017) compared to patients with either no change or an increase in RBC folate PGs. The percentage change in DAS28 or in the physician's assessment of disease activity VAS from baseline to visit 4 was associated with the percentage change in folate PG levels from baseline to visit 4 (R² = 0.200; estimate: 0.32 ± 0.10 and R² = 0.201; estimate: 0.71 ± 0.22, respectively; p < 0.01). At the sixth visit, median RBC folate PG levels were 994 nmol/L RBCs (interquartile range: 828-1290 nmol/L RBCs) and similar associations were observed (p < 0.06).

In a generalized linear model, no association was detected between the change in folate PG levels and either MTX dose or RBC MTXPGs at the fourth visit (p < 0.37). However, a greater decrease in RBC folate PGs from baseline to visit 6 was associated with a low MTX dose administered (estimate = 0.0324 ± 0.014; p = 0.018) and high MTXPG levels (estimate = -0.009 ± 0.003; p = 0.0022). Thus, an increase in MTX dose from 7.5 to 15 mg/week (7.5 unit change), which achieved an increase in MTXPG concentration of 40 nmol/L RBCs (40 unit change) at visit 6, resulted in a 11.7% decrease in folate levels (equation = 100 x (0.0324 x 7.5 - 0.009 x 40)). Also, in a multivariate analysis, a lesser decrease (less improvement) in the physician's assessment of disease activity VAS (from baseline to visit 4) was associated with a higher MTX dose administered (p = 0.026), a lower RBC MTXPG concentration (p = 0.006), and a higher RBC folate PG concentration (p = 0.02) measured at visit 4 (Global R² = 0.253). Similar results were observed at the sixth visit

In a generalized linear model including MTX dose and RBC MTXPGs, this study revealed that the *MTHFR* 677T/T (OR = 9.9; p < 0.001), *SHMT1* 1420 C/T or T/T (OR = 4.5; p < 0.001), and TS *2/*2 (OR = 3.9; p = 0.003) genotypes were associated with a lower likelihood of response to therapy at the fourth study visit. Similarly, the presence of these risk genotypes were associated with a lower decrease in the physician's assessment in disease activity (*MTHFR* 677T/T, estimate: 0.367, p < 0.001; *SHMT1* 1420 C/T or T/T, estimate: 0.252, p < 0.001; TS *2/*2, estimate: 0.360, p < 0.001). These risk genotypes were summed to construct an efficacy index for each patient. The efficacy index ranged from between 0 and 2. As shown in Figure 5, an increased number of risk genotypes was associated with a higher percentage of poor responders (p = 0.036) and a lower decrease in the disease activity score (p = 0.028). There was no difference in RBC folate PG levels at initiation of therapy between the various efficacy indexes (1206 ± 396 for and index of 0; 1304 ± 441 for an index of 1; and 1377 ± 490 nmol/L for an index of 2; p=0.52). However, at the fourth visit, patients with an efficacy index above an index cutoff value of 1 had higher RBC folate PG levels (1257 ± 70 nmol/L; 14% decrease versus baseline) than those with an index of 0 (886 ± 59 nmol/L; 36% decrease versus baseline) or 1 (1168 ± 70 nmol/L; 42% decrease versus baseline) (p = 0.0052), and also a 6.3-fold (OR CI 95%: 1.09-36.2; p = 0.03) greater likelihood of having a poor response.

### Toxicity

A total of 40 patients (83%) described some toxicity at the time of at least one visit, and the median percentage of a 4-6 week period with side-effects was 50% (range 0-100%). The occurrence of side-effects is presented in Table 5 below. Gastrointestinal and central nervous system toxicities were the most frequent MTX-related side-effects observed. There was no significant change in the percentage of patients with gastrointestinal side-effects or central nervous system side-effects over the six study visits. Four patients displayed signs of hepatotoxicity (elevation of the AST above the upper normal limit). None of the patients presented signs of hematological toxicity. The erythrocyte mean corpuscular volume (MCV) increased from 89 fl (median; interquartile range: 87-92 fl) to 93 f1 (median; interquartile range: 88-97 fl) from baseline to visit 4 (median increase 3.5%; p < 0.001), but none of the patients presented an increase in MCV above 120 fl. At visit 6, the median increase was 3.7% and only one patient presented a MCV of 124 fl. A total of 4 patients experienced a severe side-effect in one of the study visits (2 with severe lethargy and 2 with severe dyspepsia). Two patients required MTX dosage patient interruption (both at the fourth visit; one patient with gastrointestinal and central nervous system toxicities and one patient with gastrointestinal toxicity).

**Table 5: Occurrence of side-effects. Each patient was evaluated by the treating physician every 4-6 weeks for a total of 4 to 6 visits.**

| | Number of patients (% total) with at least one study visit with side-effect | Median (range) of percentage of study visits with side-effect per patient |
|---|---|---|
| All side-effects | 40 (83%) | 50% (0-100%) |
| | | |
| Gastrointestinal tract | 33 (69%) | 25% (0%-100%) |
| nausea | 22 (46%) | 0% (0%-100%) |
| diarrhea | 15(31%) | 0% (0%-75%) |
| dyspepsia | 16 (33%) | 0% (0%-100%) |
| stomatitis | 5 (10%) | 0% (0%-67%) |
| AST>40U/L | 4 (8%) | 0% (0%-75%) |
| | | |
| Central nervous system | 30 (63%) | 25% (0%-100%) |
| Headache | 14 (29%) | 0% (0%-100%) |
| Lethargy | 27 (56%) | 17% (0%-83%) |
| | | |
| Alopecia | 4 (8%) | 0% (0-67%) |
| | | |
| Cough | 12 (25%) | 0% (0-100%) |
| | | |
| Dyspnea | 1 (2.1%) | 0% (0-16%) |

In a generalized linear model, RBC MTXPG and folate PG levels were not associated with the occurrence of side-effects (p > 0.09). However, a higher MTX dose administered was associated with an increased occurrence of central nervous system side-effects (estimate = 0.186; p = 0.036) but not gastrointestinal side-effects (p = 0.31). A multivariate analysis revealed that an increased occurrence of side-effects was associated with the presence of the *GGH*-401C/C, *ATIC* 347G/G, *MTHFR* 1298A/C or C/C, *MTRR* 66G/G, and *MS* 2756A/A genotypes (Table 6 below). These risk genotypes were summed to generate a toxicogenetic index for each patient (median = 2; range = 1-4). An increased toxicogenetic index was associated with an increased occurrence of gastrointestinal and central nervous system side-effects (Figure 6; p < 0.001). Patients with a toxicogenetic index above an index cutoff value of 1 were 13.9-fold more likely to complain of a side-effect in 50% of the study visits compared to those with a toxicogenetic index less than or equal to the index value (OR CI 95%: 2.6-75.4; p = 0.006).

**Table 6: Multivariate analysis of the percentage of 4 week periods with side-effects. The dependant variables are the percentage of periods with side-effects, the percentage of periods with gastrointestinal side-effects, and the percentage of periods with central nervous system side-effects. The multivariate analysis included all the risk genotypes described herein. The table present estimates for the GGH -401C/C, MTHFR 1298A/C or C/C, ATIC 347G/G, MS 2756A/A, and MTRR 66G/G genotypes. Not shown are estimates for the SHMT1 1420C/T or T/T, MTHFR 677T/T, and RFC-1 80A/A genotypes (not significant for all; p > 0.15).**

| | All side-effects | Gastrointestinal side-effects | Central nervous system side-effects |
|---|---|---|---|
| N | 48 | 48 | 48 |
| Global R² | 0.380 | 0.493 | 0.358 |
| *GGH* -401C/C vs. *GGH* -401T/T or C/T | 0.38 ± 0.10* | 0.31 ± 0.09* | 0.24 ± 0.10* |
| *MTHFR* 1298A/C or C/C vs. *MTHFR* 1298A/A | 0.18 ± 0.10 | 0.25 ± 0.09* | 0.21 ± 0.10* |
| *ATIC* 347G/G vs. *ATIC* 347C/C or C/G | 0.26 ± 0.13* | 0.27 ± 0.11* | 0.27 ± 0.11* |
| *MS* 2756A/A vs. *MS2756* A/G or G/G | 0.20 ± 0.09* | 0.29 ± 0.08* | 0.11 ± 0.09 |
| *MTRR* 66G/G vs. *MTRR* 66A/A or A/G | 0.22 ± 0.10* | 0.28 ± 0.09* | 0.16 ± 0.09 |

| | | | |
|---|---|---|---|
| *p<0.05 | | | |

### DISCUSSION

Methotrexate (MTX) remains the most frequently prescribed DMARD in patients with rheumatoid arthritis, and the common practice is to initiate MTX at a low dose (e.g., 7.5 mg/week) and to empirically increase the dose until beneficial results are observed. MTX is a slow-acting DMARD, and the time required to achieve a maximum therapeutic effect has been shown to be 6 to 9 months (Kremer et al., Arthritis Rheum., 29:822-831 (1986)). However, recent evidence suggests that the time to reach an optimal MTX dosage is longer than initially thought and that a significant delay in MTX dose escalation may result in loss of effects (Ortendahl et al., J. Rheumatol., 29:2084-2091 (2002)). Thus, it has been suggested that new approaches may be required to better optimize MTX dose. A large body of evidence suggests that MTX effects are mediated through polyglutamation to long-chain MTXPGs that inhibit *de novo* purine synthesis and promote the release of adenosine (Kremer, Arthritis Rheum., 50:1370-1382 (2004); Dervieux et al., Arthritis Rheum., 50:2766-2774 (2004)). This study demonstrates that MTXPG levels are associated with therapeutic response, and that MTXPG metabolites can be measured in erythrocytes, a convenient and accessible surrogate to hematopoeitic cells such as lymphocytes. All patients enrolled in this study were naïve to MTX and the dose was initiated at 7.5 mg/week and increased every 4-6 weeks until a benefit was observed or a dose-limiting toxicity was reported. This study revealed that a poor response to MTX (based on the EULAR response criteria, change in the disease activity score and physician's assessment of disease activity) was associated with a low formation of RBC MTXPGs. Interestingly; a poor response was also associated with higher MTX doses administered.

The depletion in erythrocyte folate PG levels was an important determinant of response and suggests that the anti-folate effects of MTX (probably through DHFR inhibition) may contribute to the immunosuppressive and anti-inflammatory effects of the drug. These observations are supported by *in vitro* findings, as folate deficiency inhibits the proliferation of human CD8⁺ T Lymphocytes (Courtemanche et al., J. Immunol., 173:3186-3192 (2004)). If the association between a decrease in folate PG levels and therapeutic response is causal, it would follow that folic acid supplementation (which results in increased folate PG levels) could partially antagonize the therapeutic response (Whittle et al., Rheumatology (Oxford), 43:267-271 (2004); Manna et al., Rheumatology (Oxford), 44:563-564 (2005)).

Individuals with the *SHMT1* 1420C/T or T/T genotype have higher red blood cell folate levels than those with the 1420C/C genotype (Heil et al., Mol. Genet. Metab., 73:164-172 (2001)), and this activating mutation appears to confer a protective effect against the development of acute lymphocytic leukemia (Skibola et al., Blood, 99:3786-3791 (2002)). This study demonstrates that genotypes associated with increased 5,10-methylenetetrahydrofolate levels available for *de novo* purine synthesis either through increased synthesis (as seen in those with the *SHMT1* 1420C/T or T/T genotype) or decreased consumption by alternative routes (as seen in those with the *MTHFR* 677T/T or TS *2/*2 genotypes) are associated with a less robust response to MTX. This study also revealed that the summation of these three risk genotypes into an efficacy index maximized the phenotypic penetrance.

In this dose escalation study, MTX toxicity occurred frequently but did not appear to be strongly dose-dependent or related to RBC MTXPG and folate PG levels. *MTHFR* C677T was not associated with the occurrence of side-effects, which could be explained by the large number of patients receiving folic acid supplementation (van Ede et al., Arthritis Rheum., 44:2525-2530 (2001)). However, the *MTHFR* 1298A/C or C/C genotype was associated with increased risk of toxicity. Other genotypes associated with an increased risk of toxicity were in the *GGH* promoter and the *ATIC* gene.

The contribution of genotypes in homocysteine remethylation-dependent enzymes such as methionine synthase (*MS* A2756G) and methionine synthase reductase (*MTRR* A66G) was also evaluated. MS catalyses the remethylation of homocysteine to methionine in the presence of methylcobalamin, a cofactor synthesized by MTRR (Figure 1). Recently, an A2756G polymorphism in the open reading frame of *MS* was shown to result in increased homocysteine levels, decreased folate levels, and decreased cobalamin levels in patient carriers of the 2756A variant versus those with the 2756G variant (Miriuka et al., Transpl. Int., 18:29-35 (2005); Silaste et al., J. Nutr., 131:2643-2647 (2001); Chen et al., Atherosclerosis, 154:667-672 (2001); Harmon et al., Genet. Epidemiol., 17:298-309 (1999)). Conversely, an A66G polymorphism in *MTRR* was associated with decreased homocysteine levels, increased folate levels, and increased cobalamin levels in those with the 66A variant versus those with the 66G variant (Miriuka *et al., supra*; Gaughan et al., Atherosclerosis, 157:451-456 (2002) (*see*, corrigendum 167:373 (2002)). Thus, both variants appear to have an opposite contribution to homocysteine remethylation activity. In the population of patients in this study, those with the homozygous wild-type MS 2756A/A or homozygous mutant *MTRR* 66G/G genotype were more likely to experience gastrointestinal side-effects (50% of the study visits) compared to those without these risk genotypes. These observations indicate that decreased MS activity (as seen in those with the *MS* 2756A/A genotype) and decreased MTRR activity (as seen in those with the *MTRR* 66G/G genotype) result in a low homocysteine remethylation status and increased risk of MTX toxicity. Conversely, patients with a high homocysteine remethylation status are protected against the development of gastrointestinal side-effects.

Finally, the sum of the risk genotypes was converted to a toxicogenetic index maximizing phenotypic penetrance. Increased genotypic values were associated with an increased occurrence of side-effects. These genetic associations strongly support the notion of a genetic basis for the variable response to anti-folate therapy such as MTX therapy.

All publications and patent applications cited in this specification are herein incorporated by reference as if each individual publication or patent application were specifically and individually indicated to be incorporated by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

In particular, the present invention relates to the following aspects and embodiments:
1. The present invention relates to an assay method for evaluating the likelihood that a subject will respond to methotrexate (MTX), said method comprising:
   (a) determining the genotype of at least one folate pathway gene selected from the group consisting of a methylenetetrahydrofolate reductase (MTHFR) gene, a thymidylate synthase (TS) gene, a serine hydroxymethyltransferase (SHMTl) gene, and a combination thereof in a sample from said subject;
   (b) generating an efficacy index based upon the genotype of said at least one folate pathway gene; and
   (c) evaluating the likelihood that said subj ect will respond to MTX based upon said efficacy index.
2. The method of embodiment 1, wherein said subj ect has a disease selected from the group consisting of an inflammatory disease, an autoimmune disease, and cancer.
3. The method of embodiment 2, wherein said inflammatory disease is rheumatoid arthritis.
4. The method of embodiment 1, wherein the genotype of said at least one folate pathway gene is determined at a polymorphic site.
5. The method of embodiment 4, wherein said polymorphic site is a single nucleotide polymorphism (SNP).
6. The method of embodiment 1, wherein the genotype of said MTHFR gene is selected from the group consisting of MTHFR 677C/C, MTHFR 677C/T, and MTHFR 677T/T.
7. The method of embodiment 1, wherein the genotype of said TS gene is selected from the group consisting of TS *3/*3, TS *3/*2, and TS *2/*2.
8. The method of embodiment 1, wherein the genotype of said SHMTl gene is selected from the group consisting of SHMTl 1420C/C, SHMTl 1420C/T, and SHMTl 1420T/T.
9. The method of embodiment 1, wherein said efficacy index is generated based upon the genotype of said MTHFR, TS, and SHMTl genes.
10. The method of embodiment 1, wherein said efficacy index is compared to an index cutoff value.
11. The method of embodiment 10, wherein an efficacy index greater than said index cutoff value indicates that said subject does not have a high likelihood of responding to MTX.
12. The method of embodiment 11, further comprising recommending a high dose of MTX or an alternative therapy to be administered to said subject.
13. The method of embodiment 10, wherein an efficacy index less than or equal to said index cutoff value indicates that said subject has a high likelihood of responding to MTX.
14. The method of embodiment 13, further comprising recommending a low dose of MTX to be administered to said subject.
15. The method of embodiment 10, wherein an efficacy index less than or equal to said index cutoff value indicates that said subject has a moderate likelihood of responding to MTX.
16. The method of embodiment 15, further comprising recommending an intermediate dose of MTX to be administered to said subject.
17. The method of embodiment 1, wherein said sample is selected from the group consisting of whole blood, serum, plasma, and buccal cells.
18. The present invention further relates to an assay method for evaluating the risk that a subject will develop toxicity to methotrexate (MTX), said method comprising:
   (a) determining the genotype of at least one folate pathway gene selected from the group consisting of a methylenetetrahydrofolate reductase (MTHFR) gene, a thymidylate synthase (TS) gene, a serine hydroxymethyltransferase (SHMTl) gene, an aminoimidazole carboxamide ribonucleotide transformylase (ATIC) gene, a gamma-glutamyl hydrolase (GGH) gene, a methionine synthase (MS) gene, a methionine synthase reductase (MTRR) gene, and a combination thereof in a sample from said subject;
   (b) generating a toxicogenetic index based upon the genotype of said at least one folate pathway gene; and
   (c) evaluating the risk that said subject will develop toxicity to MTX based upon said toxicogenetic index.
19. The method of embodiment 18, wherein said subject has a disease selected from the group consisting of an inflammatory disease, an autoimmune disease, and cancer.
20. The method of embodiment 19, wherein said inflammatory disease is rheumatoid arthritis.
21. The method of embodiment 18, wherein the genotype of said at least one folate pathway gene is determined at a polymorphic site.
22. The method of embodiment 21, wherein said polymorphic site is a single nucleotide polymorphism (SNP).
23. The method of embodiment 18, wherein the genotype of said MTHFR gene is selected from the group consisting of MTHFR 677C/C, MTHFR 677C/T, MTHFR 677T/T, MTHFR 1298A/A, MTHFR 1298A/C, and MTHFR 1298C/C.
24. The method of embodiment 18, wherein the genotype of said TS gene is selected from the group consisting ofTS *3/*3, TS *3/*2, and TS *2/*2.
25. The method of embodiment 18, wherein the genotype of said SHMTl gene is selected from the group consisting of SHMTl 1420C/C, SHMTl 1420C/T, and SHMTl 1420T/T.
26. The method of embodiment 18, wherein the genotype of said ATIC gene is selected from the group consisting of ATIC 347C/C, ATIC 347C/G, and ATIC 347G/G.
27. The method of embodiment 18, wherein the genotype of said GGH gene is selected from the group consisting of GGH -401T/T, GGH -401C/T, and GGH -401C/C.
28. The method of embodiment 18, wherein the genotype of said MS gene is selected from the group consisting of MS 2756A/A, MS 2756A/G, and MS 2756G/G.
29. The method of embodiment 18, wherein the genotype of said MTRR gene is selected from the group consisting of MTRR 66A/A, MTRR 66 A/G, and MTRR 66G/G.
30. The method of embodiment 18, wherein said toxicogenetic index is generated based upon the genotype of said MTHFR, ATIC, TS, and SHMTl genes.
31. The method of embodiment 18, wherein said toxicogenetic index is generated based upon the genotype of said MTHFR, ATIC, GGH, MTRR, and MS genes.
32. The method of embodiment 18, wherein said toxicogenetic index is compared to an index cutoff value.
33. The method of embodiment 32, wherein said toxicogenetic index greater than said index cutoff value indicates that said subject is at high risk of developing toxicity to MTX.
34. The method of embodiment 33, further comprising recommending a low dose of MTX or an alternative therapy to be administered to said subject.
35. The method of embodiment 32, wherein said toxicogenetic index greater than said index cutoff-value indicates that said subject is at moderate risk of developing toxicity to MTX.
36. The method of embodiment 35, further comprising recommending an intermediate dose of MTX to be administered to said subject.
37. The method of embodiment 32, wherein said toxicogenetic index less than or equal to said index cutoff value indicates that said subject is not at high risk of developing toxicity to MTX.
38. The method of embodiment 37, further comprising recommending a high dose of MTX to be administered to said subject.
39. The method of embodiment 18, wherein said toxicity is selected from the group consisting of a gastrointestinal side-effect, a central nervous system side-effect, a hematopoietic system side-effect, a pulmonary system side-effect, alopecia, and a combination thereof
40. The method of embodiment 18, wherein said sample is selected from the group consisting of whole blood, serum, plasma, and buccal cells.
41. The present invention further relates to an assay method for optimizing dose efficacy in a subject receiving methotrexate (MTX), said method comprising:
   (a) determining the genotype of at least one folate pathway gene selected from the group consisting of a methylenetetrahydrofolate reductase (MTHFR) gene, a thymidylate synthase (TS) gene, a serine hydroxymethyltransferase (SHMTl) gene, and a combination thereof in a sample from said subject;
   (b) generating an efficacy index based upon the genotype of said at least one folate pathway gene; and
   (c) recommending a subsequent dose of MTX based upon said efficacy index.
42. The method of embodiment 41, wherein said subject has a disease selected from the group consisting of an inflammatory disease, an autoimmune disease, and cancer.
43. The method of embodiment 41, wherein the genotype of said at least one folate pathway gene is determined at a polymorphic site.
44. The method of embodiment 41, wherein said efficacy index is generated based upon the genotype of said MTHFR, TS, and SHMTl genes.
45. The method of embodiment 41, wherein said efficacy index is compared to an index cutoff value.
46. The method of embodiment 45, wherein said efficacy index greater than said index cutoff value indicates that the subsequent dose of MTX should be increased or an alternative therapy should be administered.
47. The method of embodiment 45, wherein said efficacy index less than or equal to said index cutoff value indicates that the subsequent dose of MTX should be maintained.
48. The method of embodiment 41, wherein said sample is selected from the group consisting of whole blood, serum, plasma, and buccal cells.
49. The present invention further relates to an assay method for reducing toxicity in a subject receiving methotrexate (MTX), said method comprising:
   (a) determining the genotype of at least one folate pathway gene selected from the group consisting of a methylenetetrahydrofolate reductase (MTHFR) gene, a thymidylate synthase (TS) gene, a serine hydroxymethyltransferase (SHMTl) gene, an aminoimidazole carboxamide ribonucleotide transformylase (ATIC) gene, a gamma-glutamyl hydrolase (GGH) gene, a methionine synthase (MS) gene, a methionine synthase reductase (MTRR) gene, and a combination thereof in a sample from said subj ect;
   (b) generating a toxicogenetic index based upon the genotype of said at least one folate pathway gene; and
   (c) recommending a subsequent dose of MTX based upon said toxicogenetic index.
50. The method of embodiment 49, wherein said subject has a disease selected from the group consisting of an inflammatory disease, an autoimmune disease, and cancer.
51. The method of embodiment 49, wherein the genotype of said at least one folate pathway gene is determined at a polymorphic site.
52. The method of embodiment 49, wherein said toxicogenetic index is generated based upon the genotype of said MTHFR ATIC, TS, and SHMTl genes.
53. The method of embodiment 49, wherein said toxicogenetic index is generated based upon the genotype of said MTHFR, ATIC, GGH, MTRR, and MS genes.
54. The method of embodiment 49, wherein said toxicogenetic index is compared to an index cutoff value.
55. The method of embodiment 54, wherein said toxicogenetic index greater than said index cutoff value indicates that the subsequent dose of MTX should be decreased or an alternative therapy should be administered.
56. The method of embodiment 54, wherein said toxicogenetic index less than or equal to said index cutoff value indicates that the subsequent dose of MTX should be maintained.
57. The method of embodiment 49, wherein said sample is selected from the group consisting of whole blood, serum, plasma, and buccal cells.
58. The present invention further relates to an assay method for evaluating the likelihood that a subject will respond to methotrexate (MTX), said method comprising:
   (a) determining a level of methotrexate polyglutamates (MTXPGs) in a sample from said subject; and
   (b) evaluating the likelihood that said subject will respond to MTX based upon the level of MTXPGs.
59. The method of embodiment 58, wherein the level of at least one long-chain MTXPG is determined.
60. The method of embodiment 59, wherein said at least one long-chain MTXPG is selected from the group consisting of MTXPG₃, MTXPG₄, MTXPG₅, MTXPG₆, MTXPG₇, and a combination thereof.
61. The method of embodiment 60, wherein said at least one long-chain MTXPG is MTXPG₃.
62. The method of embodiment 61, wherein the level of MTXPG₃ is predictive of the level of MTXPG₃₋₅, MTXPG₄₋₅, or MTXPG₅.
63. The method of embodiment 61, wherein the level of MTXPG₃ is determined within the first 6 months of starting MTX therapy.
64. The method of embodiment 63, wherein a level of MTXPG₃ greater than about 20 nmol/L indicates that said subject has a high likelihood of responding to MTX about 3 months later.
65. The method of embodiment 63, wherein a detectable level of MTXPG₃ indicates that said subject has a high likelihood of responding to MTX about 1 month later.
66. The method of embodiment 58, wherein said sample is red blood cells.
67. The method of embodiment 58, wherein the level of MTXPGs is determined using high performance liquid chromatography (HPLC).
68. The method of embodiment 58, wherein the level of MTXPGs is determined using mass spectrometry.
69. The present invention further relates to an assay method for optimizing dose efficacy in a subject receiving methotrexate (MTX), said method comprising:
   (a) determining a level of folate polyglutamates (folate PGs) in a sample from said subject; and
   (b) recommending a subsequent dose of MTX based upon the level of folate PGs.
70. The method of embodiment 69, wherein the level of folate PGs is compared to a level of folate PGs from said subject at an earlier time.
71. The method of embodiment 70, wherein a decrease in the level of folate PGs indicates that the subsequent dose of MTX should be maintained.
72. The method of embodiment 69, wherein said sample is red blood cells.
73. The method of embodiment 69, wherein the level of folate PGs is determined using a radioassay.

## Claims

1. An assay method for evaluating the likelihood that a subject will respond to methotrexate (MTX) therapy, the method comprising:
(a) determining a level of folate polyglutamates (PGs) in a sample from the subject; and
(b) evaluating the likelihood that the subject will respond to MTX therapy based upon the level of folate PGs.

2. The method of claim 1, wherein (i) a level of folate PGs less than a threshold level indicates the subject has a high likelihood of responding to MTX therapy; or (ii) a level of folate PGs greater than the threshold level indicates that the subject does not have a high likelihood of responding to MTX therapy.

3. The method of claim 2, wherein the threshold level is about 1000 nmol/L.

4. The method of claim 2 or 3, wherein the level of folate PGs is greater than the threshold level, further comprising recommending increasing a subsequent dosage of MTX therapy or administering a different therapeutic to the subject.

5. An assay method for optimizing dose efficacy in a subject receiving methotrexate (MTX) therapy, the method comprising:
(a) determining a level of folate polyglutamates (PGs) in a sample from the subject; and
(b) recommending a subsequent dose of MTX therapy based upon the level of folate PGs.

6. The method of claim 5, wherein a level of folate PGs that is greater than a threshold level indicates that the subsequent dose of MTX therapy should be increased.

7. The method of claim 6, wherein the threshold level is about 1000 nmol/L.

8. The method of claim 5, wherein the level of folate PGs is compared to a level of folate PGs from the subject at an earlier time.

9. The method of claim 8, wherein an increase in the level of folate PGs over the time period analyzed indicates that the subsequent dose of MTX therapy should be increased.

10. The method of claim 8, wherein a decrease in the level of folate PGs over the time period analyzed indicates that the subsequent dose of MTX therapy should be maintained.

11. The method of any of claims 1-10, wherein the subject has an inflammatory disease, an autoimmune disease, or cancer.

12. The method of claim 11, wherein the subject has an inflammatory disease which is rheumatoid arthritis.

13. The method of any of claims 1-12, wherein the sample is a red blood cell sample or a red blood cell extract from the subject.

14. The method of any of claims 1-13, wherein the level of folate PGs is determined by a competitive binding radioassay.

15. Use of an assay for evaluating the likelihood that a subject will respond to methotrexate (MTX) therapy, wherein the assay comprises:
(a) determining a level of folate polyglutamates (PGs) in a sample from the subject; and
(b) evaluating the likelihood that the subject will respond to MTX therapy based upon the level of folate PGs.
